# EUROPEAN PATENT APPLICATION

(11) **EP 2 112 221 A1**
(43) Date of publication of application: **28.10.2009**
(21) Application number: 08154949.5
(22) Date of filing: 22.04.2008
(51) Int. Cl.: C12N 15/11, C12N 15/85, A01K 67/027

(54) **Hybrid H1 Promoter for shRNA Expression**

(71) Applicant: TaconicArtemis GmbH, 51063 Köln (DE)
(72) Inventor: Seibler, Jost, 50733 Köln (DE); Schwenk, Frieder, 50733 Köln (DE); Reiss, Martina, 50670 Köln (DE)
(74) Representative: Helbing, Jörg

(57) **Abstract**

The invention provides a hybrid H1RNA gene promoter comprising a proximal sequence element (PSE) derived from the H1RNA gene promoter and a heterologuous distal sequence element (DSE), constructs for the expression of short nucleic acid sequences, such as shRNA, containing such a hybrid promoter, as well as biological entities containing such a hybrid promoter or such nucleic acid expression construct.

## Description

The invention provides a hybrid H1RNA gene promoter comprising a proximal sequence element (PSE) derived from the H1RNA gene promoter and a heterologuous distal sequence element (DSE), constructs for the expression of short nucleic acid sequences, such as shRNA, containing such a hybrid promoter, as well as biological entities containing such a hybrid promoter or such nucleic acid expression construct.

### Background of the Invention

RNA interference (RNAi) has been discovered some years ago as a tool for inhibition of gene expression (Fire, A. et al., Nature 391, 806-811 (1998)). It based on the introduction of double stranded RNA (dsRNA) molecules into cells, whereby one strand is complementary to the coding region of a target gene. Through pairing of the specific mRNA with the introduced RNA molecule, the mRNA is degraded by a cellular mechanism. Since long dsRNA provokes an interferon response in mammalian cells, the technology was initially restricted to organisms or cells showing no interferon response (Bass, B.L., Nature 411, 428-429 (2001)). The finding that *short* (<35 bp) *interfering RNAs* (siRNA) circumvent the interferon response extended the application to mammalian cells (Elbashir, S.M. et al., Nature 411, 494-498 (2001)).

Sustained gene silencing has been achieved by the intracellular transcription of small inverted repeat sequences separated by a spacer region (Brummelkamp et al. Science, 296, 550-553. (2002)). For the expression of these short hairpin RNAs (shRNAs), the RNA polymerase III dependent promoters U6 and H1 have been used in most cases (Brummelkamp et al. Science, 296, 550-553. (2002); Tuschl T. Nat Biotechnol. 20, 446-448 (2002)). These promoters produce small transcripts lacking nonfunctional bases at the 5' end and a polyadenosine tail. The resulting shRNAs are processed *in vivo* into active siRNAs.

Temporary control of shRNA expression can be achieved by using engineered promoters containing a tetracycline operator (tetO) sequences (Ohkawa, J. and Taira, K., Hum. Gene Ther. 11(4):577-85 (2000)). The Tetracycline operator itself has no effect on shRNA expression. In the presence of the tetracycline repressor (tetR), however, transcription is blocked through binding of the repressor to the tetO sequence. De-repression is achieved by adding the inductor doxycycline, which causes the release of the TetR protein from the tetO site and allows transcription from the H1 promoter. Several attempts have been made to apply this strategy for the temporary control of antisense or shRNA expression in cultured cell lines (Ohkawa, J. and Taira, K., Hum. Gene Ther. 11(4):577-85 (2000); van de Wetering, M. et al., EMBO reports VOL 4, NO 6:609-615 (2003); Matsukura, 2003; Czauderna, F. et al., Nucleic Acids Res., 31(21):e127 (2003)). In these reports, the degree of doxycycline-inducible mRNA degradation was variable. In addition, background RNAi activity in the uninduced state was observed (van de Wetering, M. et al., EMBO reports VOL 4, No. 6:609-615 (2003)), indicating a limiting level of tetR expression in these cell lines. We recently found that a codon-optimized repressor gene, such as the tetracycline repressor gene, completely suppresses the activity of shRNA genes under the control of an engineered H1 promoter containing the corresponding operator sequences in transgenic animals (Seibler J. et al., Nucleic Acids Res., 35:e54 (2007)). In contrast thereto, the same configuration with the U6 promoter showed a high degree of shRNA/siRNA background activity in transgenic animals in the absence of the inductor (Seibler J. et al., Nucleic Acids Res., 35:e54 (2007)).

Tissue specific control of shRNA expression in mice has been achieved by inserting inhibitory sequences flanked by *IoxP*-sites (i.e., a promoter inactivating element (PIE)) into the U6 shRNA expression vector (Ventura A. et al. Proc. Natl. Acad. Sci. USA, 101, 10380-10385 (2004); Oberdoerffer P. et al. Mol Cell Biol., 25, 3896-905. (2005); Coumoul X. et al.. Nucleic Acids Res. 33, e102 (2005)). Yu et al. (Genesis 44, 252-261 (2006)). When placed between the essential PSE and DSE control regions of the promoter (the DSE containing one or more domains recognized by transcriptional activators such as Oct-1 and Staf; the PSE recruiting a stable protein complex containing five subunits known as SNAPc or PTF (Myslinsly, E. et al., Nucleic Acids Res. 29, 2502-2509 (2001))), the IoxP flanked 'promoter inactivating element' (PIE) completely blocked the activity of the U6 promoter (Coumoul X. et al.. Nucleic Acids Res. 33, e102 (2005); Yu et al. (Genesis 44,252-261 (2006)). Upon expression of Cre recombinase, the loxP-flanked PIE segment was removed, leaving a a single loxP site behind. This IoxP site did not interfere with U6 promoter activity, enabling Cre mediated control of shRNA expression.

The structure of RNA polymerase III promoters fall into three different categories: In categories I and II, the promoter elements are located entirely within the transcribed region. In category III genes, exemplified by U6 snRNA, 7SK and H1RNA, transcription is driven by cis-acting elements within the 5'-flanking region (Paule & White, Nucleic Acids Res. 28, 1283-1298 (2000)). The sequences required for category III promoter activity are (i) a TATA box, (ii) a proximal sequence element (PSE) and (iii) a distal sequence element (DSE) consisting of octamer and staf binding sequences. In most type III promoters, the DSE is distant from the PSE and TATA box, allowing implementation of additional elements for improvement of shRNA expression, as demonstrated by Yu et al. (Genesis 44,252-261 (2006)) and Coumoul et al. (Nucleic Acids Res. 33, e102 (2005)). In contrast, the H1RNA gene promoter harbors an unusual compact organization within 100 bp of 5'-flanking sequences, with the PSE and DSE sequences being adjacent (Myslinsly E. et al. Nucleic Acids Res. 29, 2502-2509 (2001)). Therefore, the H1 promoter is inaccessible to further modifications, such as integration of elements mediating tissue-specific control of transcription (Yu et al. Genesis 44,252-261 (2006)).

The RNA polymerase III dependent H1RNA and U6 snRNA gene promoters are, however, most commonly used for transgenic shRNA expression, allowing efficient gene silencing in cell lines and animals. Multiple modifications of the H1 promoter have been introduced to achieve for temporal or tissue-specific control of shRNA transcription in transgenic animals. For example, operator sequences of the *E. coli* tetracycline resistance operon (tetO) have been inserted downstream of the regulatory elements of the promoter, allowing inducible regulation of shRNA expression through reversible binding of the tet repressor (van de Wetering, M. et al., EMBO reports VOL 4, NO 6:609-615 (2003)). However, a similar configuration with the U6 promoter is not suitable as a high degree of shRNA background activity in the non-induced state was observed (Seibler J. et al., Nucleic Acids Res., 35:e54 (2007)).

As set forth above, the H1 promoter is compact, exhibits distal and proximal sequence elements (DSE, PSE) within 100 bp of DNA sequence (Myslinsly E. et al. Nucleic Acids Res. 29, 2502-2509 (2001)), and is inaccessible to further modifications within the regulatory region, such as integration of elements mediating tissue-specific control of transcription. These kinds of modifications have been demonstrated by using the U6 promoter, in which the DSE is distant from the PSE and TATA box (Coumoul X. et al. Nucleic Acids Res. 33, e102 (2005); Yu et al. (Genesis 44,252-261 (2006)).

The provision of a promoter that is capable of mediating tissue specific and/or inducible control of transcription of short DNA sequences, specifically for shRNA transcription, is highly desirable.

### Summary of the Invention

It was now surprisingly found that the proximal sequence element (PSE) of the H1-RNA promoter retains its favorable properties for the expression of short nucleic acid sequences such as shRNA when fused to a DSE from a heterologous promoter. Such a hybrid promoter combines the well-characterized features of the H1 promoter (e.g. inducibility by using the H1-tetO-promoter with a tet-operator sequence (SEQ ID NO:1; nucleotides 2537-2555) in the 3' position of the TATA-box (Seibler, Nucleic Acids Res., 35:e54(2007)) and additional features of other promoters (e.g. tissue-specific control) for shRNA expression.

Thus, the present invention provides
(1) a hybrid H1RNA gene promoter comprising a proximal sequence element (PSE) derived from the H1RNA gene promoter and a heterologuous distal sequence element (DSE), which is derived from a promoter differing from the H1RNA gene promoter;
(2) a preferred embodiment of aspect (1) above, wherein the hybrid promoter further comprises a promoter inactivation element (PIE) that is located between the PSE and DSE of the hybrid promoter (the presence of said PIE has the consequence that the promoter is in its inactive state)
(3) an expression construct comprising an expressible short nucleic acid sequence under control of a hybrid promoter as defined in (1) or (2) above;
(4) a biological entity comprising the hybrid promoter of (1) or (2) above and/or the expression construct of (3) above;
(5) a method for the inducible expression of an shRNA sequence in a biological entity which comprises transforming/transfecting a starting biological entity with the expression construct as defined in (3) above; and
(6) the use of the hybrid promoter as defined in (1) or (2) above for the expression of nucleic acid sequences, preferably shRNA.

### Description of the Figures

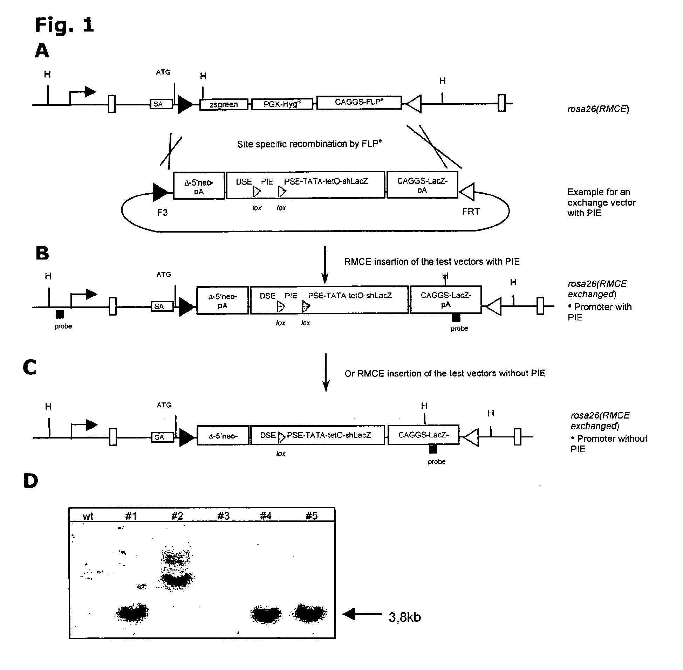

Figure 1: Experimental approach to test Cre-inducible promoter configurations. (A) The *rosa26* targeting vector comprising zsgreen, PGK-Hyg and CAGGS-FLP was inserted into the *rosa26* locus via homologous recombination in ES cells. The F3/FRT sites are oriented in opposite direction to each other. (B) RMCE by Flp^{e}-mediated recombination generates the *rosa26*(RMCE) allele. The exchange vector carries the shRNA expression cassette under the control of the engineered promoter ( e.g. the U6/H1 hybrid promoter), containing the IoxP-flanked 'promoter inactivation Element' (PIE) located between the proximal sequence element (PSE) and the distal sequence element (DSE). A lacZ gene under the control of the CAGGS promoter is included to provide the target for the β-galactosidase-specific shRNA. The truncated neo^{R} gene allows for positive selection upon successful RMCE. (C) Activation of the engineered promoter through Cre-mediated deletion of PIE. Expression of the β-galactosidase-specific shRNA leads to RNAi mediated degradation of the LacZ-transcript. (D) Southern blot analysis of genomic DNA from transfected ES cells. The size for targeted allele is 3.8 kb. No signal is detectable in wt-DNA. In clones 1, 4 and 5 successful RMCE had occurred. Genomic DNA was digested with Hind III and analyzed using probe 1.

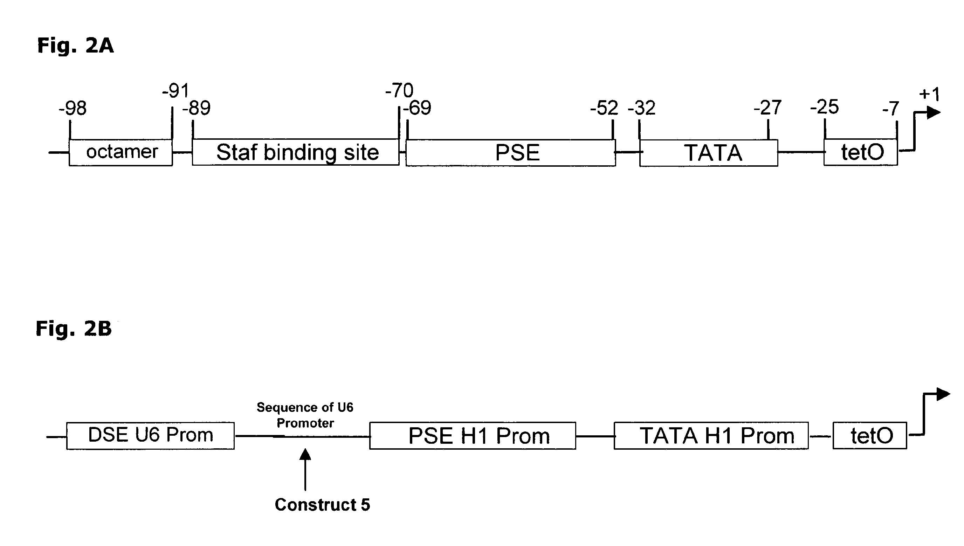

Figure 2: Description of Cre-inducible promoter configurations. Arrows indicate the position of the IoxP-site. (A) Constructs 1, 2, 3a, 3b, 4, 6, 7 (SEQ ID NOs:5, 7, 9, 11, 13, 17 and 19) represent variants of the human H1-promoter carrying a single IoxP-site in different positions between the PSE and the TATA-box (constructs 1, 2, 3a, 3b, and 4) or in between the regulatory sequences upstream of PSE (constructs 6 and 7). (B) Hybrid H1RNA promoter configuration including human U6 DSE and the human H1RNA PSE in construct 5 (SEQ ID NO:15; nucleotides 4989-5006). A single IoxP-site is placed between DSE and PSE control regions.

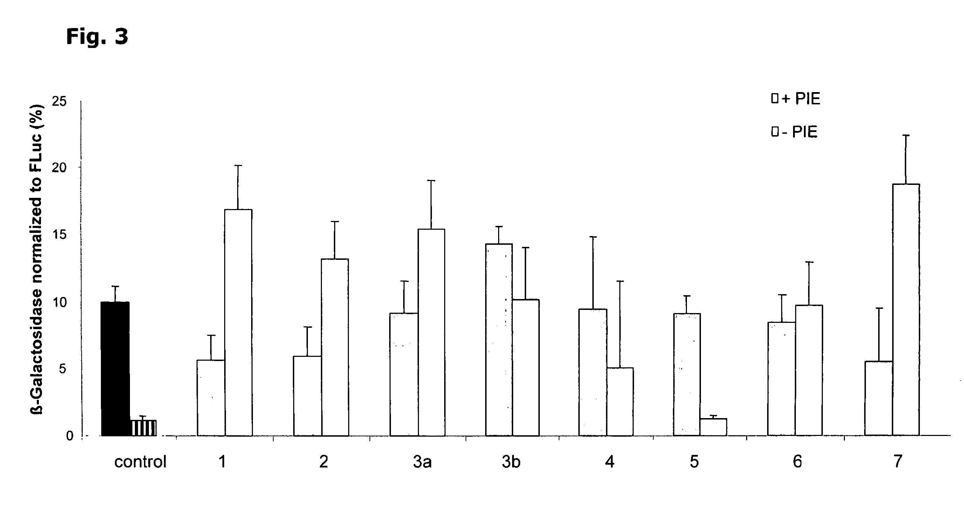

Figure 3: Efficiency of shRNA mediated knockdown of β-galactosidase in mouse embryonic stem cells before and after Cre-mediated activation. Activities of 8 different configurations in the presence or absence of PIE are tested. Black bar: β-galactosidase activity in the presence of an insulin receptor-specific shRNA (negative control). Stripped bar: LacZ-specific shRNA under the control of the wild type H1-tet-promoter lacking IoxP (positive control). β-galactosidase activities in cells carrying the lacZ-specific shRNA constructs before (gray bars) and after deletion of PIE (white bars) +/standard error of the mean are shown are shown. All values of Fluc activity were normalized by using the Rluc activity for reference. Configurations indicated at the X-axis represent constructs shown in Figure 2.

### Detailed Description of the Invention

The hybrid promoter of aspect (1) of the invention (hereinafter also shortly referred to as the "hybrid promoter of the invention") is composed of a proximal proximal sequence element (PSE) derived from the H1RNA gene promoter and a heterologuous distal sequence element (DSE). A "distil sequence element" (DSE) within the meaning of the invention is composed of several functional elements that can be present either simultaneously or separately. Two of these are often the octamer and the Staf motifs (Myslinsly E. et al. Nucleic Acids Res. 29, 2502-2509 (2001)). The octamer motif binds Oct-1, a homeodomain transcriptional activator. The transcriptional activator Staf recognizes the Staf motif.

A "proximal sequence element" (PSE) within the meaning of the invention recruits a stable protein complex, known as SNAPc or PTF, containing five subunits (Myslinsly E. et al. Nucleic Acids Res. 29, 2502-2509 (2001)).

According to a preferred embodiment of the invention, the PSE can be derived from H1RNA gene promoters of various species including human, mouse, rat, goat, zebrafish etc. The human H1RNA PSE notably that consisting of nucleotides 4989-5006 of SEQ ID NO:14 is particularly preferred.

The hybrid promoter of the invention may further comprising a TATA box, said TATA box being derived from the H1RNA gene promoter, for from any other heterologous promoter (including the promoter of the heterologous DSE). Preferably the TATA box is derived from the human H1RNA gene promoter.

According to a further preferred embodiment of the invention, the DSE is derived from tissue specific or ubiquitous, constitutive or inducible RNA Polymerase I, II or III dependent promoters. Among said promoters, RNA polymerase III dependent promoters are preferred. Particularly preferred is that the heterologous DSE is derived from the human U6 snRNA gene promoter (notably that of SEQ ID NO:20), the human U2 promoter (notably that of SEQ ID NO:21), the mouse U2 promoter (notably that of SEQ ID NO:22), the *Xenopus* U2 promoter, the rat U2 promoter, the chicken U4B promoter (notably that of SEQ ID NO:23), the human U4C promoter, Trsp, cmv enhancer (SEQ ID NO:24), sv40 large T antigen enhancer (SEQ ID NO:25), etc. Here it is preferred that the DSE is derived from the human U6 snRNA gene promoter, particularly preferred is the human U6 snRNA gene promoter DSE having SEQ ID NO:20 (corresponding to nucleotides 2464-2486 of SEQ ID NO:14).

The preferred structure of the hybrid promoter is the following structure: 5'- DSE - PSE - TATA box -3',
wherein terms DSE, PSE and TATAbox have the meaning as defined hereinbefore.

A particular preferred hybrid promoter is shown in SEQ ID NO:26.

Said promoter may, however, contain further functional elements including operator sequences, enhancer elements, transcription factor binding sites, entire gene expression cassettes, promoter inactivation elements, etc.

The promoter may be present in an inactive state (aspect (2) of the invention) if it further comprises a promoter inactivation element (PIE). Such PIE is preferably an excisable inactivation sequence that is located between the PSE and DSE of the hybrid promoter. Upon excision of the PIE the hybrid promoter is converted into its activated state.

In a particularly preferred embodiment of aspect (2) the excisable inactivation sequence is flanked by recombinase recognition sequences (RRSs) resulting in a conditional excision if contacted with the respective recombinase. Particularly preferred RRSs are selected from IoxP sites frt sites , att sites, etc. (that allow excision by contact with Cre, Flp λ-Int). The inactivation sequence is an arbitrary non-functional or functional sequence including marker sequences including marker sequences, polyA sequences, splice-acceptor sequences, recombinase genes, etc.

Most preferably the PIE is the IoxP-flanked Ubi-AsRed or Ubi-Zsgreen sequence shown in SEQ ID NOs: 2 and 3, respectively.

The expression construct according to embodiment (3) of the invention comprises expressible short nucleic acid sequence (namely sequences of less than 200 nucleotides that are normally not sufficiently transcribed by DNA Pol II dependent promoters) under control of a hybrid promoter as defined hereinbefore. The promoter is of course also suitable for transcription/expression of larger nucleic acid sequences

In said expression construct the nucleic acid sequence may be coding or noncoding and is preferably selected from shRNA, DNA/RNA, miRNA, cDNA, genomic DNA, etc. Particularly preferred is shRNA (including the conventional short hairpin RNA (shRNA) and the corresponding complementary short interfering RNA (siRNA) strands) or miRNA. In case shRNA segments are utilized within the expression construct, said construct preferably comprises at least one shRNA segment having a nucleotide (e.g. DNA) sequence of the structure A-B-C or C-B-A. In case siRNA segments are utilized within the expression construct, said cassette preferably comprises at least least two DNA segments A and C or C and A, wherein each of said at least two segments is under the control of a separate promoter as defined above. In the above segments
A is a 15 to 35, preferably a 19 to 29 bp DNA sequence being at least 90%, preferably 100% complementary to the gene to be knocked down (e.g. firefly luciferase, p53, etc.);
B is a spacer DNA sequence having 5 to 9 bp forming the loop of the expressed RNA hairpin molecule, and
C is a 15 to 35, preferably a 19 to 29 bp DNA sequence being at least 85% complementary to the sequence A.

The above shRNA and siRNA segments may further comprise stop and/or polyadenylation sequences.

Suitable shRNA sequences for the knock down of a given target gene are well known in the art (see e.g. the particular shRNA sequences mentioned in Tables 1 and 2 below) or can readily be determined by the skilled artesian.

**Table 1:**

| target gene | shRNA sequence /SEQ ID NO | Reference |
|---|---|---|
| CDH-1 p53 CDC20 | TgagaagtctcccagtcagTTCAAGAGActgactgggagacttctca (29) GactccagtggtaatctacTTCAAGAGAgtagattaccactggagtc (30) CggcaggactccgggccgaTTCAAGAGAtcggcccggagtcctgccg (31) | Brummelkamp et al., Science, 296:550-3 (2002). |
| CYLD | CctcatgcagttctctttgTTCAAGAGAcaaagagaactgcatgagg (32) | Kovalenko et al., Nature, 424:801-5 (2003). |
| Ras-Gap | AagatgaagccactccctatttCAAGAGAaaatagggagtggcttcatctt (33) | Kunath et al., Nature Biotech, 21:559-561 (2003). |
| tubulin | GacagagccaagtggactcACAgagtccacttggctctgtc (34) | Yu et al., PNAS, 99: 6047-52 (2002) |
| lamin | Ctggacttccagaagaacattcgtgttcttctggaagtccag (35) | Paul et al., Nature Biotech, 20:505-8 (2002). |

**Table 2 shRNA sequences known from Brummelkamp et al., Nature, 424:797-801 (2003):**

| | |
|---|---|
| | |

| Target Gene | shRNA Sequence / SEQ ID NO |
|---|---|
| UBIQUITIN | GAGATTGGTCCAGAACAGTTTCAAGAGAACTGTTCTGGACCAATCTC (36) |
| CARBOXYL- | GCCCTTCCGATCATGGTAGTTCAAGAGACTACCATGATCGGAAGGGC (37) |
| TERMINAL | TCTTTAGAATTCTTAAGTATTCAAGAGATACTTAAGAATTCTAAAGA (38) |
| HYDROLASE 12 | CATTAGCTATATCAACATGTTCAAGAGACATGTTGATATAGCTAATG (39) |
| UBIQUITIN | ACCACAAACGGCGGAACGATTCAAGAGATCGTTCCGCCGTTTGTGGT (40) |
| CARBOXYL- | GAGGGTCTTGGAGGTCTTCTTCAAGAGAGAAGACCTCCAAGACCCTC (41) |
| TERMINAL | GTCCATGCCCAGCCGTACATTCAAGAGATGTACGGCTGGGCATGGAC (42) |
| HYDROLASE 11 | GCTGGACACCCTCGTGGAGTTCAAGAGACTCCACGAGGGTGTCCAGC (43) |
| UBIQUITIN | GAATATCAGAGAATTGAGTTTCAAGAGAACTCAATTCTCTGATATTC (44) |
| CARBOXYL- | TGGACTTCATGAGGAAATGTTCAAGAGACATTTCCTCATGAAGTCCA (45) |
| TERMINAL | TATTGAATATCCTGTGGACTTCAAGAGAGTCCACAGGATATTCAATA (46) |
| HYDROLASE 10 | TTGTACTGAGAGAAACTGCTTCAAGAGAGCAGTTTCTCTCAGTACAA (47) |
| HAUSP | GATCAATGATAGGTTTGAATTCAAGAGATTCAAACCTATCATTGATC (48) |
| | GGAGTTTGAGAAGTTTAAATTCAAGAGATTTAAACTTCTCAAACTCC (49) |
| | GAACTCCTCGCTTGCTGAGTTCAAGAGACTCAGCAAGCGAGGAGTTC (50) |
| | CCGAATTTAACAGAGAGAATTCAAGAGATTCTCTCTGTTAAATTCGG (51) |
| UBIQUITIN | GACAGCAGAAGAATGCAGATTCAAGAGATCTGCATTCTTCTGCTGTC (52) |
| CARBOXYL- | ATAAAGCTCAACGAGAACCTTCAAGAGAGGTTCTCGTTGAGCTTTAT (53) |
| TERMINAL | GGTGAAGTGGCAGAAGAATTTCAAGAGAATTCTTCTGCCACTTCACC (54) |
| HYDROLASE 8 | GTATTGCAGTAATCATCACTTCAAGAGAGTGATGATTACTGCAATAC (55) |
| FLJ10785 | GATATGGGGTTCCATGTCATTCAAGAGATGACATGGAACCCCATATC (56) |
| | GGAGACATGGTTCTTAGTGTTCAAGAGACACTAAGAACCATGTCTCC (57) |
| | AGCACCAAGTTCGTCTCAGTTCAAGAGACTGAGACGAACTTGGTGCT (58) |
| | GATGCAACACTGAAAGAACTTCAAGAGAGTTCTTTCAGTGTTGCATC (59) |
| KIAA0710 | GTCAATGGCAGTGATGATATTCAAGAGATATCATCACTGCCATTGAC (60) |
| | CCTGCTAGCTGCCTGTGGCTTCAAGAGAGCCACAGGCAGCTAGCAGG (61) |
| | CCACCTTTGCCAGAAGGAGTTCAAGAGACTCCTTCTGGCAAAGGTGG (62) |
| | CCCTATTGAGGCAAGTGTCTTCAAGAGAGACACTTGCCTCAATAGGG (63) |
| FLJ12552/ | GAAGGAAAACTTGCTGACGTTCAAGAGACGTCAGCAAGTTTTCCTTC (64) |
| FLJ14256 | CTCACCTGGGTCCATGAGATTCAAGAGATCTCATGGACCCAGGTGAG (65) |
| | GCTGTCTTACCGTGTGGTCTTCAAGAGAGACCACACGGTAAGACAGC (66) |
| | CCTGGACCGCATGTATGACTTCAAGAGAGTCATACATGCGGTCCAGG (67) |
| KIAA1203 | GTCAATGGCAGTGATGATATTCAAGAGATATCATCACTGCCATTGAC (68) |
| | CCTGCTAGCTGCCTGTGGCTTCAAGAGAGCCACAGGCAGCTAGCAGG (69) |
| | CCACCTTTGCCAGAAGGAGTTCAAGAGACTCCTTCTGGCAAAGGTGG (70) |
| | CCCTATTGAGGCAAGTGTCTTCAAGAGAGACACTTGCCTCAATAGGG (71) |
| FLJ23277 | GGAAATCCGAATTGCTTGGTTCAAGAGACCAAGCAATTCGGATTTCC (72) |
| | CACATTTCTTCAAGTGTGGTTCAAGAGACCACACTTGAAGAAATGTG (73) |
| | CAGCAGGATGCTCAAGAATTTCAAGAGAATTCTTGAGCATCCTGCTG (74) |
| | GCTGAATACCTACATTGGCTTCAAGAGAGCCAATGTAGGTATTCAGC (75) |
| FLJ14914 | (similarGGGCTTGTGCCTGGCCTTGTTCAAGAGACAAGGCCAGGCACAAGCCC (76) |
| to UBP4) | GCCTTGTCCTGCCAAGAAGTTCAAGAGACTTCTTGGCAGGACAAGGC (77) |
| | GATTGAAGCCAAGGGAACGTTCAAGAGACGTTCCCTTGGCTTCAATC (78) |
| | TGGCGCCTGCTCCCCATCTTTCAAGAGAAGATGGGGAGCAGGCGCCA (79) |
| UBIQUITIN CAR- | GAACCAGCAGGCTCTGTGGTTCAAGAGACCACAGAGCCTGCTGGTTC (80) |
| BOXYL-TERMINAL | GGAAGCATAATTATCTGCCTTCAAGAGAGGCAGATAATTATGCTTCC (81) |
| HYDROLASE | AGAAGAAGATGCTTTTCACTTCAAGAGAGTGAAAAGCATCTTCTTCT (82) |
| ISOZYME L5 | CTTGCAGAGGAGGAACCCATTCAAGAGATGGGTTCCTCCTCTGCAAG (83) |
| UBIQUITIN CAR- | GCAAACAATCAGCAATGCCTTCAAGAGAGGCATTGCTGATTGTTTGC (84) |
| BOXYL-TERMINAL | TTGGACTGATTCATGCTATTTCAAGAGAATAGCATGAATCAGTCCAA (85) |
| HYDROLASE | CTGGCAATTCGTTGATGTATTCAAGAGATACATCAACGAATTGCCAG (86) |
| ISOZYME L3 | TTAGATGGGCGGAAGCCATTTCAAGAGAATGGCTTCCGCCCATCTAA (87) |
| UBIQUITIN CAR- | GAGGAGTCTCTGGGCTCGGTTCAAGAGACCGAGCCCAGAGACTCCTC (88) |
| BOXYL-TERMINAL | GAGCTGAAGGGACAAGAAGTTCAAGAGACTTCTTGTCCCTTCAGCTC (89) |
| HYDROLASE | TGTCGGGTAGATGACAAGGTTCAAGAGACCTTGTCATCTACCCGACA (90) |
| ISOZYMEL1 | CACAGCTGTTCTTCTGTTCTTCAAGAGAGAACAGAAGAACAGCTGTG (91) |
| KIAA1891 / | GTGGAAGCCTTTACAGATCTTCAAGAGAGATCTGTAAAGGCTTCCAC (92) |
| FLJ25263 | CAACAGCTGCCTTCATCTGTTCAAGAGACAGATGAAGGCAGCTGTTG (93) |
| | CCATAGGCAGTCCTCCTAATTCAAGAGATTAGGAGGACTGCCTATGG (94) |
| | TGTATCACTGCCACTGGTTTTCAAGAGAAACCAGTGGCAGTGATACA (95) |
| FLJ14528 | (similar CATGTTGGGCAGCTGCAGCTTCAAGAGAGCTGCAGCTGCCCAACATG (96) |
| to UBP8) | CACAACTGGAGACCTGAAGTTCAAGAGACTTCAGGTCTCCAGTTGTG (97) |
| | GTATGCCTCCAAGAAAGAGTTCAAGAGACTCTTTCTTGGAGGCATAC (98) |
| | CTTCACAGTACATTTCTCTTTCAAGAGAAGAGAAATGTACTGTGAAG (99) |
| U4/U6 TRI | GTACTTTCAAGGCCGGGGTTTCAAGAGAACCCCGGCCTTGAAAGTAC |
| SNRNP 65 kDa | (100) |
| protein | CTTGGACAAGCAAGCCAAATTCAAGAGATTTGGCTTGCTTGTCCAAG (101) |
| | GACTATTGTGACTGATGTTTTCAAGAGAAACATCAGTCACAATAGTC (102) |
| | GGAGAACTTTCTGAAGCGCTTCAAGAGAGCGCTTCAGAAAGTTCTCC (103) |
| XM_089437 | GACGAGAGAAACCTTCACCTTCAAGAGAGGTGAAGGTTTCTCTCGTC (104) |
| | ACATTATTCTACATTCTTTTTCAAGAGAAAAGAATGTAGAATAATGT (105) |
| | AGATTCGCAAATGGATGTATTCAAGAGATACATCCATTTGCGAATCT (106) |
| | CATTCCCACCATGAGTCTGTTCAAGAGACAGACTCATGGTGGGAATG (107) |
| KIAA1453 | GATCGCCCGACACTTCCGCTTCAAGAGAGCGGAAGTGTCGGGCGATC |
| | (108) |
| | CCAGCAGGCCTACGTGCTGTTCAAGAGACAGCACGTAGGCCTGCTGG |
| | (109) |
| | GCCAGCTCCTCCACAGCACTTCAAGAGAGTGCTGTGGAGGAGCTGGC(110) |
| | CGCCGCCAAGTGGAGCAGATTCAAGAGATCTGCTCCACTTGGCGGCG (111) |
| FLJ12697 | GAAGATGCCCATGAATTCCTTCAAGAGAGGAATTCATGGGCATCTTC (112) |
| | CAAACAGGCTGCGCCAGGCTTCAAGAGAGCCTGGCGCAGCCTGTTTG (113) |
| | ACGGCCTAGCGCCTGATGGTTCAAGAGACCATCAGGCGCTAGGCCGT(114) |
| | CTGTAACCTCTCTGATCGGTTCAAGAGACCGATCAGAGAGGTTACAG (115) |
| UBIQUITIN | TCTGTCAGTCCATCCTGGCTTCAAGAGAGCCAGGATGGACTGACAGA (116) |
| SPECIFIC | TGAAGCGAGAGTCTTGTGATTCAAGAGATCACAAGACTCTCGCTTCA (117) |
| PROTEASE 18 | GATGGAGTGCTAATGGAAATTCAAGAGATTTCCATTAGCACTCCATC (118) |
| (USP18) | CCTTCAGAGATTGACACGCTTCAAGAGAGCGTGTCAATCTCTGAAGG (119) |
| UBIQUITIN | CCTGACCACGTTCCGACTGTTCAAGAGACAGTCGGAACGTGGTCAGG (120) |
| CARBOXYL- | GAGTTCCTTCGCTGCCTGATTCAAGAGATCAGGCAGCGAAGGAACTC (121) |
| TERMINAL | GACTGCCTTGCTGCCTTCTTTCAAGAGAAGAAGGCAGCAAGGCAGTC(122) |
| HYDROLASE 20 | CGCCGAGGGCTACGTACTCTTCAAGAGAGAGTACGTAGCCCTCGGCG (123) |
| UBIQUITIN | GGCGAGAAGAAAGGACTGTTTCAAGAGAACAGTCCTTTCTTCTCGCC (124) |
| CARBOXYL- | GGACGAGAATTGATAAAGATTCAAGAGATCTTTATCAATTCTCGTCC (125) |
| TERMINAL | GCACGAGAATTTGGGAATCTTCAAGAGAGATTCCCAAATTCTCGTGC (126) |
| HYDROLASE 24 | CTACTTCATGAAATATTGGTTCAAGAGACCAATATTTCATGAAGTAG (127) |
| KIAA1594 | GATAACAGCTTCTTGTCTATTCAAGAGATAGACAAGAAGCTGTTATC (128) |
| | GAGAATAGGACATCAGGGCTTCAAGAGAGCCCTGATGTCCTATTCTC (129) |
| | CTTGGAAGACTGAACCTGTTTCAAGAGAACAGGTTCAGTCTTCCAAG (130) |
| | CAACTCCTTTGTGGATGCATTCAAGAGATGCATCCACAAAGGAGTTG (131) |
| KIAA1350 | GATGTTGTCTCCAAATGCATTCAAGAGATGCATTTGGAGACAACATC(132) |
| | CGTGGGGACTGTACCTCCCTTCAAGAGAGGGAGGTACAGTCCCCACG (133) |
| | GTACAGCTTCAGAACCAAGTTCAAGAGACTTGGTTCTGAAGCTGTAC(134) |
| UBIQUITIN | GATGATCTTCAGAGAGCAATTCAAGAGATTGCTCTCTGAAGATCATC (135) |
| CARBOXYL- | GGAACATCGGAATTTGCCTTTCAAGAGAAGGCAAATTCCGATGTTCC (136) |
| TERMINAL | GAGCTAGTGAGGGACTCTTTTCAAGAGAAAGAGTCCCTCACTAGCTC(137) |
| HYDROLASE 25 | GCAGGGTTCTTTAAGGCAATTCAAGAGATTGCCTTAAAGAACCCTGC(138) |
| UBIQUITIN | TCGATGATTCCTCTGAAACTTCAAGAGAGTTTCAGAGGAATCATCGA (139) |
| CARBOXYL- | GATAATGGAAATATTGAACTTCAAGAGAGTTCAATATTTCCATTATC(140) |
| TERMINAL | GTTCTTCATTTAAATGATATTCAAGAGATATCATTTAAATGAAGAAC(141) |
| HYDROLASE 16 | GTTAACAAACACATAAAGTTTCAAGAGAACTTTATGTGTTTGTTAAC(142) |
| USP9X | GTTAGAGAAGATTCTTCGTTTCAAGAGAACGAAGAATCTTCTCTAAC(143) |
| | GTTGATTGGACAATTAAACTTCAAGAGAGTTTAATTGTCCAATCAAC (144) |
| | GGTTGATACCGTAAAGCGCTTCAAGAGAGCGCTTTACGGTATCAACC(145) |
| | GCAATGAAACGTCCAATGGTTCAAGAGACCATTGGACGTTTCATTGC(146) |
| USP9Y | AGCTAGAGAAAATTCTTCGTTCAAGAGACGAAGAATTTTCTCTAGCT(147) |
| | GATCCTATATGATGGATGATTCAAGAGATCATCCATCATATAGGATC (148) |
| | GTTCTTCTTGTCAGTGAAATTCAAGAGATTTCACTGACAAGAAGAAC(149) |
| | CTTGAGCTTGAGTGACCACTTCAAGAGAGTGGTCACTCAAGCTCAAG (150) |
| UBIQUITIN | GACCGGCCAGCGAGTCTACTTCAAGAGAGTAGACTCGCTGGCCGGTC |
| CARBOXYL- | (151) |
| TERMINAL | GGACCTGGGCTACATCTACTTCAAGAGAGTAGATGTAGCCCAGGTCC |
| HYDROLASE 5 | (152) |
| | CTCTGTGGTCCAGGTGCTCTTCAAGAGAGAGCACCTGGACCACAGAG (153) |
| | GACCACACGATTTGCCTCATTCAAGAGATGAGGCAAATCGTGTGGTC (154) |
| UBIQUITIN | TGGCTTGTTTATTGAAGGATTCAAGAGATCCTTCAATAAACAAGCCA (155) |
| CARBOXYL- | GTGAATTTGGGGAAGATAATTCAAGAGATTATCTTCCCCAAATTCAC(156) |
| TERMINAL | CGCTATAGCTTGAATGAGTTTCAAGAGAACTCATTCAAGCTATAGCG (157) |
| HYDROLASE 26 | GATATCCTGGCTCCACACATTCAAGAGATGTGTGGAGCCAGGATATC (158) |
| KIAA1097 | GAGCCAGTCGGATGTAGATTTCAAGAGAATCTACATCCGACTGGCTC (159) |
| | GTAAATTCTGAAGGCGAATTTCAAGAGAATTCGCCTTCAGAATTTAC(160) |
| | GCCCTCCTAAATCAGGCAATTCAAGAGATTGCCTGATTTAGGAGGGC (161) |
| | GTTGAGAAATGGAGTGAAGTTCAAGAGACTTCACTCCATTTCTCAAC(162) |
| UBIQUITIN | GCTTGGAAAATGCAAGGCGTTCAAGAGACGCCTTGCATTTTCCAAGC |
| SPECIFIC | (163) |
| PROTEASE 22 | CTGCATCATAGACCAGATCTTCAAGAGAGATCTGGTCTATGATGCAG (164) |
| (USP22) | GATCACCACGTATGTGTCCTTCAAGAGAGGACACATACGTGGTGATC (165) |
| | TGACAACAAGTATTCCCTGTTCAAGAGACAGGGAATACTTGTTGTCA (166) |
| UBIQUITIN- | GAAATATAAGACAGATTCCTTCAAGAGAGGAATCTGTCTTATATTTC (167) |
| SPECIFIC | CCCATCAAGTTTAGAGGATTTCAAGAGAATCCTCTAAACTTGATGGG (168) |
| PROCESSING | GGTGTCCCATGGGAATATATTCAAGAGATATATTCCCATGGGACACC (169) |
| PROTEASE 29 | GAATGCCGACCTACAAAGATTCAAGAGATCTTTGTAGGTCGGCATTC(170) |
| CYLD | CAGTTATATTCTGTGATGTTTCAAGAGAACATCACAGAATATAACTG (171) |
| | GAGGTGTTGGGGACAAAGGTTCAAGAGACCTTTGTCCCCAACACCTC (172) |
| | GTGGGCTCATTGGCTGAAGTTCAAGAGACTTCAGCCAATGAGCCCAC (173) |
| | GAGCTACTGAGGACAGAAATTCAAGAGATTTCTGTCCTCAGTAGCTC(174) |
| UBIQUITIN | TCAGCAGGATGCTCAGGAGTTCAAGAGACTCCTGAGCATCCTGCTGA |
| CARBOXYL- | (175) |
| TERMINAL | GAAGTTCTCCATCCAGAGGTTCAAGAGACCTCTGGATGGAGAACTTC |
| HYDROLASE 2 | (176) |
| | GCCGGTCCCCACCAGCAGCTTCAAGAGAGCTGCTGGTGGGGACCGGC(17 7) |
| | CACTCGGGAGTTGAGAGATTTCAAGAGAATCTCTCAACTCCCGAGTG (178) |
| UBIQUITIN | GCCCTTGGGTCTGTTTGACTTCAAGAGAGTCAAACAGACCCAAGGGC |
| SPECIFIC | (179) |
| PROTEASE 3 | CTCAACACTAAACAGCAAGTTCAAGAGACTTGCTGTTTAGTGTTGAG (180) |
| (USP3) | GATTTCATTGGACAGCATATTCAAGAGATATGCTGTCCAATGAAATC(181) |
| | CATGGGGCACCAACTAATTTTCAAGAGAAATTAGTTGGTGCCCCATG (182) |
| UBIQUITIN | GGTGTCTCTGCGGGATTGTTTCAAGAGAACAATCCCGCAGAGACACC |
| CARBOXYL- | (183) |
| TERMINAL | AGTTCAGTAGGTGTAGACTTTCAAGAGAAGTCTACACCTACTGAACT(184) |
| HYDROLASE 23 | GAGTTCCTGAAGCTCCTCATTCAAGAGATGAGGAGCTTCAGGAACTC (185) |
| | GGATTTGCTGGGGGCAAGGTTCAAGAGACCTTGCCCCCAGCAAATCC (186) |
| UBP-32.7 | CTCAGAAAGCCAACATTCATTCAAGAGATGAATGTTGGCTTTCTGAG (187) |
| | CGCATTGTAATAAGAAGGTTTCAAGAGAACCTTCTTATTACAATGCG (188) |
| | GGGAGGAAAATGCAGAAATTTCAAGAGAATTTCTGCATTTTCCTCCC(189) |
| | TTACAAATTTAGGAAATACTTCAAGAGAGTATTTCCTAAATTTGTAA(190) |
| HOMO SAPIENS | GTTATGAATTGATATGCAGTTCAAGAGACTGCATATCAATTCATAAC (191) |
| UBIQUITIN SPE- | GTGATAACACAACTAATGGTTCAAGAGACCATTAGTTGTGTTATCAC (192) |
| CIFIC PROTEASE | GTAGAGGAGAGTTCTGAAATTCAAGAGATTTCAGAACTCTCCTCTAC (193) |
| 13 (ISOPEP- | GCCTCTAATCCTGATAAGGTTCAAGAGACCTTATCAGGATTAGAGGC (194) |
| TIDASE T-3) | |
| UBIQUITIN | GATGATCTTCAGGCTGCCATTCAAGAGATGGCAGCCTGAAGATCATC (195) |
| CARBOXYL- | GTATGGACAAGAGCGTTGGTTCAAGAGACCAACGCTCTTGTCCATAC (196) |
| TERMINAL | CGAACCCTTCTGGAACAGTTTCAAGAGAACTGTTCCAGAAGGGTTCG (197) |
| HYDROLASE 28 | GTGGCATGAAGATTATAGTTTCAAGAGAACTATAATCTTCATGCCAC(198) |
| UBIQUITIN | GGTGAACAAGGACAGTATCTTCAAGAGAGATACTGTCCTTGTTCACC (199) |
| CARBOXYL- | GCAATAGAGGATGATTCTGTTCAAGAGACAGAATCATCCTCTATTGC (200) |
| TERMINAL | TCTGTGAATGCCAAAGTTCTTCAAGAGAGAACTTTGGCATTCACAGA(201) |
| HYDROLASE 14 | CACACCAGGGAAGGTCTAGTTCAAGAGACTAGACCTTCCCTGGTGTG (202) |
| DUB1 | GCAGGAAGATGCCCATGAATTCAAGAGATTCATGGGCATCTTCCTGC (203) |
| | GAATGTGCAATATCCTGAGTTCAAGAGACTCAGGATATTGCACATTC (204) |
| | TGGATGATGCCAAGGTCACTTCAAGAGAGTGACCTTGGCATCATCCA (205) |
| | GCTCCGTGCTAAACCTCTCTTCAAGAGAGAGAGGTTTAGCACGGAGC(206) |
| MOUSE USP27 | GCCTCCACCTCAACAGAGGTTCAAGAGACCTCTGTTGAGGTGGAGGC (207) |
| HOMOLOG | CTGCATCATAGACCAAATCTTCAAGAGAGATTTGGTCTATGATGCAG (208) |
| | GATCACTACATACATTTCCTTCAAGAGAGGAAATGTATGTAGTGATC(209) |
| | GTAAAGAGAGCAGAATGAATTCAAGAGATTCATTCTGCTCTCTTTAC(210) |
| UBIQUITIN | CGCGGGGCGCAGTGGTATCTTCAAGAGAGATACCACTGCGCCCCGCG |
| CARBOXYL- | (211) |
| TERMINAL | CAGAAGGCAGTGGGGAAGATTCAAGAGATCTTCCCCACTGCCTTCTG (212) |
| HYDROLASE 4 | GCCTGGGAGAATCACAGGTTTCAAGAGAACCTGTGATTCTCCCAGGC(213) |
| | ACCAGACAAGGAAATACCCTTCAAGAGAGGGTATTTCCTTGTCTGGT(214) |
| TRE-2 | CACATCCACCACATCGACCTTCAAGAGAGGTCGATGTGGTGGATGTG (215) |
| | GTCACAACCCAAGACCATGTTCAAGAGACATGGTCTTGGGTTGTGAC(216) |
| | CTCAACAGGACAAATCCCATTCAAGAGATGGGATTTGTCCTGTTGAG (217) |
| | TAGATCAATTATTGTGGATTTCAAGAGAATCCACAATAATTGATCTA(218) |
| UBIQUITIN CAR- | GGAACACCTTATTGATGAATTCAAGAGATTCATCAATAAGGTGTTCC (219) |
| BOXYL-TERMINAL | CTTTAACAGAAATTGTCTCTTCAAGAGAGAGACAATTTCTGTTAAAG (220) |
| HYDROLASE 15 | CCTATGCAGTACAAAGTGGTTCAAGAGACCACTTTGTACTGCATAGG (221) |
| (UNPH-2). | GATCTTTTCTTGCTTTGGATTCAAGAGATCCAAAGCAAGAAAAGATC(222) |
| KIAA1372 | CAGCATCCTTCAGGCCTTATTCAAGAGATAAGGCCTGAAGGATGCTG (223) |
| | GATAGTGACTCGGATCTGCTTCAAGAGAGCAGATCCGAGTCACTATC(224) |
| | GACATCACAGCCCGGGAGTTTCAAGAGAACTCCCGGGCTGTGATGTC(225) |
| | GGACACAGCCTATGTGCTGTTCAAGAGACAGCACATAGGCTGTGTCC(226) |
| BRCA1 | GTGGAGGAGATCTACGACCTTCAAGAGAGGTCGTAGATCTCCTCCAC(227) |
| ASSOCIATED | CTCTTGTGCAACTCATGCCTTCAAGAGAGGCATGAGTTGCACAAGAG (228) |
| PROTEIN-1 | ACAGGGCCCCTGCAGCCTCTTCAAGAGAGAGGCTGCAGGGGCCCTGT (229) |
| | GAAGACCTGGCGGCAGGTGTTCAAGAGACACCTGCCGCCAGGTCTTC(230) |

Suitable siRNA sequences for the knockdown of a given target gene are well known in the art ( e.g. the particular siRNA sequences mentioned in Lee N.S. et al., J. Nat. Biotechnol. 20(5):500-5 (2002) gcctgtgcctcttcagctacc (SEQ ID NO:231) and gcggagacagcgacgaagagc (SEQ ID NO:232) and in Du, Q. et al., Nucl. Acids Res. 21; 33(5):1671-7 (2005) cttattggagagagcacga (SEQ ID NO:233)) or can readily be determined by the skilled artisan.

The "expression construct" (hereinafter shortly referred as "vector") according to aspect (3) of the invention of the present application are suitable for stable integration into a biological entity e.g. by homologous recombination, recombinase mediated cassette exchange (hereinafter "RMCE") reaction, or random integration. The vector(s) for integration of the construct into the vertebrates by homologous recombination preferably contain(s) homologous sequences suitable for targeted integration at a defined locus, preferably at a polymerase II or III dependent locus of the living organisms or cells of the cell culture. Such polymerase II or III dependent loci include, but are not limited to, the Rosa26 locus (the murine Rosa26 locus being depicted in SEQ ID NO:27), collagen, RNA polymerase, actin, and HPRT. Homologous sequences suitable for integration into a murine Rosa26 locus can be derived from the targeting vector for homologous recombination shown in SEQ ID Nos:28 (the Rosa 5' targeting arm being nucleotide 30-1079 and the Rosa 3' targeting arm being nucleotides 7982-12313 of said targeting vector).

As set forth above, the vector of aspect (3) of the invention is suitable for recombinase mediated cassette exchange (RMCE). The insertion of transgenes or DNA segments into the genome can be mediated by site specific recombination (Fukushige & Sauer, Proc. Natl. Acad. Sci. USA 89(17):7905-9 (1992)). A site specific recombinase like cre or FLP recombines two recognition target sites like IoxP or FRT, respectively. It goes without saying that the site specific recombinase should not interfere with the site specific recombinase of the hybrid promoter. The use of two incompatible recognition target sites (F3 or F5, Schlake & Bode, Biochemistry, 1994 Nov. 1, 33(43):12746-51) or inverted recognition target sites (Feng et al., J. Mol. Biol. 292(4):779-85 (1999)) allows the insertion of DNA segments flanked by two incompatible or inverted target sites. This exchange system has been called recombinase mediated cassette exchange (RMCE). In a preferred embodiment a FLP based RMCE system is inserted into a particular locus of the biological entity, notably the Rosa26 locus. Said recombinase mediated recombination preferably comprises the steps:
(a1) introducing into the starting cells an acceptor DNA which integrates into the genome of the starting cell, the acceptor DNA comprising two mutally incompatible first RRSs, and introducing into the therewith obtained cell
(a2) a donor DNA comprising the same two mutually incompatible first RRSs contained in the acceptor DNA by utilizing a recombination vector as defined above; and
(a3) the recombinase which catalyzes recombination between the RRSs of the acceptor and donor

For further selectable markers it is referred to U.S. Patents Nos. 5,487,932 and 5,464,763 which are hereby incorporated in their entirety.

A preferred embodiment of the method (1) or (2) of the invention concerns the following steps:
1. Generation of the short hairpin DNA containing the antisense- and sense-strand of the coding region of a gene (e.g. firefly luciferase; p53). Antisense and sense-strand are separated by a spacer of 5 to 9 bp.
2. Generation of constructs for the expression of the above mentioned shRNA under the control of the hybrid promoter.
3. Insertion of the mentioned expression constructs into an exchange vector and subsequent insertion of the exchange vector into a ubiquitously expressed locus in ES cells by RMCE.
4. Analysis of the constitutive and inducible inhibition of gene expression (e.g. firefly luciferase; p53) in ES cells (e.g. through Western blot analysis).
5. Generation of mice using the mentioned ES cells and analysis of the inhibition of gene expression in several tissues (e.g. firefly luciferase; p53; e.g. through Western blot analysis).

The vector of aspect (3) of the invention is suitable for stable integration and transient expression. Said vector is also suitable for gene transfer, e.g. into a living organism.

The above expression construct may further contain functional sequences, preferably selected from selection markers, polyadenylation sites, IRES, gene expression cassettes, additional RNAi vectors, etc.

The "biological entity" of aspect (4) of the invention includes, but is not limited to, a vertebrate, a tissue culture derived from a vertebrate, or one or more cells of a cell culture derived from a vertebrate.

The term "vertebrate" according to the present invention relates to multicellular organisms such as mammals, e.g. non-human animals such as rodents (including mice, rats, etc.) and humans, or non-mammals, e.g. fish. Most preferred vertebrates are mice, rats and fish.

"Tissue culture" according to the present invention refers to parts of the above-defined "vertebrates" (including organs and the like) which are cultured *in vitro.*

"Cell culture" according to the present invention includes cells isolated from the above-defined "vertebrates" which are cultured *in vitro.* These cells can be transformed (immortalized) or untransformed (directly derived from vertebrates; primary cell culture).

The term "living organisms" according to the present invention relates to multi-cell organisms which can be vertebrates such as mammals (e.g. non-human animals such as rodents including mice and rats; and humans) or non-mammals (e.g. fish) or can be invertebrates such as insects or worms, or can be plants (higher plants, algi or fungi). Most preferred living organisms are mice and fish.

"Eukaryotic cells" and "starting eukaryotic cells" according to the present invention include cells isolated (derived) from the above defined living organisms and cultured *in vitro.* These cells can be transformed (immortalized) or untransformed (directly derived from living organisms; primary cell culture). The term "eukaryotic cells" also includes mono-cellular eukaryotic cells such as yeasts, etc.

The method of aspect (5) of the invention also includes a method for preparing a transgenenic multi-cell organism carrying the expression construct of embodiment (3) of the invention, notably at a modified Rosa26 locus. This includes a method for preparing a non-human mammal comprising modifying starting ES cells transforming/transfecting a starting biological entity with the expression construct and selecting for ES cells carrying the expression construct. The ES cells may subsequently processed according one or more of the following steps:
- the ES cells obtained in steps are injected into blastocysts; and/or
- transgenic non-human animals carrying the expression construct are generated (viz. by well known breeding procedures).

The invention is described in more detail in the following examples which are, however, not to be construed as limiting the invention.

### Examples

### Material and Methods

Plasmid construction: All plasmid constructs (construct 1, 2, 3a, 3b, 4, 5, 6, and 7, corresponding to SEQ ID NOs: 4, 6, 8, 10, 12, 14, 16 and 18) were generated by using standard DNA cloning methods. Modification of the H1-Promoter construct (SEQ ID NO: 1) was achieved either by in-fusion (Clontech), PCR amplification or oligonucleotide synthesis (Sloning). The PIE (promoter inhibitory element) consists of the AsRed (SEQ ID NO: 2) or ZsGreen coding region (SEQ ID NO: 3) under the control of the ubiquitin promoter. To test the activity of shRNA constructs after Cre-mediated recombination, the constructs were introduced into the Cre expressing *E.coli* strain 294-Cre for deletion of PIE (Buchholz et al., Nucleic Acids Res 24: 3118-3119 (1996)) resulting in constructs having SEQ ID NOs: 5, 7, 9, 11, 13, 15, 17 and 19, respectively.

Cell culture: Culture of embryonic stem (ES) cells were carried out as described in Hogan, B. et al. ('A Laboratory Manual'. In: Manipulating the Mouse Embryo, Cold Spring Harbor Laboratory Press, Cold Spring Harbor NY, pp. 253-289 (1994)). Targeted integration of shRNA constructs into the Rosa26 locus through RMCE was performed as described (Seibler J. et al. Nucleic Acids Res., 35:e54 (2007)). The ES cell line *Rosa26*(RMCE) is described in Seibler J. et al. (Nucleic Acids Res., 33:e67 (2005)).

Luciferase / β-Galactosidase measurement: Cell were incubated at 4°C in lysis buffer (0.1 M KH2PO4, 1 mM DTT, 0.1% Triton^{®} X-100) and centrifuged for 5' at 2000 g (4°C). Supernatant was assayed for Fluc activities using the Dual-Glo Luciferase Assay (Promega, Inc.) according to the manufacturer's protocol. The luminescence was detected using a Lumat LB 9507 (EG&G Berthold). Relative values of Fluc activity in different organs are given as indicated. All values of Fluc activity were normalized by using the Rluc activity for reference. β-Galactosidase activtity was detected using the β-Galactosidase Reporter Gene Assay Kit (Roche Diagnostics) and a Wallac 1420 Multilabel counter (PerkinElmer) according to the manufacturer's protocol.

Analysis of transfected firefly luciferase expressing 226-ES-cells: 10 days after transfection, the cells were incubated in 100 µl of lysate reagent according to the manufacturers protocol (β-Galactosidase Reporter Gene Assay Kit, Roche Diagnostics) and shaked for 20 minutes at room temperature. 25 µl lysate were mixed with 50 µl of substrat reagent and 25 µl of initiating solution (β-Galactosidase Reporter Gene Assay Kit, Roche Diagnostics) and β-Galactosidase activity was determined using a Wallac 1420 Multilabel counter (PerkinElmer). To normalize β-Galactosidase activities, 35 µl lysate was added to 35 µl Luciferase Reagent solution (Dual-Glo Luciferase Assay system, Promega) and shaked for 10 minutes at room temperature before detecting luciferase activity using the Wallac 1420 Multilabel counter (PerkinElmer).

Discussion: To establish a system for Cre-inducible activation of shRNA constructs, a 'promoter inhibitory element' (PIE) consisting of the AsRed or ZsGreen gene under the control of the ubiquitin promoter was generated. A IoxP-flanked version of PIE was inserted at different positions of the H1RNA gene promoter, driving the expression of a lacZ-specific shRNA (Fig. 2A). In addition, a LacZ gene under the control of the CAGGS promoter was included to provide a substrate for shRNA mediated RNAi (Figure 1). The PIE element was subsequently deleted from the shRNA constructs upon transformation of the Cre expressing E.coli strain 294-Cre (Buchholz et al., Nucleic Acids Res 24: 3118-3119 (1996)). The RMCE strategy (Seibler et al., Nucleic Acids Res. 2005 Apr 14; 33(7):e67; WO 2006/003215) was subsequently applied for targeted insertion of constructs (SEQ. ID NOs: 4-13 and 16-19; Fig. 2) into the Rosa26 locus, as depicted in Fig. 1. Upon transfection of embryonic stem (ES) cells, recombinase mediated integration of the exchange vector into the rosa26 locus was observed in >90 % of G418 resistant colonies. Successful integration of constructs was confirmed through Southern blot analysis (Fig. 1D). Measurement of β-Galactosidase activity revealed that none of the constructs showed a tight regulation of shRNA activity through Cre-mediated deletion of PIE (Fig. 3).

Further, a hybrid promoter consisting of the DSE from the human U6 snRNA gene promoter and the PSE/TATA-box of the human H1RNA gene promoter was consructed. A IoxP-flanked version of PIE was inserted at between the DSE and PSE control regions of the hybrid promoter, resulting in construct 5 (SEQ ID NO:14; Figure 2B). The PIE element was deleted from construct 5 upon transformation of the Cre expressing E.coli strain 294-Cre (Buchholz et al., Nucleic Acids Res 24: 3118-3119 (1996)) resulting in a construct having SEQ ID NO: 15. The RMCE strategy (Seibler et al., Nucleic Acids Res. 33(7): e67(2005)) was used for targeted insertion of the construct (SEQ ID NO:15; Fig. 1) into the Rosa26 locus (Fig. 1). A high level of β-Galactosidase activity was detected in the presence, but not in the absence of PIE, indicating a tight regulation of shRNA expression through Cre (Fig. 3).

### Sequence Listing (free text)

| | |
|---|---|
| SEQ ID NO:1 | MRbasic (wt H1tetOshLacZ) |
| SEQ ID NO:2 | floxed PIE(Ubi-AsRed) |
| SEQ ID NO:3 | floxed PIE(Ubi-Zsgreen) |
| SEQ ID NOs:4/5 | construct 1 before and after Cre-Rekombination |
| SEQ ID NOs:6/7 | construct 2 before and after Cre-Rekombination |
| SEQ ID NOs:8/9 | construct 3a before and after Cre-Rekombination |
| SEQ ID NOs10/11 | construct 3b before and after Cre-Rekombination |
| SEQ ID NOs:12/13 | construct 4 before and after Cre-Rekombination |
| SEQ ID NOs:14/15 | construct 5 before and after Cre-Rekombination |
| SEQ ID NOs:16/17 | construct 6 before and after Cre-Rekombination |
| SEQ ID NOs:18/19 | construct 7 before and after Cre-Rekombination |
| SEQ ID NOs:20 | DSE from human U6 snRNA gene promoter |
| SEQ ID NO:21 | DSE from the human U2 promoter |
| SEQ ID NO:22 | DSE from the mouse U2 promoter |
| SEQ ID NO:23 | DSE from the chicken U4B promoter |
| SEQ ID NO:24 | DSE from the cmv enhancer |
| SEQ ID NO:25 | DSE from the sv40 large T antigen enhancer |
| SEQ ID NO:26 | hybrid promoter |
| SEQ ID NO:27 | murine Rosa26 locus |
| SEQ ID NO:28 | Rosa26 targeting vector |
| SEQ ID NOs:29-230 | shRNA |
| SEQ ID NOs:231-233 | siRNA |

## Claims

1. A hybrid H1RNA gene promoter comprising a proximal sequence element (PSE) derived from a H1RNA gene promoter and a heterologuous distal sequence element (DSE), which is derived from a promoter differing from the H1RNA gene promoter.

2. The hybrid promoter of claim 1, wherein the PSE is composed of functional elements recognizing transcriptional activators, preferably is derived from the human, mouse or rat H1RNA gene promoter, most preferably is the human H1RNA PSE consisting of nucleotides 4989-5006 of SEQ ID NO:14.

3. The hybrid promoter of claim 1 or 2 further comprising a TATA box, said TATA box being derived from the H1RNA gene promoter or from any other heterologous promoter, preferably the TATA box is derived from the human H1RNA gene promoter.

4. The hybrid promoter of any one of claims 1 to 3, wherein the heterologous DSE recruits a stable protein complex, preferably is derived from tissue specific or ubiquitous, constitutive or inducible RNA Polymerase I, II or III dependent promoters, most preferably is derived from RNA polymerase III dependent promoters

5. The hybrid promoter of claim 4, wherein the heterologous DSE is derived from the human U6 snRNA gene promoter (SEQ ID NO:20), the human U2 promoter (SEQ ID NO:21), the mouse U2 promoter (SEQ ID NO:22), the *Xenopus* U2 promoter, the rat U2 promoter, the chicken U4B promoter (SEQ ID NO:23), human U4C, Trsp, cmv enhancer (SEQ ID NO:24), sv40 large T antigen enhancer (SEQ ID NO:25), etc., preferably is derived from the human U6 snRNA gene promoter, most preferably is the human U6 snRNA gene promoter DSE having SEQ ID NO:20.

6. The hybrid promoter of claim 1, which
(i) has the structure: 5'- DSE - PSE - TATA box -3',
wherein the terms are as defined in claims 2 to 5; and/or
(ii) is a hybrid promoter comprising a human U6 snRNA gene promoter DSE and a human H1RNA PSE, preferably comprises a sequence having SEQ ID NO:26; and/or
(iii) optionally contains further functional elements including operator sequences, enhancer elements, transcription factor binding sites, entire gene expression cassettes, promoter inactivation elements, etc.

7. The hybrid promoter of any one of claims 1 to 6 further comprising a promoter inactivation element (PIE), preferably the PIE is an excisable inactivation sequence that is located between the PSE and DSE of the hybrid promoter.

8. The hybrid promoter of claim 7, wherein the excisable inactivation sequence is flanked by recombinase recognition sequences (RRSs) allowing conditional excision by the respective recombinase, preferably
(i) the RRSs are selected from IoxP sites, frt sites , att sites, etc; and/or
(ii) the inactivation sequence is an arbitrary non-functional or functional sequence including marker sequences, polyA sequences, splice-acceptor sequences, recombinase genes etc.,
most preferably the PIE is the IoxP-flanked Ubi-AsRed or Ubi-Zsgreen sequence shown in SEQ ID NOs: 2 and 3, respectively.

9. An expression construct comprising a short nucleic acid sequence under control of a hybrid promoter as defined in claims 1 to 8.

10. The expression construct of claim 9, wherein the short nucleic acid sequence is selected from shRNA, DNA/RNA, miRNA, cDNA, genomic DNA, etc., preferably is shRNA or miRNA.

11. The expression construct of claim 9 or 10, which is suitable for
(i) recombinase mediated cassette exchange (RMCE) and preferably comprises recombinase recognition sites that do not interfere with the RRSs of the hybrid promoter and allow RMCE; or
(ii) homologous recombination and preferably comprises sequences that are homologuous to the sequences flanking the target site; or
(iii) random integration;
the constructs of (i), (ii) and (iii) may further contain functional sequences, preferably selected from selection markers, polyadenylation sites, IRES, gene expression cassettes, additional RNAi vectors, etc.

12. A biological entity comprising the hybrid promoter of claims 1 to 8 and/or the expression construct of claims 9 or 11.

13. The biological entity of claim 12, wherein
(i) the hybrid promoter and/or the expression construct has been integrated into the genome of the biological entity; and/or
(ii) the biological entity is selected from non-human vertebrates, invertebrates, tissue culture of non-human vertebrates and invertebrates, cell culture of vertebrate and invertebrate cells, cell culture of prokaryotic and lower eukaryotic cells, preferably the biological entity is a non-human mammal or a cell culture of mammalian cells, most preferably the mammal is a mouse or rat; and/or
(iii) the biological entity further comprises an inducer construct providing for the expression of a protein which serves for excision of the PIE of the hybrid promoter; and/or
(iv) the biological entity is suitable as test system, preferably for the detection and evaluation of gene function, drug targets and pharmaceutics.

14. A method for the inducible and/or tissue specific expression of a nucleic acid sequence in a biological entity which comprises transforming/transfecting a starting biological entity with the expression construct as defined in claims 9 to 11.

15. The method of claim 14, wherein the biological entity is a non-human mammal, preferably a mouse or rat, and the method comprises transforming an ES cell with the expression construct.


SEQUENCE LISTING

<110>  TaconicArtemis

<120>  Hybrid H1 Promoter for shRNA Expression

<130>  080453ep

<160>  233

<170>  PatentIn version 3.3

<210>  1
<211>  5787
<212>  DNA
<213>  Artificial

<220>
<223>  H1tetOshLacZ promoter

<400>  1
ttaatgcgcc gctacagggc gcgtcccatt cgccattcag gctgcgcaac tgttgggaag      60

ggcgatcggt gcgggcctct tcgctattac gccagctggc gaaaggggga tgtgctgcaa     120

ggcgattaag ttgggtaacg ccagggtttt cccagtcacg acgttgtaaa acgacggcca     180

gtgaattgta atacgactca ctatagggcg aattgggggt aactaagtaa ggatcgagat     240

ccaggcgcgg atcaataaaa gatcattatt ttcaatagat ctgtgtgttg gttttttgtg     300

tgccttgggg gagggggagg ccagaatgag gcgcggccaa gggggagggg gaggccagaa     360

tgaccttggg ggagggggag gccagaatga ccttggggga gggggaggcc agaatgaggc     420

gcggatccgg agaagttcct attccgaagt tcctattctt caaatagtat aggaacttcg     480

ctcgagggat cggccattga acaagatgga ttgcacgcag gttctccggc cgcttgggtg     540

gagaggctat tcggctatga ctgggcacaa cagacaatcg gctgctctga tgccgccgtg     600

ttccggctgt cagcgcaggg gcgcccggtt ctttttgtca agaccgacct gtccggtgcc     660

ctgaatgaac tgcaggacga ggcagcgcgg ctatcgtggc tggccacgac gggcgttcct     720

tgcgcagctg tgctcgacgt tgtcactgaa gcgggaaggg actggctgct attgggcgaa     780

gtgccggggc aggatctcct gtcatctcac cttgctcctg ccgagaaagt atccatcatg     840

gctgatgcaa tgcggcggct gcatacgctt gatccggcta cctgcccatt cgaccaccaa     900

gcgaaacatc gcatcgagcg agcacgtact cggatggaag ccggtcttgt cgatcaggat     960

gatctggacg aagagcatca ggggctcgcg ccagccgaac tgttcgccag gctcaaggcg    1020

cgcatgcccg acggcgagga tctcgtcgtg acccatggcg atgcctgctt gccgaatatc    1080

atggtggaaa atggccgctt ttctggattc atcgactgtg gccggctggg tgtggcggac    1140

cgctatcagg acatagcgtt ggctacccgt gatattgctg aagagcttgg cggcgaatgg    1200

gctgaccgct tcctcgtgct ttacggtatc gccgctcccg attcgcagcg catcgccttc    1260

tatcgccttc ttgacgagtt cttctgaggg gatcgatccg ctgtaagtct gcagaaattg 1320
atgatctatt aaacaataaa gatgtccact aaaatggaag tttttcctgt catactttgt 1380
taagaagggt gagaacagag tacctacatt ttgaatggaa ggattggagc tacgggggtg 1440
ggggtggggt gggattagat aaatgcctgc tctttactga aggctcttta ctattgcttt 1500
atgataatgt ttcatagttg gatatcataa tttaaacaag caaaaccaaa ttaagggcca 1560
gctcattcct cccactcatg atctatagat ctatagatct ctcgtgggat cattgttttt 1620
ctcttgattc ccactttgtg gttctaagta ctgtggtttc caaatgtgtc agtttcatag 1680
cctgaagaac gagatcagca gcctctgttc cacatacact tcattctcag tattgttttg 1740
ccaagttcta attccatcag aagctgactc tagatggcgc gtatgcatta attaaggcca 1800
gggatcttca agcagaccta cagcaagttc gacacaaact cacacaacga tgacgcacta 1860
ctcaagaact acgggctgct ctactgcttc aggaaggaca tggacaaggt cgagacattc 1920
ctgcgcatcg tgcagtgccg ctctgtggag ggcagctgtg gcttctagct gcccgggtgg 1980
catccctgtg acccctcccc agtgcctctc ctggccctgg aagttgccac tccagtgccc 2040
accagccttg tcctaataaa attaagttgc atcattttgt ctgactaggt gtccttctat 2100
aatattatgg ggtggagggg ggtggtatgg agcaaggggc aagttgggaa gacaacctgt 2160
agggcctgcg gggtctattg ggaaccaagc tggagtgcag tggcacaatc ttggctcact 2220
gcaatctccg cctcctgggt tcaagcgatt ctcctgcctc agcctcccga gttgttggga 2280
ttccaggcat gcatgaccag gctcagctaa tttttgtttt tttggtagag acggggtttc 2340
accatattgg ccaggctggt ctccaactcc taatctcagg tgatctaccc accttggcct 2400
cccaaattgc tgggattaca ggcgtgaacc actgctccct tccctgtcct tctgatttta 2460
aaataactat accagcagga ggacgtccag acacagcata ggctacctgg ccatgcccaa 2520
ccggtgggac atttgagttg cttgcttggc actgtcctct catgcgttgg gtccactcag 2580
tagatgcctg ttgaattaag cttatttaaa taggccggcc agatctgtcg acaattggat 2640
cctcacagta ggtggcatcg ttcctttctg actgcccgcc ccccgcatgc cgtcccgcga 2700
tattgagctc cgaacctctc gccctgccgc cgccggtgct ccgtcgccgc cgcgccgcca 2760
tggaattcga acgctgacgt catcaacccg ctccaaggaa tcgcgggccc agtgtcacta 2820
ggcgggaaca cccagcgcgc gtgcgccctg gcaggaagat ggctgtgagg gacaggggag 2880
tggcgccctg caatatttgc atgtcgctat gtgttctggg aaatcaccat aaacgtgaaa 2940
tgtctttgga tttgggaatc ttataagtcc ctatcagtga tagagattcc cagtggatgg 3000
agccgatatt ggattcaaga gattcaatat tggcttcatc cactttttgg cgcgtgacac 3060
tagttatgcc cgggcatagg cgcgccggta accgaagttc ctatactttc tagagaatag 3120
gaacttcgga ataggaactt caagccggta cccagctttt gttcccttta gtgagggtta 3180

atttcgagct tggcgtaatc atggtcatag ctgtttcctg tgtgaaattg ttatccgctc 3240

acaattccac acaacatacg agccggaagc ataaagtgta aagcctgggg tgcctaatga 3300

gtgagctaac tcacattaat tgcgttgcgc tcactgcccg ctttccagtc gggaaacctg 3360

tcgtgccagc tgcattaatg aatcggccaa cgcgcgggga gaggcggttt gcgtattggg 3420

cgctcttccg cttcctcgct cactgactcg ctgcgctcgg tcgttcggct gcggcgagcg 3480

gtatcagctc actcaaaggc ggtaatacgg ttatccacag aatcagggga taacgcagga 3540

aagaacatgt gagcaaaagg ccagcaaaag gccaggaacc gtaaaaaggc cgcgttgctg 3600

gcgtttttcc ataggctccg cccccctgac gagcatcaca aaaatcgacg ctcaagtcag 3660

aggtggcgaa acccgacagg actataaaga taccaggcgt ttccccctgg aagctccctc 3720

gtgcgctctc ctgttccgac cctgccgctt accggatacc tgtccgcctt tctcccttcg 3780

ggaagcgtgg cgctttctca tagctcacgc tgtaggtatc tcagttcggt gtaggtcgtt 3840

cgctccaagc tgggctgtgt gcacgaaccc cccgttcagc ccgaccgctg cgccttatcc 3900

ggtaactatc gtcttgagtc caacccggta agacacgact tatcgccact ggcagcagcc 3960

actggtaaca ggattagcag agcgaggtat gtaggcggtg ctacagagtt cttgaagtgg 4020

tggcctaact acggctacac tagaaggaca gtatttggta tctgcgctct gctgaagcca 4080

gttaccttcg gaaaaagagt tggtagctct tgatccggca aacaaaccac cgctggtagc 4140

ggtggttttt ttgtttgcaa gcagcagatt acgcgcagaa aaaaaggatc tcaagaagat 4200

cctttgatct tttctacggg gtctgacgct cagtggaacg aaaactcacg ttaagggatt 4260

ttggtcatga gattatcaaa aaggatcttc acctagatcc ttttaaatta aaaatgaagt 4320

tttaaatcaa tctaaagtat atatgagtaa acttggtctg acagttacca atgcttaatc 4380

agtgaggcac ctatctcagc gatctgtcta tttcgttcat ccatagttgc ctgactcccc 4440

gtcgtgtaga taactacgat acgggagggc ttaccatctg gccccagtgc tgcaatgata 4500

ccgcgagacc cacgctcacc ggctccagat ttatcagcaa taaaccagcc agccggaagg 4560

gccgagcgca gaagtggtcc tgcaacttta tccgcctcca tccagtctat taattgttgc 4620

cgggaagcta gagtaagtag ttcgccagtt aatagtttgc gcaacgttgt tgccattgct 4680

acaggcatcg tggtgtcacg ctcgtcgttt ggtatggctt cattcagctc cggttcccaa 4740

cgatcaaggc gagttacatg atcccccatg ttgtgcaaaa aagcggttag ctccttcggt 4800

cctccgatcg ttgtcagaag taagttggcc gcagtgttat cactcatggt tatggcagca 4860

ctgcataatt ctcttactgt catgccatcc gtaagatgct tttctgtgac tggtgagtac 4920

tcaaccaagt cattctgaga atagtgtatg cggcgaccga gttgctcttg cccggcgtca 4980

atacgggata ataccgcgcc acatagcaga actttaaaag tgctcatcat tggaaaacgt 5040

tcttcggggc gaaaactctc aaggatctta ccgctgttga gatccagttc gatgtaaccc 5100

actcgtgcac ccaactgatc ttcagcatct tttactttca ccagcgtttc tgggtgagca 5160

aaaacaggaa ggcaaaatgc cgcaaaaaag ggaataaggg cgacacggaa atgttgaata 5220

ctcatactct tcctttttca atattattga agcatttatc agggttattg tctcatgagc 5280

ggatacatat ttgaatgtat ttagaaaaat aaacaaatag gggttccgcg cacatttccc 5340

cgaaaagtgc cacctaaatt gtaagcgtta atattttgtt aaaattcgcg ttaaattttt 5400

gttaaatcag ctcatttttt aaccaatagg ccgaaatcgg caaaatccct tataaatcaa 5460

aagaatagac cgagataggg ttgagtgttg ttccagtttg gaacaagagt ccactattaa 5520

agaacgtgga ctccaacgtc aaagggcgaa aaaccgtcta tcagggcgat ggcccactac 5580

gtgaaccatc accctaatca agttttttgg ggtcgaggtg ccgtaaagca ctaaatcgga 5640

accctaaagg gagcccccga tttagagctt gacggggaaa gccggcgaac gtggcgagaa 5700

aggaagggaa gaaagcgaaa ggagcgggcg ctagggcgct ggcaagtgta gcggtcacgc 5760

tgcgcgtaac caccacaccc gccgcgc 5787

<210> 2
<211> 2403
<212> DNA
<213> Artificial

<220>
<223> floxed PIE

<400> 2
ataacttcgt ataatgtatg ctatacgaag ttatgcgtaa tacgactcac tatagggcga 60

attggagctc caccgcggcc tgcaggggcc ggccatttaa atagttacgc tagggataac 120

agggtaaata gttaattaac ccgtgtcggc tccagatctg gcctccgcgc cgggttttgg 180

cgcctcccgc gggcgccccc ctcctcacgg cgagcgctgc cacgtcagac gaagggcgca 240

gcgagcgtcc tgatccttcc gcccggacgc tcaggacagc ggcccgctgc tcataagact 300

cggccttaga accccagtat cagcagaagg acattttagg acgggacttg ggtgactcta 360

gggcactggt ttctttccca gagagcggaa caggcgagga aaagtagtcc cttctcggcg 420

attctgcgga gggatctccg tggggcggtg aacgccgatg attatataag gacgcgccgg 480

gtgtggcaca gctagttccg tcgcagccgg gatttgggtc gcggttcttg tttgtggatc 540

gctgtgatcg tcacttggtg agtagcgggc tgctgggctg gccggggctt tcgtggccgc 600

cgggccgctc ggtgggacgg aagcgtgtgg agagaccgcc aagggctgta gtctgggtcc 660

gcgagcaagg ttgccctgaa ctgggggttg gggggagcgc agcaaaatgg cggctgttcc 720

cgagtcttga atggaagacg cttgtgaggc gggctgtgag gtcgttgaaa caaggtgggg 780

ggcatggtgg gcggcaagaa cccaaggtct tgaggccttc gctaatgcgg gaaagctctt 840

attcgggtga gatgggctgg ggcaccatct ggggaccctg acgtgaagtt tgtcactgac 900

tggagaactc ggtttgtcgt ctgttgcggg ggcggcagtt atggcggtgc cgttgggcag 960

tgcacccgta cctttgggag cgcgcgccct cgtcgtgtcg tgacgtcacc cgttctgttg 1020

gcttataatg cagggtgggg ccacctgccg gtaggtgtgc ggtaggcttt tctccgtcgc 1080

aggacgcagg gttcgggcct agggtaggct ctcctgaatc gacaggcgcc ggacctctgg 1140

tgaggggagg gataagtgag gcgtcagttt ctttggtcgg ttttatgtac ctatcttctt 1200

aagtagctga agctccggtt ttgaactatg cgctcggggt tggcgagtgt gttttgtgaa 1260

gttttttagg caccttttga aatgtaatca tttgggtcaa tatgtaattt tcagtgttag 1320

actagtaaat tgtccgctaa attctggccg tttttggctt ttttgttaga cgaagcttgg 1380

gctgcaggtc gactctagag gatccccggg taccggtcgc caccatggcc tctttgctga 1440

agaagaccat gcccttcagg accaccatcg agggcaccgt gaacggccac tacttcaagt 1500

gcaccggcaa gggcgagggc aaccccctgg agggcaccca ggagatgaag atcgaggtga 1560

tcgagggcgg ccccctgccc ttcgccttcc acatcctgtc cacctcctgc atgtacggct 1620

ccaaggcctt catcaagtac gtgtccggca tccccgacta cttcaagcag tccctccccg 1680

agggcttcac ctgggagcgc accaccacct acgaggacgg cggcttcctg accgcccacc 1740

aggacacctc cctggacggc gactgcctgg tgtacaaggt gaagatcctg ggcaacaact 1800

tccccgccga cggccccgtg atgcagaaca aggccggccg ctgggagccc tccaccgaga 1860

tcgtgtacga ggtggacggc gtgctgcgcg gccagtccct gatggccctg gagtgccccg 1920

gcggtcgcca cctgacctgc cacctgcaca ccacctaccg ctccaagaag cccgcctccg 1980

ccctgaagat gcccggcttc cacttcgagg accaccgcat cgagatcctg gaggaggtgg 2040

agaagggcaa gtgctacaag cagtacgagg ccgccgtggg ccgctactgc gacgccgccc 2100

cctccaagct gggccacaac tgaagcggcc gcgactctag aattggccgc gtcgacctcg 2160

agatccaggc gcggatcaat aaaagatcat tattttcaat agatctgtgt gttggttttt 2220

tgtgtgcctt gggggagggg gaggccagaa tgaggcgcgg ccaaggggga gggggaggcc 2280

agaatgacct tgggggaggg ggaggccaga atgaccttgg gggaggggga ggccagaatg 2340

aggcgcgcca gttacgctag ggataacaga taacttcgta taatgtatgc tatacgaagt 2400

tat 2403

<210> 3
<211> 2403
<212> DNA
<213> Artificial

<220>
<223> floxed FIE

<400>  3
ataacttcgt ataatgtatg ctatacgaag ttatgcgtaa tacgactcac tatagggcga      60

attggagctc caccgcggcc tgcaggggcc ggccatttaa atagttacgc tagggataac     120

agggtaaata gttaattaac ccgtgtcggc tccagatctg gcctccgcgc cgggttttgg     180

cgcctcccgc gggcgccccc ctcctcacgg cgagcgctgc cacgtcagac gaagggcgca     240

gcgagcgtcc tgatccttcc gcccggacgc tcaggacagc ggcccgctgc tcataagact     300

cggccttaga accccagtat cagcagaagg acattttagg acgggacttg ggtgactcta     360

gggcactggt tttctttcca gagagcggaa caggcgagga aaagtagtcc cttctcggcg     420

attctgcgga gggatctccg tggggcggtg aacgccgatg attatataag gacgcgccgg     480

gtgtggcaca gctagttccg tcgcagccgg gatttgggtc gcggttcttg tttgtggatc     540

gctgtgatcg tcacttggtg agtagcgggc tgctgggctg gccggggctt tcgtggccgc     600

cgggccgctc ggtgggacgg aagcgtgtgg agagaccgcc aagggctgta gtctgggtcc     660

gcgagcaagg ttgccctgaa ctgggggttg gggggagcgc agcaaaatgg cggctgttcc     720

cgagtcttga atggaagacg cttgtgaggc gggctgtgag gtcgttgaaa caaggtgggg     780

ggcatggtgg gcggcaagaa cccaaggtct tgaggccttc gctaatgcgg gaaagctctt     840

attcgggtga gatgggctgg ggcaccatct ggggaccctg acgtgaagtt tgtcactgac     900

tggagaactc ggtttgtcgt ctgttgcggg ggcggcagtt atggcggtgc cgttgggcag     960

tgcacccgta cctttgggag cgcgcgccct cgtcgtgtcg tgacgtcacc cgttctgttg    1020

gcttataatg caggtggggc ccacctgccg gtaggtgtgc ggtaggcttt tctccgtcgc    1080

aggacgcagg gttcgggcct agggtaggct ctcctgaatc gacaggcgcc ggacctctgg    1140

tgaggggagg gataagtgag gcgtcagttt ctttggtcgg ttttatgtac ctatcttctt    1200

aagtagctga agctccggtt ttgaactatg cgctcggggt tggcgagtgt gttttgtgaa    1260

gttttttagg caccttttga aatgtaatca tttgggtcaa tatgtaattt tcagtgttag    1320

actagtaaat tgtccgctaa attctggccg tttttggctt ttttgttaga cgaagcttgg    1380

gctgcaggtc gactctagag gatccccggg taccggtcgc caccatggcc tctttgctga    1440

agaagaccat gcccttcagg accaccatcg agggcaccgt gaacggccac tacttcaagt    1500

gcaccggcaa gggcgagggc aaccccctgg agggcaccca ggagatgaag atcgaggtga    1560

tcgagggcgg ccccctgccc ttcgccttcc acatcctgtc cacctcctgc atgtacggct    1620

ccaaggcctt catcaagtac gtgtccggca tccccgacta cttcaagcag tccctccccg    1680

agggcttcac ctgggagcgc accaccacct acgaggacgg cggcttcctg accgcccacc    1740

aggacacctc cctggacggc gactgcctgg tgtacaaggt gaagatcctg ggcaacaact    1800

tccccgccga cggccccgtg atgcagaaca aggccggccg ctgggagccc tccaccgaga    1860

tcgtgtacga ggtggacggc gtgctgcgcg gccagtccct gatggccctg gagtgccccg 1920

gcggtcgcca cctgacctgc cacctgcaca ccacctaccg ctccaagaag cccgcctccg 1980

ccctgaagat gcccggcttc cacttcgagg accaccgcat cgagatcctg gaggaggtgg 2040

agaagggcaa gtgctacaag cagtacgagg ccgccgtggg ccgctactgc gacgccgccc 2100

cctccaagct gggccacaac tgaagcggcc gcgactctag aattggccgc gtcgacctcg 2160

agatccaggc gcggatcaat aaaagatcat tattttcaat agatctgtgt gttggttttt 2220

tgtgtgcctt gggggagggg gaggccagaa tgaggcgcgg ccaaggggga gggggaggcc 2280

agaatgacct tgggggaggg ggaggccaga atgaccttgg gggaggggga ggccagaatg 2340

aggcgcgcca gttacgctag ggataacaga taacttcgta taatgtatgc tatacgaagt 2400

tat 2403

<210> 4
<211> 2392
<212> DNA
<213> Artificial

<220>
<223> construct 1 before Cre Recombination

<400> 4
atttgcatgt cgctatgtgt tctgggaaat caccataaac gtgaaataac ttcgtataat 60

gtatgctata cgaagttatt ttttgcgtta attaagtgcg attaagggtg cagcggcctc 120

cgcgccgggt tttggcgcct cccgcgggcg cccccctcct cacggcgagc gctgccacgt 180

cagacgaagg gcgcaggagc gttcctgatc cttccgcccg gacgctcagg acagcggccc 240

gctgctcata agactcggcc ttagaacccc agtatcagca gaaggacatt ttaggacggg 300

acttgggtga ctctagggca ctggttttct ttccagagag cggaacaggc gaggaaaagt 360

agtcccttct cggcgattct gcggagggat ctccgtgggg cggtgaacgc cgatgattat 420

ataaggacgc gccgggtgtg gcacagctag ttccgtcgca gccgggattt gggtcgcggt 480

tcttgtttgt ggatcgctgt gatcgtcact tggtgagttg cgggctgctg ggctggccgg 540

ggctttcgtg gccgccgggc cgctcggtgg gacggaagcg tgtggagaga ccgccaaggg 600

ctgtagtctg ggtccgcgag caaggttgcc ctgaactggg ggttgggggg agcgcacaaa 660

atggcggctg ttcccgagtc ttgaatggaa gacgcttgta aggcgggctg tgaggtcgtt 720

gaaacaaggt ggggggcatg gtgggcggca agaacccaag gtcttgaggc cttcgctaat 780

gcgggaaagc tcttattcgg gtgagatggg ctggggcacc atctggggac cctgacgtga 840

agtttgtcac tgactggaga actcgggttt gtcgtctggt tgcgggggcg gcagttatgc 900

ggtgccgttg ggcagtgcac ccgtaccttt gggagcgcgc gcctcgtcgt gtcgtgacgt 960

cacccgttct gttggcttat aatgcagggt ggggccacct gccggtaggt gtgcggtagg 1020

cttttctccg tcgcaggacg cagggttcgg gcctagggta ggctctcctg aatcgacagg 1080

cgccggacct ctggtgaggg gagggataag tgaggcgtca gtttctttgg tcggttttat 1140

gtacctatct tcttaagtag ctgaagctcc ggttttgaac tatgcgctcg gggttggcga 1200

gtgtgttttg tgaagttttt taggcacctt ttgaaatgta atcatttggg tcaatatgta 1260

attttcagtg ttagactagt aaagcttctg caggtcgact ctagaaaatt gtccgctaaa 1320

ttctggccgt ttttggcttt tttgttagac aggatcctgc actagtcggc tagctgtccg 1380

gtcgccacca tggcccagtc caagcacggc ctgaccaagg agatgaccat gaagtaccgc 1440

atggagggct gcgtggacgg ccacaagttc gtgatcaccg gcgagggcat cggctacccc 1500

ttcaagggca agcaggccat caacctgtgc gtggtggagg gcggcccctt gcccttcgcc 1560

gaggacatct tgtccgccgc cttcatgtac ggcaaccgcg tgttcaccga gtacccccag 1620

gacatcgtcg actacttcaa gaactcctgc cccgccggct acacctggga ccgctccttc 1680

ctgttcgagg acggcgccgt gtgcatctgc aacgccgaca tcaccgtgag cgtggaggag 1740

aactgcatgt accacgagtc caagttctac ggcgtgaact tccccgccga cggccccgtg 1800

atgaagaaga tgaccgacaa ctgggagccc tcctgcgaga agatcatccc cgtgcccaag 1860

cagggcatct tgaagggcga cgtgagcatg tacctgctgc tgaaggacgg tggccgcttg 1920

cgctgccagt tcgacaccgt gtacaaggcc aagtccgtgc cccgcaagat gcccgactgg 1980

cacttcatcc agcacaagct gacccgcgag gaccgcagcg acgccaagaa ccagaagtgg 2040

cacctgaccg agcacgccat cgcctccggc tccgccttgc cctgagcggc cgaccggaca 2100

gcggccgcag tcctcaggat catatgctac gcgtgacaat tggcggcccg attctagacc 2160

ggttcgagat ccaggcgcgg atcaataaaa gatcattatt ttcaatagat ctgtgtgttg 2220

gttttttgtg tgccttgggg gagggggagg ccagaatgag gcgcggccaa gggggagggg 2280

gaggccagaa tgaccttggg ggagggggag gccagaatga ccttggggga gggggaggcc 2340

agaatgaggc gcgtcgataa cttcgtataa tgtatgctat acgaagttat aa 2392

<210> 5
<211> 81
<212> DNA
<213> Artificial

<220>
<223> construct 1 after Cre Recombination

<400> 5
atttgcatgt cgctatgtgt tctgggaaat caccataaac gtgaaataac ttcgtataat 60

gtatgctata cgaagttata a 81

<210> 6
<211> 2413
<212> DNA
<213> Artificial

<220>
<223> construct 2 before Cre Recombination

<400> 6
atttgcatgt cgctatgtgt tctgggaaat caccataaac gtgaaatgtc ttataacttc 60

gtataatgta tgctatacga agttattttt tgcgttaatt aagtgcgatt aagggtgcag 120

cggcctccgc gccgggtttt ggcgcctccc gcgggcgccc ccctcctcac ggcgagcgct 180

gccacgtcag acgaagggcg caggagcgtt cctgatcctt ccgcccggac gctcaggaca 240

gcggcccgct gctcataaga ctcggcctta gaaccccagt atcagcagaa ggacatttta 300

ggacgggact tgggtgactc tagggcactg gttttctttc cagagagcgg aacaggcgag 360

gaaaagtagt cccttctcgg cgattctgcg gagggatctc cgtggggcgg tgaacgccga 420

tgattatata aggacgcgcc gggtgtggca cagctagttc cgtcgcagcc gggatttggg 480

tcgcggttct tgtttgtgga tcgctgtgat cgtcacttgg tgagttgcgg gctgctgggc 540

tggccggggc tttcgtggcc gccgggccgc tcggtgggac ggaagcgtgt ggagagaccg 600

ccaagggctg tagtctgggt ccgcgagcaa ggttgccctg aactgggggt tggggggagc 660

gcacaaaatg gcggctgttc ccgagtcttg aatggaagac gcttgtaagg cgggctgtga 720

ggtcgttgaa acaaggtggg gggcatggtg ggcggcaaga acccaaggtc ttgaggcctt 780

cgctaatgcg ggaaagctct tattcgggtg agatgggctg gggcaccatc tggggaccct 840

gacgtgaagt ttgtcactga ctggagaact cgggtttgtc gtctggttgc gggggcggca 900

gttatgcggt gccgttgggc agtgcacccg tacctttggg agcgcgcgcc tcgtcgtgtc 960

gtgacgtcac ccgttctgtt ggcttataat gcagggtggg gccacctgcc ggtaggtgtg 1020

cggtaggctt ttctccgtcg caggacgcag ggttcgggcc tagggtaggc tctcctgaat 1080

cgacaggcgc cggacctctg gtgaggggag ggataagtga ggcgtcagtt tctttggtcg 1140

gttttatgta cctatcttct taagtagctg aagctccggt tttgaactat gcgctcgggg 1200

ttggcgagtg tgttttgtga agttttttag gcaccttttg aaatgtaatc atttgggtca 1260

atatgtaatt ttcagtgtta gactagtaaa gcttctgcag gtcgactcta gaaaattgtc 1320

cgctaaattc tggccgtttt tggctttttt gttagacagg atcctgcact agtcggctag 1380

ctgtccggtc gccaccatgg cccagtccaa gcacggcctg accaaggaga tgaccatgaa 1440

gtaccgcatg gagggctgcg tggacggcca caagttcgtg atcaccggcg agggcatcgg 1500

ctaccccttc aagggcaagc aggccatcaa cctgtgcgtg gtggagggcg gccccttgcc 1560

cttcgccgag gacatcttgt ccgccgcctt catgtacggc aaccgcgtgt tcaccgagta 1620

cccccaggac atcgtcgact acttcaagaa ctcctgcccc gccggctaca cctgggaccg 1680

ctccttcctg ttcgaggacg gcgccgtgtg catctgcaac gccgacatca ccgtgagcgt 1740

ggaggagaac tgcatgtacc acgagtccaa gttctacggc gtgaacttcc ccgccgacgg 1800

ccccgtgatg aagaagatga ccgacaactg ggagccctcc tgcgagaaga tcatccccgt 1860

gcccaagcag ggcatcttga agggcgacgt gagcatgtac ctgctgctga aggacggtgg 1920

ccgcttgcgc tgccagttcg acaccgtgta caaggccaag tccgtgcccc gcaagatgcc 1980

cgactggcac ttcatccagc acaagctgac ccgcgaggac cgcagcgacg ccaagaacca 2040

gaagtggcac ctgaccgagc acgccatcgc ctccggctcc gccttgccct gagcggccga 2100

ccggacagcg gccgcagtcc tcaggatcat atgctacgcg tgacaattgg cggcccgatt 2160

ctagaccggt tcgagatcca ggcgcggatc aataaaagat cattattttc aatagatctg 2220

tgtgttggtt ttttgtgtgc cttgggggag ggggaggcca gaatgaggcg cggccaaggg 2280

ggagggggag gccagaatga ccttggggga gggggaggcc agaatgacct tgggggaggg 2340

ggaggccaga atgaggcgcg tcgataactt cgtataatgt atgctatacg aagttatttt 2400

gggaatctta taa 2413

<210> 7
<211> 102
<212> DNA
<213> Artificial

<220>
<223> construct 2 after Cre Recombination

<400> 7
atttgcatgt cgctatgtgt tctgggaaat caccataaac gtgaaatgtc ttataacttc 60

gtataatgta tgctatacga agttattttg ggaatcttat aa 102

<210> 8
<211> 2385
<212> DNA
<213> Artificial

<220>
<223> construct 3a before Cre Recombination

<400> 8
atttgcatgt cgctatgtgt tctgggaaat caccataaac gtgaaattcg tataatgtat 60

gctatacgaa gttatttttt gcgttaatta agtgcgatta agggtgcagc ggcctccgcg 120

ccgggttttg gcgcctcccg cgggcgcccc cctcctcacg gcgagcgctg ccacgtcaga 180

cgaagggcgc aggagcgttc ctgatccttc cgcccggacg ctcaggacag cggcccgctg 240

ctcataagac tcggccttag aaccccagta tcagcagaag gacattttag gacgggactt 300

gggtgactct agggcactgg ttttctttcc agagagcgga acaggcgagg aaaagtagtc 360

ccttctcggc gattctgcgg agggatctcc gtggggcggt gaacgccgat gattatataa 420

ggacgcgccg ggtgtggcac agctagttcc gtcgcagccg ggatttgggt cgcggttctt 480

gtttgtggat cgctgtgatc gtcacttggt gagttgcggg ctgctgggct ggccggggct 540

ttcgtggccg ccgggccgct cggtgggacg gaagcgtgtg gagagaccgc caagggctgt 600

agtctgggtc cgcgagcaag gttgccctga actgggggtt ggggggagcg cacaaaatgg 660

cggctgttcc cgagtcttga atggaagacg cttgtaaggc gggctgtgag gtcgttgaaa 720

caaggtgggg ggcatggtgg gcggcaagaa cccaaggtct tgaggccttc gctaatgcgg 780

gaaagctctt attcgggtga gatgggctgg ggcaccatct ggggaccctg acgtgaagtt 840

tgtcactgac tggagaactc gggtttgtcg tctggttgcg ggggcggcag ttatgcggtg 900

ccgttgggca gtgcacccgt acctttggga gcgcgcgcct cgtcgtgtcg tgacgtcacc 960

cgttctgttg gcttataatg cagggtgggg ccacctgccg gtaggtgtgc ggtaggcttt 1020

tctccgtcgc aggacgcagg gttcgggcct agggtaggct ctcctgaatc gacaggcgcc 1080

ggacctctgg tgaggggagg gataagtgag gcgtcagttt ctttggtcgg ttttatgtac 1140

ctatcttctt aagtagctga agctccggtt ttgaactatg cgctcggggt tggcgagtgt 1200

gttttgtgaa gttttttagg caccttttga aatgtaatca tttgggtcaa tatgtaattt 1260

tcagtgttag actagtaaag cttctgcagg tcgactctag aaaattgtcc gctaaattct 1320

ggccgttttt ggcttttttg ttagacagga tcctgcacta gtcggctagc tgtccggtcg 1380

ccaccatggc ccagtccaag cacggcctga ccaaggagat gaccatgaag taccgcatgg 1440

agggctgcgt ggacggccac aagttcgtga tcaccggcga gggcatcggc taccccttca 1500

agggcaagca ggccatcaac ctgtgcgtgg tggagggcgg ccccttgccc ttcgccgagg 1560

acatcttgtc cgccgccttc atgtacggca accgcgtgtt caccgagtac ccccaggaca 1620

tcgtcgacta cttcaagaac tcctgccccg ccggctacac ctgggaccgc tccttcctgt 1680

tcgaggacgg cgccgtgtgc atctgcaacg ccgacatcac cgtgagcgtg gaggagaact 1740

gcatgtacca cgagtccaag ttctacggcg tgaacttccc cgccgacggc cccgtgatga 1800

agaagatgac cgacaactgg gagccctcct gcgagaagat catccccgtg cccaagcagg 1860

gcatcttgaa gggcgacgtg agcatgtacc tgctgctgaa ggacggtggc cgcttgcgct 1920

gccagttcga caccgtgtac aaggccaagt ccgtgccccg caagatgccc gactggcact 1980

tcatccagca caagctgacc cgcgaggacc gcagcgacgc caagaaccag aagtggcacc 2040

tgaccgagca cgccatcgcc tccggctccg ccttgccctg agcggccgac cggacagcgg 2100

ccgcagtcct caggatcata tgctacgcgt gacaattggc ggcccgattc tagaccggtt 2160

cgagatccag gcgcggatca ataaaagatc attattttca atagatctgt gtgttggttt 2220

tttgtgtgcc ttgggggagg gggaggccag aatgaggcgc ggccaagggg gagggggagg 2280

ccagaatgac cttgggggag ggggaggcca gaatgacctt gggggagggg gaggccagaa 2340

tgaggcgcgt cgataacttc gtataatgta tgctatacgt tataa 2385

<210> 9
<211> 74
<212> DNA
<213> Artificial

<220>
<223> construct 3a after Cre Recombination

<400> 9
atttgcatgt cgctatgtgt tctgggaaat caccataaac gtgaaattcg tataatgtat 60

gctatacgtt ataa 74

<210> 10
<211> 2572
<212> DNA
<213> Artificial

<220>
<223> construct 3b before Cre Recombination

<400> 10
aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcatg tcgctatgtg ttctgggaaa tcaccataaa cgtgaaatcg 180

tataatgtat gctatacgaa gttatgcgta atacgactca ctatagggcg aattggagct 240

ccaccgcggc ctgcaggggc cggccattta aatagttacg ctagggataa cagggtaaat 300

agttaattaa cccgtgtcgg ctccagatct ggcctccgcg ccgggttttg gcgcctcccg 360

cgggcgcccc cctcctcacg gcgagcgctg ccacgtcaga cgaagggcgc agcgagcgtc 420

ctgatccttc cgcccggacg ctcaggacag cggcccgctg ctcataagac tcggccttag 480

aaccccagta tcagcagaag gacattttag gacgggactt gggtgactct agggcactgg 540

ttttctttcc agagagcgga acaggcgagg aaaagtagtc ccttctcggc gattctgcgg 600

agggatctcc gtggggcggt gaacgccgat gattatataa ggacgcgccg ggtgtggcac 660

agctagttcc gtcgcagccg ggatttgggt cgcggttctt gtttgtggat cgctgtgatc 720

gtcacttggt gagtagcggg ctgctgggct ggccggggct ttcgtggccg ccgggccgct 780

cggtgggacg gaagcgtgtg gagagaccgc caagggctgt agtctgggtc cgcgagcaag 840

gttgccctga actgggggtt ggggggagcg cagcaaaatg gcggctgttc ccgagtcttg 900

aatggaagac gcttgtgagg cgggctgtga ggtcgttgaa acaaggtggg gggcatggtg 960

ggcggcaaga acccaaggtc ttgaggcctt cgctaatgcg ggaaagctct tattcgggtg 1020

agatgggctg gggcaccatc tggggaccct gacgtgaagt ttgtcactga ctggagaact 1080

cggtttgtcg tctgttgcgg gggcggcagt tatggcggtg ccgttgggca gtgcacccgt 1140

acctttggga gcgcgcgccc tcgtcgtgtc gtgacgtcac ccgttctgtt ggcttataat 1200

gcagggtggg gccacctgcc ggtaggtgtg cggtaggctt ttctccgtcg caggacgcag 1260

ggttcgggcc tagggtaggc tctcctgaat cgacaggcgc cggacctctg gtgaggggag 1320

ggataagtga ggcgtcagtt tctttggtcg gttttatgta cctatcttct taagtagctg 1380

aagctccggt tttgaactat gcgctcgggg ttggcgagtg tgttttgtga agttttttag 1440

gcaccttttg aaatgtaatc atttgggtca atatgtaatt ttcagtgtta gactagtaaa 1500

ttgtccgcta aattctggcc gtttttggct tttttgttag acgaagcttg ggctgcaggt 1560

cgactctaga ggatccccgg gtaccggtcg ccaccatggc ctctttgctg aagaagacca 1620

tgcccttcag gaccaccatc gagggcaccg tgaacggcca ctacttcaag tgcaccggca 1680

agggcgaggg caaccccctg gagggcaccc aggagatgaa gatcgaggtg atcgagggcg 1740

gccccctgcc cttcgccttc cacatcctgt ccacctcctg catgtacggc tccaaggcct 1800

tcatcaagta cgtgtccggc atccccgact acttcaagca gtccctcccc gagggcttca 1860

cctgggagcg caccaccacc tacgaggacg gcggcttcct gaccgcccac caggacacct 1920

ccctggacgg cgactgcctg gtgtacaagg tgaagatcct gggcaacaac ttccccgccg 1980

acggccccgt gatgcagaac aaggccggcc gctgggagcc ctccaccgag atcgtgtacg 2040

aggtggacgg cgtgctgcgc ggccagtccc tgatggccct ggagtgcccc ggcggtcgcc 2100

acctgacctg ccacctgcac accacctacc gctccaagaa gcccgcctcc gccctgaaga 2160

tgcccggctt ccacttcgag gaccaccgca tcgagatcct ggaggaggtg gagaagggca 2220

agtgctacaa gcagtacgag gccgccgtgg gccgctactg cgacgccgcc ccctccaagc 2280

tgggccacaa ctgaagcggc cgcgactcta gaattggccg cgtcgacctc gagatccagg 2340

cgcggatcaa taaaagatca ttattttcaa tagatctgtg tgttggtttt ttgtgtgcct 2400

tgggggaggg ggaggccaga atgaggcgcg gccaaggggg agggggaggc cagaatgacc 2460

ttgggggagg gggaggccag aatgaccttg ggggaggggg aggccagaat gaggcgcgcc 2520

agttacgcta gggataacag ataacttcgt ataatgtatg ctatacttat aa 2572

<210> 11
<211> 203
<212> DNA
<213> Artificial

<220>
<223> construct 3b after Cre Recombination

<400> 11

aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcatg tcgctatgtg ttctgggaaa tcaccataaa cgtgaaatcg 180

tataatgtat gctatactta taa 203

<210> 12
<211> 2572
<212> DNA
<213> Artificial

<220>
<223> construct 4 before Cre Recombination

<400> 12
aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcatg tcgctatgtg ttctgggaaa tcaccataac ttcgtataat 180

gtatgctata cgaagttatg cgtaatacga ctcactatag ggcgaattgg agctccaccg 240

cggcctgcag ggccggccca tttaaatagt tacgctaggg ataacagggt aaatagttaa 300

ttaaggtgtc ggctccagat ctggcctccg cgccgggttt tggcgcctcc cgcgggcgcc 360

cccctcctca cggcgagcgc tgccacgtca gacgaagggc gcagcgagcg tcctgatcct 420

tccgcccgga cgctcaggac agcggcccgc tgctcataag actcggcctt agaaccccag 480

tatcagcaga aggacatttt aggacgggac ttgggtgact ctagggcact ggttttcttt 540

ccagagagcg gaacaggcga ggaaaagtag tcccttctcg gcgattctgc ggagggatct 600

ccgtggggcg gtgaacgccg atgattatat aaggacgcgc cgggtgtggc acagctagtt 660

ccgtcgcagc cgggatttgg gtcgcggttc ttgtttgtgg atcgctgtga tcgtcacttg 720

gtgagtagcg ggctgctggg ctggccgggg ctttcgtggc cgccgggccg ctcggtggga 780

cggaagcgtg tggagagacc gccaagggct gtagtctggg tccgcgagca aggttgccct 840

gaactggggg ttggggggag cgcagcaaaa tggcggctgt tcccgagtct tgaatggaag 900

acgcttgtga ggcgggctgt gaggtcgttg aaacaaggtg gggggcatgg tgggcggcaa 960

gaacccaagg tcttgaggcc ttcgctaatg cgggaaagct cttattcggg tgagatgggc 1020

tggggcacca tctggggacc ctgacgtgaa gtttgtcact gactggagaa ctcggtttgt 1080

cgtctgttgc gggggcggca gttatggcgg tgccgttggg cagtgcaccc gtacctttgg 1140

gagcgcgcgc cctcgtcgtg tcgtgacgtc acccgttctg ttggcttata atgcagggtg 1200

gggccacctg ccggtaggtg tgcggtaggc ttttctccgt cgcaggacgc agggttcggg 1260

cctagggtag gctctcctga atcgacaggc gccggacctc tggtgagggg agggataagt 1320

gaggcgtcag tttctttggt cggttttatg tacctatctt cttaagtagc tgaagctccg 1380

gttttgaact atgcgctcgg ggttggcgag tgtgttttgt gaagtttttt aggcaccttt 1440

tgaaatgtaa tcatttgggt caatatgtaa ttttcagtgt tagactagta aattgtccgc 1500

taaattctgg ccgtttttgg cttttttgtt agacgaagct tgggctgcag gtcgactcta 1560

gaggatcccc gggtaccggt cgccaccatg gcctctttgc tgaagaagac catgcccttc 1620

aggaccacca tcgagggcac cgtgaacggc cactacttca agtgcaccgg caagggcgag 1680

ggcaaccccc tggagggcac ccaggagatg aagatcgagg tgatcgaggg cggccccctg 1740

cccttcgcct tccacatcct gtccacctcc tgcatgtacg gctccaaggc cttcatcaag 1800

tacgtgtccg gcatccccga ctacttcaag cagtccctcc ccgagggctt cacctgggag 1860

cgcaccacca cctacgagga cggcggcttc ctgaccgccc accaggacac ctccctggac 1920

ggcgactgcc tggtgtacaa ggtgaagatc ctgggcaaca acttccccgc cgacggcccc 1980

gtgatgcaga acaaggccgg ccgctgggag ccctccaccg agatcgtgta cgaggtggac 2040

ggcgtgctgc gcggccagtc cctgatggcc ctggagtgcc ccggcggtcg ccacctgacc 2100

tgccacctgc acaccaccta ccgctccaag aagcccgcct ccgccctgaa gatgcccggc 2160

ttccacttcg aggaccaccg catcgagatc ctggaggagg tggagaaggg caagtgctac 2220

aagcagtacg aggccgccgt gggccgctac tgcgacgccg ccccctccaa gctgggccac 2280

aacttgaagc ggccgcgact ctagaattgg ccgcgtcgac ctcgagatcc aggcgcggat 2340

caataaaaga tcattatttt caatagatct gtgtgttggt tttttgtgtg ccttggggga 2400

gggggaggcc agaatgaggc gcggccaagg gggaggggga ggccagaatg accttggggg 2460

agggggaggc cagaatgacc ttgggggagg gggaggccag aatgaggcgc gccagttacg 2520

ctagggataa cagataactt cgtataatgt atgctatacg aagttattat aa 2572

<210> 13
<211> 201
<212> DNA
<213> Artificial

<220>
<223> construct 4 after Cre Recombination

<400> 13
aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcatg tcgctatgtg ttctgggaaa tcaccataac ttcgtataat 180

gtatgctata cgaagttata a 201

<210> 14
<211> 2699
<212> DNA

<213>  Artificial

<220>
<223>  construct 5 before Cre Recombination

<400>  14
aattcgaacg ctgacgtcat caacccgctc caaggaatcg cgggcccagt gtcactaggc      60

gggaacaccc agcgcgcgtg cgccctggca ggaagatggc tgtgagggac aggggagtgg     120

cgccctgcaa tatttgcata tacgatacaa ggctgttaga gagataatta gaattaattt     180

gactgtaaac acaaagatat tagtacaaaa taataacttc gtataatgta tgctatacga     240

agttatgcgt aatacgactc actatagggc gaattggagc tccaccgcgg cctgcagggg     300

ccggccattt aaatagttac gctagggata acagggtaaa tagttaatta acccgtgtcg     360

gctccagatc tggcctccgc gccgggtttt ggcgcctccc gcgggcgccc ccctcctcac     420

ggcgagcgct gccacgtcag acgaagggcg cagcgagcgt cctgatcctt ccgcccggac     480

gctcaggaca gcggcccgct gctcataaga ctcggcctta gaaccccagt atcagcagaa     540

ggacatttta ggacgggact tgggtgactc tagggcactg gttttctttc cagagagcgg     600

aacaggcgag gaaaagtagt cccttctcgg cgattctgcg gagggatctc cgtggggcgg     660

tgaacgccga tgattatata aggacgcgcc gggtgtggca cagctagttc cgtcgcagcc     720

gggatttggg tcgcggttct tgtttgtgga tcgctgtgat cgtcacttgg tgagtagcgg     780

gctgctgggc tggccggggc tttcgtggcc gccgggccgc tcggtgggac ggaagcgtgt     840

ggagagaccg ccaagggctg tagtctgggt ccgcgagcaa ggttgccctg aactgggggt     900

tggggggagc gcagcaaaat ggcggctgtt cccgagtctt gaatggaaga cgcttgtgag     960

gcgggctgtg aggtcgttga aacaaggtgg ggggcatggt gggcggcaag aacccaaggt    1020

cttgaggcct tcgctaatgc gggaaagctc ttattcgggt gagatgggct ggggcaccat    1080

ctggggaccc tgacgtgaag tttgtcactg actggagaac tcggtttgtc gtctgttgcg    1140

ggggcggcag ttatggcggt gccgttgggc agtgcacccg tacctttggg agcgcgcgcc    1200

ctcgtcgtgt cgtgacgtca cccgttctgt tggcttataa tgcagggtgg ggccacctgc    1260

cggtaggtgt gcggtaggct tttctccgtc gcaggacgca gggttcgggc ctagggtagg    1320

ctctcctgaa tcgacaggcg ccggacctct ggtgagggga gggataagtg aggcgtcagt    1380

ttctttggtc ggttttatgt acctatcttc ttaagtagct gaagctccgg ttttgaacta    1440

tgcgctcggg gttggcgagt gtgttttgtg aagtttttta ggcacctttt gaaatgtaat    1500

catttgggtc aatatgtaat tttcagtgtt agactagtaa attgtccgct aaattctggc    1560

cgtttttggc ttttttgtta gacgaagctt gggctgcagg tcgactctag aggatccccg    1620

ggtaccggtc gccaccatgg cctctttgct gaagaagacc atgcccttca ggaccaccat    1680

cgagggcacc gtgaacggcc actacttcaa gtgcaccggc aagggcgagg gcaaccccct     1740

ggagggcacc caggagatga agatcgaggt gatcgagggc ggccccctgc ccttcgcctt 1800

ccacatcctg tccacctcct gcatgtacgg ctccaaggcc ttcatcaagt acgtgtccgg 1860

catccccgac tacttcaagc agtccctccc cgagggcttc acctgggagc gcaccaccac 1920

ctacgaggac ggcggcttcc tgaccgccca ccaggacacc tccctggacg gcgactgcct 1980

ggtgtacaag gtgaagatcc tgggcaacaa cttccccgcc gacggccccg tgatgcagaa 2040

caaggccggc cgctgggagc cctccaccga gatcgtgtac gaggtggacg gcgtgctgcg 2100

cggccagtcc ctgatggccc tggagtgccc cggcggtcgc cacctgacct gccacctgca 2160

caccacctac cgctccaaga agcccgcctc cgccctgaag atgcccggct tccacttcga 2220

ggaccaccgc atcgagatcc tggaggaggt ggagaagggc aagtgctaca agcagtacga 2280

ggccgccgtg ggccgctact gcgacgccgc cccctccaag ctgggccaca actgaagcgg 2340

ccgcgactct agaattggcc gcgtcgacct cgagatccag gcgcggatca ataaaagatc 2400

attattttca atagatctgt gtgttggttt tttgtgtgcc ttgggggagg gggaggccag 2460

aatgaggcgc ggccaagggg gagggggagg ccagaatgac cttgggggag ggggaggcca 2520

gaatgacctt gggggagggg gaggccagaa tgaggcgcgc cagttacgct agggataaca 2580

gataacttcg tataatgtat gctatacgaa gttattgcag ttttaaaatt atgttttaaa 2640

atggactatc atatgctcac cataaacgtg aaatgtcttt ggatttggga atcttataa 2699

<210> 15
<211> 330
<212> DNA
<213> Artificial

<220>
<223> construct 5 after Cre Recombination

<400> 15
aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcata tacgatacaa ggctgttaga gagataatta gaattaattt 180

gactgtaaac acaaagatat tagtacaaaa taataacttc gtataatgta tgctatacga 240

agttattgca gttttaaaat tatgttttaa aatggactat catatgctca ccataaacgt 300

gaaatgtctt tggatttggg aatcttataa 330

<210> 16
<211> 2427
<212> DNA
<213> Artificial

<220>
<223> construct 6 before Cre Recombination

<400>  16
atttgcatta gagaataact tcgtataatg tatgctatac gaagttattt tttgcgttaa      60

ttaagtgcga ttaagggtgc agcggcctcc gcgccgggtt ttggcgcctc ccgcgggcgc     120

ccccctcctc acggcgagcg ctgccacgtc agacgaaggg cgcaggagcg ttcctgatcc     180

ttccgcccgg acgctcagga cagcggcccg ctgctcataa gactcggcct tagaacccca     240

gtatcagcag aaggacattt taggacggga cttgggtgac tctagggcac tggttttctt     300

tccagagagc ggaacaggcg aggaaaagta gtcccttctc ggcgattctg cggagggatc     360

tccgtggggc ggtgaacgcc gatgattata taaggacgcg ccgggtgtgg cacagctagt     420

tccgtcgcag ccgggatttg ggtcgcggtt cttgtttgtg gatcgctgtg atcgtcactt     480

ggtgagttgc gggctgctgg gctggccggg gctttcgtgg ccgccgggcc gctcggtggg     540

acggaagcgt gtggagagac cgccaagggc tgtagtctgg gtccgcgagc aaggttgccc     600

tgaactgggg gttgggggga gcgcacaaaa tggcggctgt tcccgagtct tgaatggaag     660

acgcttgtaa ggcgggctgt gaggtcgttg aaacaaggtg gggggcatgg tgggcggcaa     720

gaacccaagg tcttgaggcc ttcgctaatg cgggaaagct cttattcggg tgagatgggc     780

tggggcacca tctggggacc ctgacgtgaa gtttgtcact gactggagaa ctcgggtttg     840

tcgtctggtt gcgggggcgg cagttatgcg gtgccgttgg gcagtgcacc cgtacctttg     900

ggagcgcgcg cctcgtcgtg tcgtgacgtc acccgttctg ttggcttata atgcagggtg     960

gggccacctg ccggtaggtg tgcggtaggc ttttctccgt cgcaggacgc agggttcggg    1020

cctagggtag gctctcctga atcgacaggc gccggacctc tggtgagggg agggataagt    1080

gaggcgtcag tttctttggt cggttttatg tacctatctt cttaagtagc tgaagctccg    1140

gttttgaact atgcgctcgg ggttggcgag tgtgttttgt gaagtttttt aggcaccttt    1200

tgaaatgtaa tcatttgggt caatatgtaa ttttcagtgt tagactagta aagcttctgc    1260

aggtcgactc tagaaaattg tccgctaaat tctggccgtt tttggctttt ttgttagaca    1320

ggatcctgca ctagtcggct agctgtccgg tcgccaccat ggcccagtcc aagcacggcc    1380

tgaccaagga gatgaccatg aagtaccgca tggagggctg cgtggacggc cacaagttcg    1440

tgatcaccgg cgagggcatc ggctacccct tcaagggcaa gcaggccatc aacctgtgcg    1500

tggtggaggg cggccccttg cccttcgccg aggacatctt gtccgccgcc ttcatgtacg    1560

gcaaccgcgt gttcaccgag tacccccagg acatcgtcga ctacttcaag aactcctgcc    1620

ccgccggcta cacctgggac cgctccttcc tgttcgagga cggcgccgtg tgcatctgca    1680

acgccgacat caccgtgagc gtggaggaga actgcatgta ccacgagtcc aagttctacg    1740

gcgtgaactt ccccgccgac ggccccgtga tgaagaagat gaccgacaac tgggagccct    1800

cctgcgagaa gatcatcccc gtgcccaagc agggcatctt gaagggcgac gtgagcatgt    1860

acctgctgct gaaggacggt ggccgcttgc gctgccagtt cgacaccgtg tacaaggcca 1920

agtccgtgcc ccgcaagatg cccgactggc acttcatcca gcacaagctg acccgcgagg 1980

accgcagcga cgccaagaac cagaagtggc acctgaccga gcacgccatc gcctccggct 2040

ccgccttgcc ctgagcggcc gaccggacag cggccgcagt cctcaggatc atatgctacg 2100

cgtgacaatt ggcggcccga ttctagaccg gttcgagatc caggcgcgga tcaataaaag 2160

atcattattt tcaatagatc tgtgtgttgg ttttttgtgt gccttggggg agggggaggc 2220

cagaatgagg cgcggccaag gggagggggg aggccagaat gaccttgggg gagggggagg 2280

ccagaatgac cttgggggag ggggaggcca gaatgaggcg cgtataactt cgtataatgt 2340

atgctatacg aagttattca tatgtcgcta tgtgttctgg gaaatcacca taaacgtgaa 2400

atgtctttgg atttgggaat cttataa 2427

<210> 17
<211> 118
<212> DNA
<213> Artificial

<220>
<223> construct 6 after Cre Recombination

<400> 17
atttgcatta gagaataact tcgtataatg tatgctatac gaagttattc atatgtcgct 60

atgtgttctg ggaaatcacc ataaacgtga aatgtctttg gatttgggaa tcttataa 118

<210> 18
<211> 2619
<212> DNA
<213> Artificial

<220>
<223> construct 7 before Cre Recombination

<400> 18
aattcgaacg ctgacgtcat caacccgctc caaggaatcg cgggcccagt gtcactaggc 60

gggaacaccc agcgcgcgtg cgccctggca ggaagatggc tgtgagggac aggggagtgg 120

cgccctgcaa tatttgcatg tcgctatgtg ttctgggaaa tagagaataa cttcgtataa 180

tgtatgctat acgaagttat gcgtaatacg actcactata gggcgaattg gagctccacc 240

gcggcctgca ggggccggcc atttaaatag ttacgctagg gataacaggg taaatagtta 300

attaacccgt gtcggctcca gatctggcct ccgcgccggg ttttggcgcc tcccgcgggc 360

gcccccctcc tcacggcgag cgctgccacg tcagacgaag ggcgcagcga gcgtcctgat 420

ccttccgccc ggacgctcag gacagcggcc cgctgctcat aagactcggc cttagaaccc 480

cagtatcagc agaaggacat tttaggacgg gacttgggtg actctagggc actggttttc 540

tttccagaga gcggaacagg cgaggaaaag tagtcccttc tcggcgattc tgcggaggga 600

tctccgtggg gcggtgaacg ccgatgatta tataaggacg cgccgggtgt ggcacagcta 660

gttccgtcgc agccgggatt tgggtcgcgg ttcttgtttg tggatcgctg tgatcgtcac 720

ttggtgagta gcgggctgct gggctggccg gggctttcgt ggccgccggg ccgctcggtg 780

ggacggaagc gtgtggagag accgccaagg gctgtagtct gggtccgcga gcaaggttgc 840

cctgaactgg gggttggggg gagcgcagca aaatggcggc tgttcccgag tcttgaatgg 900

aagacgcttg tgaggcgggc tgtgaggtcg ttgaaacaag gtggggggca tggtgggcgg 960

caagaaccca aggtcttgag gccttcgcta atgcgggaaa gctcttattc gggtgagatg 1020

ggctggggca ccatctgggg accctgacgt gaagtttgtc actgactgga gaactcggtt 1080

tgtcgtctgt tgcgggggcg gcagttatgg cggtgccgtt gggcagtgca cccgtacctt 1140

tgggagcgcg cgccctcgtc gtgtcgtgac gtcacccgtt ctgttggctt ataatgcagg 1200

gtggggccac ctgccggtag gtgtgcggta ggcttttctc cgtcgcagga cgcagggttc 1260

gggcctaggg taggctctcc tgaatcgaca ggcgccggac ctctggtgag gggagggata 1320

agtgaggcgt cagtttcttt ggtcggtttt atgtacctat cttcttaagt agctgaagct 1380

ccggttttga actatgcgct cggggttggc gagtgtgttt tgtgaagttt tttaggcacc 1440

ttttgaaatg taatcatttg ggtcaatatg taattttcag tgttagacta gtaaattgtc 1500

cgctaaattc tggccgtttt tggctttttt gttagacgaa gcttgggctg caggtcgact 1560

ctagaggatc cccgggtacc ggtcgccacc atggcctctt tgctgaagaa gaccatgccc 1620

ttcaggacca ccatcgaggg caccgtgaac ggccactact tcaagtgcac cggcaagggc 1680

gagggcaacc ccctggaggg cacccaggag atgaagatcg aggtgatcga gggcggcccc 1740

ctgcccttcg ccttccacat cctgtccacc tcctgcatgt acggctccaa ggccttcatc 1800

aagtacgtgt ccggcatccc cgactacttc aagcagtccc tccccgaggg cttcacctgg 1860

gagcgcacca ccacctacga ggacggcggc ttcctgaccg cccaccagga cacctccctg 1920

gacggcgact gcctggtgta caaggtgaag atcctgggca acaacttccc cgccgacggc 1980

cccgtgatgc agaacaaggc cggccgctgg gagccctcca ccgagatcgt gtacgaggtg 2040

gacggcgtgc tgcgcggcca gtccctgatg gccctggagt gccccggcgg tcgccacctg 2100

acctgccacc tgcacaccac ctaccgctcc aagaagcccg cctccgccct gaagatgccc 2160

ggcttccact tcgaggacca ccgcatcgag atcctggagg aggtggagaa gggcaagtgc 2220

tacaagcagt acgaggccgc cgtgggccgc tactgcgacg ccgccccctc caagctgggc 2280

cacaactgaa gcggccgcga ctctagaatt ggccgcgtcg acctcgagat ccaggcgcgg 2340

atcaataaaa gatcattatt ttcaatagat ctgtgtgttg gttttttgtg tgccttgggg 2400

gagggggagg ccagaatgag gcgcggccaa gggggagggg gaggccagaa tgaccttggg 2460

ggagggggag gccagaatga ccttggggga gggggaggcc agaatgaggc gcgccagtta    2520

cgctagggat aacagataac ttcgtataat gtatgctata cgaagttatt catatgtcac    2580

cataaacgtg aaatgtcttt ggatttggga atcttataa                           2619


<210>  19
<211>  250
<212>  DNA
<213>  Artificial

<220>
<223>  construct 7 after Cre Recombination

<400>  19
aattcgaacg ctgacgtcat caacccgctc caaggaatcg cgggcccagt gtcactaggc      60

gggaacaccc agcgcgcgtg cgccctggca ggaagatggc tgtgagggac aggggagtgg     120

cgccctgcaa tatttgcatg tcgctatgtg ttctgggaaa tagagaataa cttcgtataa     180

tgtatgctat acgaagttat tcatatgtca ccataaacgt gaaatgtctt tggatttggg     240

aatcttataa                                                            250


<210>  20
<211>  329
<212>  DNA
<213>  Homo sapiens

<400>  20
agtggaaaga cgcgcaggca aaacgcacca cgtgacggag cgtgaccgcg cgccgagcgc      60

gcgccaaggt cgggcaggaa gagggcctat ttcccatgat tccttcatat ttgcatatac     120

gatacaaggc tgttagagag ataattagaa ttaatttgac tgtaaacaca aagatattag     180

tacaaaatac gtgacgtaga aagtaataat ttcttgggta gtttgcagtt ttaaaattat     240

gttttaaaat ggactatcat atgcttaccg taacttgaaa gtatttcgat ttcttggctt     300

tatatatctt gtggaaagga cgaaacacc                                       329


<210>  21
<211>  186
<212>  DNA
<213>  Homo sapiens

<400>  21
atcgcttctc ggccttttgg ctaagatcaa gtgtagtatc tgttcatcag tttaatatct      60

gatacgtcct ctatccgagg acaatatatt aaatggattt ttggagcagg gagatggaat     120

aggagcttgc tccgtccact ccacgcatcg acctggtatt gcagtacctc caggaacggt     180

gcacca                                                                186


<210>  22

<211>  200
<212>  DNA
<213>  Mus musculus

<400>  22
ggttaatttg catataatat ttcctagtaa ctatagaggc ttaatgtgcg ataaaagaca      60

gataatctgt tctttttaat actagctaca ttttacatga taggcttgga tttctataag     120

agatacaaat actaaattat tattttaaaa aacagcacaa aaggaaactc accctaactg     180

taaagtaatt gtgtgttttg                                                 200


<210>  23
<211>  50
<212>  DNA
<213>  Gallus gallus

<400>  23
acgtcacacc tactttgcat agcgcttccc agcatgcctc gcggccgccc                 50


<210>  24
<211>  407
<212>  DNA
<213>  Cauliflower mosaic virus

<400>  24
cgcgttacat aacttacggt aaatggcccg cctggctgac cgcccaacga cccccgccca      60

ttgacgtcaa taatgacgta tgttcccata gtaacgccaa tagggacttt ccattgacgt     120

caatgggtgg agtatttacg gtaaactgcc cacttggcag tacatcaagt gtatcatatg     180

ccaagtacgc cccctattga cgtcaatgac ggtaaatggc ccgcctggca ttatgcccag     240

tacatgacct tatgggactt tcctacttgg cagtacatct acgtattagt catcgctatt     300

accatggtga tgcggttttg gcagtacatc aatgggcgtg gatagcggtt tgactcacgg     360

ggatttccaa gtctccaccc cattgacgtc aatgggagtt tgttttg                   407


<210>  25
<211>  211
<212>  DNA
<213>  Simian virus 40

<400>  25
ggtgtggaaa gtccccaggc tccccagcag gcagaagtat gcaaagcatg catctcaatt      60

agtcagcaac caggtgtgga aagtccccag gctccccagc aggcagaagt atgcaaagca     120

tgcatctcaa ttagtcagca accatagtcc cgcccctaac tccgcccatc ccgcccctaa     180

ctccgcccag ttccgcccat tctccgcccc a                                    211


<210>  26
<211>  11411
<212>  DNA
<213>  Artificial

<220>
<223>  H1 hybrid promoter

<400>  26
gaagttccta ttccgaagtt cctattcttc aaatagtata ggaacttcgc tcgagggatc      60

ggccattgaa caagatggat tgcacgcagg ttctccggcc gcttgggtgg agaggctatt     120

cggctatgac tgggcacaac agacaatcgg ctgctctgat gccgccgtgt tccggctgtc     180

agcgcagggg cgcccggttc tttttgtcaa gaccgacctg tccggtgccc tgaatgaact     240

gcaggacgag gcagcgcggc tatcgtggct ggccacgacg ggcgttcctt gcgcagctgt     300

gctcgacgtt gtcactgaag cgggaaggga ctggctgcta ttgggcgaag tgccggggca     360

ggatctcctg tcatctcacc ttgctcctgc cgagaaagta tccatcatgg ctgatgcaat     420

gcggcggctg catacgcttg atccggctac ctgcccattc gaccaccaag cgaaacatcg     480

catcgagcga gcacgtactc ggatggaagc cggtcttgtc gatcaggatg atctggacga     540

agagcatcag gggctcgcgc cagccgaact gttcgccagg ctcaaggcgc gcatgcccga     600

cggcgaggat ctcgtcgtga cccatggcga tgcctgcttg ccgaatatca tggtggaaaa     660

tggccgcttt tctggattca tcgactgtgg ccggctgggt gtggcggacc gctatcagga     720

catagcgttg gctacccgtg atattgctga agagcttggc ggcgaatggg ctgaccgctt     780

cctcgtgctt tacggtatcg ccgctcccga ttcgcagcgc atcgccttct atcgccttct     840

tgacgagttc ttctgagggg atcgatccgc tgtaagtctg cagaaattga tgatctatta     900

aacaataaag atgtccacta aaatggaagt ttttcctgtc atactttgtt aagaagggtg     960

agaacagagt acctacattt tgaatggaag gattggagct acgggggtgg gggtggggtg    1020

ggattagata aatgcctgct ctttactgaa ggctctttac tattgcttta tgataatgtt    1080

tcatagttgg atatcataat ttaaacaagc aaaaccaaat taagggccag ctcattcctc    1140

ccactcatga tctatagatc tatagatctc tcgtgggatc attgtttttc tcttgattcc    1200

cactttgtgg ttctaagtac tgtggtttcc aaatgtgtca gtttcatagc ctgaagaacg    1260

agatcagcag cctctgttcc acatacactt cattctcagt attgttttgc caagttctaa    1320

ttccatcaga agctgactct agatggcgcg tatgcattaa ttaaggccag ggatcttcaa    1380

gcagacctac agcaagttcg acacaaactc acacaacgat gacgcactac tcaagaacta    1440

cgggctgctc tactgcttca ggaaggacat ggacaaggtc gagacattcc tgcgcatcgt    1500

gcagtgccgc tctgtggagg gcagctgtgg cttctagctg cccgggtggc atccctgtga    1560

cccctcccca gtgcctctcc tggccctgga agttgccact ccagtgccca ccagccttgt    1620

cctaataaaa ttaagttgca tcattttgtc tgactaggtg tccttctata atattatggg    1680

gtggaggggg gtggtatgga gcaaggggca agttgggaag acaacctgta gggcctgcgg    1740

ggtctattgg gaaccaagct ggagtgcagt ggcacaatct tggctcactg caatctccgc 1800

ctcctgggtt caagcgattc tcctgcctca gcctcccgag ttgttgggat tccaggcatg 1860

catgaccagg ctcagctaat ttttgttttt ttggtagaga cggggtttca ccatattggc 1920

caggctggtc tccaactcct aatctcaggt gatctaccca ccttggcctc ccaaattgct 1980

gggattacag gcgtgaacca ctgctccctt ccctgtcctt ctgattttaa aataactata 2040

ccagcaggag gacgtccaga cacagcatag gctacctggc catgcccaac cggtgggaca 2100

tttgagttgc ttgcttggca ctgtcctctc atgcgttggg tccactcagt agatgcctgt 2160

tgaattaagc ttatttaaat aggccggcca gatctgtcga caattggatc ctcacagtag 2220

gtggcatcgt tcctttctga ctgcccgccc cccgcatgcc gtcccgcgat attgagctcc 2280

gaacctctcg ccctgccgcc gccggtgctc cgtcgccgcc gcgccgccat ggaattcgaa 2340

cgctgacgtc atcaacccgc tccaaggaat cgcgggccca gtgtcactag gcgggaacac 2400

ccagcgcgcg tgcgccctgg caggaagatg gctgtgaggg acaggggagt ggcgccctgc 2460

aatatttgca tatacgatac aaggctgtta gagagataat tagaattaat ttgactgtaa 2520

acacaaagat attagtacaa aatatgcagt tttaaaatta tgttttaaaa tggactatca 2580

tatgctcacc ataaacgtga aatgtctttg gatttgggaa tcttataagt ccctatcagt 2640

gatagagatt cccagtggat ggagccgata ttggattcaa gagattcaat attggcttca 2700

tccacttttt ggcgcgtgac actagttatg cccctagtta ttaatagtaa tcaattacgg 2760

ggtcattagt tcatagccca tatatggagt tccgcgttac ataacttacg gtaaatggcc 2820

cgcctggctg accgcccaac gacccccgcc cattgacgtc aataatgacg tatgttccca 2880

tagtaacgcc aatagggact ttccattgac gtcaatgggt ggactattta cggtaaactg 2940

cccacttggc agtacatcaa gtgtatcata tgccaagtac gccccctatt gacgtcaatg 3000

acggtaaatg gcccgcctgg cattatgccc agtacatgac cttatgggac tttcctactt 3060

ggcagtacat ctacgtatta gtcatcgcta ttaccatggg tcgaggtgag ccccacgttc 3120

tgcttcactc tccccatctc ccccccctcc ccacccccaa ttttgtattt atttattttt 3180

taattatttt gtgcagcgat gggggcgggg gggggggggg cgcgcgccag gcggggcggg 3240

gcggggcgag gggcggggcg gggcgaggcg gagaggtgcg gcggcagcca atcagagcgg 3300

cgcgctccga aagtttcctt ttatggcgag gcggcggcgg cggcggccct ataaaaagcg 3360

aagcgcgcgg cgggcgggag tcgctgcgtt gccttcgccc cgtgccccgc tccgcgccgc 3420

ctcgcgccgc ccgccccggc tctgactgac cgcgttactc ccacaggtga gcgggcggga 3480

cggcccttct cctccgggct gtaattagcg cttggtttaa tgacggctcg tttcttttct 3540

gtggctgcgt gaaagcctta aagggctccg ggagggccct ttgtgcgggg gggagcggct 3600

cggggggtgc gtgcgtgtgt gtgtgcgtgg ggagcgccgc gtgcggcccg cgctgcccgg 3660

cggctgtgag cgctgcgggc gcggcgcggg gctttgtgcg ctccgcgtgt gcgcgagggg 3720
agcgcggccg ggggcggtgc cccgcggtgc gggggggctg cgaggggaac aaaggctgcg 3780
tgcggggtgt gtgcgtgggg gggtgagcag ggggtgtggg cgcggcggtc gggctgtaac 3840
ccccccctgc acccccctcc ccgagttgct gagcacggcc cggcttcggg tgcggggctc 3900
cgtgcggggc gtggcgcggg gctcgccgtg ccgggcgggg ggtggcggca ggtgggggtg 3960
ccgggcgggg cggggccgcc tcgggccggg gagggctcgg gggaggggcg cggcggcccc 4020
ggagcgccgg cggctgtcga ggcgcggcga gccgcagcca ttgcctttta tggtaatcgt 4080
gcgagagggc gcagggactt cctttgtccc aaatctggcg gagccgaaat ctgggaggcg 4140
ccgccgcacc ccctctagcg ggcgcgggcg aagcggtgcg gcgccggcag gaaggaaatg 4200
ggcggggagg gccttcgtgc gtcgccgcgc cgccgtcccc ttctccatct ccagcctcgg 4260
ggctgccgca gggggacggc tgccttcggg ggggacgggg cagggcgggg ttcggcttct 4320
ggcgtgtgac cggcggctct agtaagcgtt ggggtgagta ctccctctca aaagcgggca 4380
tgacttctgc gctaagattg tcagtttcca aaaacgagga ggatttgata ttcacctggc 4440
ccgcggtgat gcctttgagg gtggccgcgt ccatctggtc agaaaagaca atctttttgt 4500
tgtcaagctt gaggtgtggc aggcttgaga tctggccata cacttgagtg acattgacat 4560
ccactttgcc tttctctcca caggtgtcca ctcccagggc ggccgcaatt cccggggatc 4620
gaaagagcct gctaaagcaa aaaagaagtc accatgtcgt ttactttgac caacaagaac 4680
gtgattttcg ttgccggtct gggaggcatt ggtctggaca ccagcaagga gctgctcaag 4740
cgcgatcccg tcgttttaca acgtcgtgac tgggaaaacc ctggcgttac ccaacttaat 4800
cgccttgcag cacatccccc tttcgccagc tggcgtaata gcgaagaggc ccgcaccgat 4860
cgcccttccc aacagttgcg cagcctgaat ggcgaatggc gctttgcctg gtttccggca 4920
ccagaagcgg tgccggaaag ctggctggag tgcgatcttc ctgaggccga tactgtcgtc 4980
gtcccctcaa actggcagat gcacggttac gatgcgccca tctacaccaa cgtaacctat 5040
cccattacgg tcaatccgcc gtttgttccc acggagaatc cgacgggttg ttactcgctc 5100
acatttaatg ttgatgaaag ctggctacag gaaggccaga cgcgaattat ttttgatggc 5160
gttaactcgg cgtttcatct gtggtgcaac gggcgctggg tcggttacgg ccaggacagt 5220
cgtttgccgt ctgaatttga cctgagcgca tttttacgcg ccggagaaaa ccgcctcgcg 5280
gtgatggtgc tgcgttggag tgacggcagt tatctggaag atcaggatat gtggcggatg 5340
agcggcattt tccgtgacgt ctcgttgctg cataaaccga ctacacaaat cagcgatttc 5400
catgttgcca ctcgctttaa tgatgatttc agccgcgctg tactggaggc tgaagttcag 5460
atgtgcggcg agttgcgtga ctacctacgg gtaacagttt ctttatggca gggtgaaacg 5520

caggtcgcca gcggcaccgc gcctttcggc ggtgaaatta tcgatgagcg tggtggttat 5580

gccgatcgcg tcacactacg tctgaacgtc gaaaacccga aactgtggag cgccgaaatc 5640

ccgaatctct atcgtgcggt ggttgaactg cacaccgccg acggcacgct gattgaagca 5700

gaagcctgcg atgtcggttt ccgcgaggtg cggattgaaa atggtctgct gctgctgaac 5760

ggcaagccgt tgctgattcg aggcgttaac cgtcacgagc atcatcctct gcatggtcag 5820

gtcatggatg agcagacgat ggtgcaggat atcctgctga tgaagcagaa caactttaac 5880

gccgtgcgct gttcgcatta tccgaaccat ccgctgtggt acacgctgtg cgaccgctac 5940

ggcctgtatg tggtggatga agccaatatt gaaacccacg gcatggtgcc aatgaatcgt 6000

ctgaccgatg atccgcgctg gctaccggcg atgagcgaac gcgtaacgcg aatggtgcag 6060

cgcgatcgta atcacccgag tgtgatcatc tggtcgctgg ggaatgaatc aggccacggc 6120

gctaatcacg acgcgctgta tcgctggatc aaatctgtcg atccttcccg cccggtgcag 6180

tatgaaggcg gcggagccga caccacggcc accgatatta tttgcccgat gtacgcgcgc 6240

gtggatgaag accagccctt cccggctgtg ccgaaatggt ccatcaaaaa atggctttcg 6300

ctacctggag agacgcgccc gctgatcctt tgcgaatacg cccacgcgat gggtaacagt 6360

cttggcggtt tcgctaaata ctggcaggcg tttcgtcagt atccccgttt acagggcggc 6420

ttcgtctggg actgggtgga tcagtcgctg attaaatatg atgaaaacgg caacccgtgg 6480

tcggcttacg gcggtgattt tggcgatacg ccgaacgatc gccagttctg tatgaacggt 6540

ctggtctttg ccgaccgcac gccgcatcca gcgctgacgg aagcaaaaca ccagcagcag 6600

tttttccagt tccgtttatc cgggcaaacc atcgaagtga ccagcgaata cctgttccgt 6660

catagcgata acgagctcct gcactggatg gtggcgctgg atggtaagcc gctggcaagc 6720

ggtgaagtgc ctctggatgt cgctccacaa ggtaaacagt tgattgaact gcctgaacta 6780

ccgcagccgg agagcgccgg gcaactctgg ctcacagtac gcgtagtgca accgaacgcg 6840

accgcatggt cagaagccgg gcacatcagc gcctggcagc agtggcgtct ggcggaaaac 6900

ctcagtgtga cgctccccgc cgcgtcccac gccatcccgc atctgaccac cagcgaaatg 6960

gatttttgca tcgagctggg taataagcgt tggcaattta accgccagtc aggctttctt 7020

tcacagatgt ggattggcga taaaaaacaa ctgctgacgc cgctgcgcga tcagttcacc 7080

cgtgcaccgc tggataacga cattggcgta agtgaagcga cccgcattga ccctaacgcc 7140

tgggtcgaac gctggaaggc ggcgggccat taccaggccg aagcagcgtt gttgcagtgc 7200

acggcagata cacttgctga tgcggtgctg attacgaccg ctcacgcgtg gcagcatcag 7260

gggaaaacct tatttatcag ccggaaaacc taccggattg atggtagtgg tcaaatggcg 7320

attaccgttg atgttgaagt ggcgagcgat acaccgcatc cggcgcggat tggcctgaac 7380

tgccagctgg cgcaggtagc agagcgggta aactggctcg gattagggcc gcaagaaaac 7440

tatcccgacc gccttactgc cgcctgtttt gaccgctggg atctgccatt gtcagacatg 7500

tataccccgt acgtcttccc gagcgaaaac ggtctgcgct gcgggacgcg cgaattgaat 7560

tatggcccac accagtggcg cggcgacttc cagttcaaca tcagccgcta cagtcaacag 7620

caactgatgg aaaccagcca tcgccatctg ctgcacgcgg aagaaggcac atggctgaat 7680

atcgacggtt tccatatggg gattggtggc gacgactcct ggagcccgtc agtatcggcg 7740

gaattacagc tgagcgccgg tcgctaccat taccagttgg tctggtgtca aaaataataa 7800

taaccgggca ggccatgtct gcccgtattt cgcgtaagga aatccattat gtactattta 7860

aaaaacacaa acttttggat gttcggttta ttctttttct tttacttttt tatcatggga 7920

gcctacttcc cgtttttccc gatttggcta catgacatca accatatcag caaaagtgat 7980

acgggtatta tttttgccgc tatttctctg ttctcgctat tattccaacc gctgtttggt 8040

ctgctttctg acaaactcgg cctcgactct aggcggccgc gtcgacctcg agatccaggc 8100

gcggatcaat aaaagatcat tattttcaat agatctgtgt gttggttttt tgtgtgcctt 8160

gggggagggg gaggccagaa tgaggcgcgg ccaaggggga gggggaggcc agaatgacct 8220

tgggggaggg ggaggccaga atgaccttgg gggaggggga ggccagaatg aggcgcgccg 8280

gtaaccgaag ttcctatact ttctagagaa taggaacttc ggaataggaa cttcaagccg 8340

gtacccagct tttgttccct ttagtgaggg ttaatttcga gcttggcgta atcatggtca 8400

tagctgtttc ctgtgtgaaa ttgttatccg ctcacaattc cacacaacat acgagccgga 8460

agcataaagt gtaaagcctg gggtgcctaa tgagtgagct aactcacatt aattgcgttg 8520

cgctcactgc ccgctttcca gtcgggaaac ctgtcgtgcc agctgcatta atgaatcggc 8580

caacgcgcgg ggagaggcgg tttgcgtatt gggcgctctt ccgcttcctc gctcactgac 8640

tcgctgcgct cggtcgttcg gctgcggcga gcggtatcag ctcactcaaa ggcggtaata 8700

cggttatcca cagaatcagg ggataacgca ggaaagaaca tgtgagcaaa aggccagcaa 8760

aaggccagga accgtaaaaa ggccgcgttg ctggcgtttt tccataggct ccgcccccct 8820

gacgagcatc acaaaaatcg acgctcaagt cagaggtggc gaaacccgac aggactataa 8880

agataccagg cgtttccccc tggaagctcc ctcgtgcgct ctcctgttcc gaccctgccg 8940

cttaccggat acctgtccgc ctttctccct tcgggaagcg tggcgctttc tcatagctca 9000

cgctgtaggt atctcagttc ggtgtaggtc gttcgctcca agctgggctg tgtgcacgaa 9060

ccccccgttc agcccgaccg ctgcgcctta tccggtaact atcgtcttga gtccaacccg 9120

gtaagacacg acttatcgcc actggcagca gccactggta acaggattag cagagcgagg 9180

tatgtaggcg gtgctacaga gttcttgaag tggtggccta actacggcta cactagaagg 9240

acagtatttg gtatctgcgc tctgctgaag ccagttacct tcggaaaaag agttggtagc 9300

tcttgatccg gcaaacaaac caccgctggt agcggtggtt tttttgtttg caagcagcag 9360

attacgcgca gaaaaaaagg atctcaagaa gatcctttga tcttttctac ggggtctgac 9420

gctcagtgga acgaaaactc acgttaaggg attttggtca tgagattatc aaaaaggatc 9480

ttcacctaga tccttttaaa ttaaaaatga agttttaaat caatctaaag tatatatgag 9540

taaacttggt ctgacagtta ccaatgctta atcagtgagg cacctatctc agcgatctgt 9600

ctatttcgtt catccatagt tgcctgactc cccgtcgtgt agataactac gatacgggag 9660

ggcttaccat ctggccccag tgctgcaatg ataccgcgag acccacgctc accggctcca 9720

gatttatcag caataaacca gccagccgga agggccgagc gcagaagtgg tcctgcaact 9780

ttatccgcct ccatccagtc tattaattgt tgccgggaag ctagagtaag tagttcgcca 9840

gttaatagtt tgcgcaacgt tgttgccatt gctacaggca tcgtggtgtc acgctcgtcg 9900

tttggtatgg cttcattcag ctccggttcc caacgatcaa ggcgagttac atgatccccc 9960

atgttgtgca aaaaagcggt tagctccttc ggtcctccga tcgttgtcag aagtaagttg 10020

gccgcagtgt tatcactcat ggttatggca gcactgcata attctcttac tgtcatgcca 10080

tccgtaagat gcttttctgt gactggtgag tactcaacca agtcattctg agaatagtgt 10140

atgcggcgac cgagttgctc ttgcccggcg tcaatacggg ataataccgc gccacatagc 10200

agaactttaa aagtgctcat cattggaaaa cgttcttcgg ggcgaaaact ctcaaggatc 10260

ttaccgctgt tgagatccag ttcgatgtaa cccactcgtg cacccaactg atcttcagca 10320

tcttttactt tcaccagcgt ttctgggtga gcaaaaacag gaaggcaaaa tgccgcaaaa 10380

aagggaataa gggcgacacg gaaatgttga atactcatac tcttcctttt tcaatattat 10440

tgaagcattt atcagggtta ttgtctcatg agcggataca tatttgaatg tatttagaaa 10500

aataaacaaa taggggttcc gcgcacattt ccccgaaaag tgccacctaa attgtaagcg 10560

ttaatatttt gttaaaattc gcgttaaatt tttgttaaat cagctcattt tttaaccaat 10620

aggccgaaat cggcaaaatc ccttataaat caaaagaata gaccgagata gggttgagtg 10680

ttgttccagt ttggaacaag agtccactat taaagaacgt ggactccaac gtcaaagggc 10740

gaaaaaccgt ctatcagggc gatggcccac tacgtgaacc atcaccctaa tcaagttttt 10800

tggggtcgag gtgccgtaaa gcactaaatc ggaaccctaa agggagcccc cgatttagag 10860

cttgacgggg aaagccggcg aacgtggcga gaaaggaagg gaagaaagcg aaaggagcgg 10920

gcgctagggc gctggcaagt gtagcggtca cgctgcgcgt aaccaccaca cccgccgcgc 10980

ttaatgcgcc gctacagggc gcgtcccatt cgccattcag gctgcgcaac tgttgggaag 11040

ggcgatcggt gcgggcctct tcgctattac gccagctggc gaaaggggga tgtgctgcaa 11100

ggcgattaag ttgggtaacg ccagggtttt cccagtcacg acgttgtaaa acgacggcca 11160

gtgaattgta atacgactca ctatagggcg aattgggggt aactaagtaa ggatcgagat 11220

ccaggcgcgg atcaataaaa gatcattatt ttcaatagat ctgtgtgttg gttttttgtg 11280

tgccttgggg gagggggagg ccagaatgag gcgcggccaa gggggagggg gaggccagaa 11340

tgaccttggg ggagggggag gccagaatga ccttggggga gggggaggcc agaatgaggc 11400

gcggatccgg a 11411

<210> 27
<211> 13139
<212> DNA
<213> Mus musculus

<400> 27
aagcttctca cgtagcaacc agagctccag agccagcagc tgctgccgcc ttgtatactc 60

actcctgtga tccaacacag gagcaacctt ttctttaccc cacccccact tcttaacaca 120

cttttttttg gggggggggg gggaacaagt gctccatgct ggaaggattg gaactatgct 180

tttagaaagg aacaatccta aggtcacttt taaattgagg tctttgattt gaaaatcaac 240

aaataccaaa ttccaaatat tcgttttaat taaaccagca atgtggatat aagcattaag 300

ttttagtttt aaaaaggtca attttccaaa cattcagcaa tcatatttaa atttacagct 360

aggaacaaga gccttgggtc atgtcctacc aaagaacata actcaatatt ctacacatga 420

caatctgaat aaccttaaag cctctaatcc cataacaggc cacaaatttt ggacagagaa 480

ctaatgatcc tcctgagaaa actggaagaa atccagggaa aagaaattcc tgtgtcctcc 540

aaactcagaa atctctaatt atgtcagtat tctctgcttt agtcctaggt cagattgcac 600

acatctaaaa taacctctta aagttttcct cctagcgacc taaaccatta ttaatatcaa 660

attaaccatc aaaacacttt cctctcaata tgctgcacac aaacctcctc ctggaacctc 720

ctccatctgg atcctcccca atcaaaagta taggtattta acatataagc aaggaagtaa 780

tgtaaacatg accttggtca caaatatgtc atctaaaaac aatttagtca aggtatggag 840

gaaattcgag aacctgaatc tttttaagta ttttgagcac aggaacaatt ggcaaaagga 900

atccaggtat agacaaaacc cagagcccag agctctgggc gaaaaatgag ttgctggtga 960

agacgttaca caagtaacat gagaaagcag aaaatgcagg tcatccacgc acccctgacc 1020

caggccagca gggcgggctg cagcatcagt acacaggaga aagatcctta ttcctaagaa 1080

tgagaaaggc aaaggcgccc gatagaataa attagcatag aaggggcttt cccaggagtt 1140

aaaactttcc ttctgagcga ttacctacta aaaccagggc ttttgcccac taccatttac 1200

ctaggatctt ggcttgcacg gattcatagg ggcatatccc tccccctctt ctttagagtc 1260

gttcttaaaa gatcgctctc cacgccctag gcagggaaaa cgacaaaatc tggctcaatt 1320

ccaggctaga accctacaaa ttcaacaggg atatcgcaag gatactgggg catacgccac 1380

agggagtcca agaatgtgag gtgggggtgg cgaaggtaat gtctttggtg tgggaaaagc 1440

agcagccatc tgagatagga actggaaaac cagaggagag gcgttcagga agattatgga 1500

ggggaggact gggcccccac gagcgaccag agttgtcaca aggccgcaag aacaggggag 1560

gtggggggct cagggacaga aaaaaaagta tgtgtatttt gagagcaggg ttgggaggcc 1620

tctcctgaaa agggtataaa cgtggagtag gcaataccca ggcaaaaagg ggagaccaga 1680

gtagggggag gggaagagtc ctgacccagg gaagacatta aaaaggtagt ggggtcgact 1740

agatgaagga gagcctttct ctctgggcaa gagcggtgca atggtgtgta aaggtagctg 1800

agaagacgaa aagggcaagc atcttcctgc taccaggctg ggaggcccca ggcccacgac 1860

cccgaggaga gggaacgcag ggagactgag gtgacccttc tttcccccgg ggcccggtcg 1920

tgtggttcgg tgtctctttt ctgttggacc cttaccttga cccaggcgct gccggggcct 1980

gggcccgggc tgcggcgcac ggcactcccg ggaggcagcg agactcgagt taggcccaac 2040

gcggcgccac ggcgtttcct ggccgggaat ggcccgtacc cgtgaggtgg gggtgggggg 2100

cagaaaaggc ggagcgagcc cgagcgggga gggggagggc caggggcgga gggggccggc 2160

actactgtgt tggcggactg gcgggactag ggctgcgtga gtctctgagc gcaggcgggc 2220

ggcggccgcc cctcccccgg cggcggcagc ggcggcagcg gcggcagctc actcagcccg 2280

ctgcccgagc ggaaacgcca ctgaccgcac ggggattccc agtgccggcg ccaggggcac 2340

gcgggacacg ccccctcccg ccgcgccatt ggcctctccg cccaccgccc cacacttatt 2400

ggccggtgcg ccgccaatca gcggaggctg ccggggccgc ctaaagaaga ggctgtgctt 2460

tggggctccg gctcctcaga gagcctcggc taggtagggg atcgggactc tggcgggagg 2520

gcggcttggt gcgtttgcgg ggatgggcgg ccgcggcagg ccctccgagc gtggtggagc 2580

cgttctgtga gacagccggg tacgagtcgt gacgctggaa ggggcaagcg ggtggtgggc 2640

aggaatgcgg tccgccctgc agcaaccgga gggggaggga gaagggagcg gaaaagtctc 2700

caccggacgc ggccatggct cggggggggg ggggcagcgg aggagcgctt ccggccgacg 2760

tctcgtcgct gattggcttc ttttcctccc gccgtgtgtg aaaacacaaa tggcgtgttt 2820

tggttggcgt aaggcgcctg tcagttaacg gcagccggag tgcgcagccg ccggcagcct 2880

cgctctgccc actgggtggg gcgggaggta ggtggggtga ggcgagctgg acgtgcgggc 2940

gcggtcggcc tctggcgggg cgggggaggg gagggagggt cagcgaaagt agctcgcgcg 3000

cgagcggccg cccaccctcc ccttcctctg ggggagtcgt tttacccgcc gccggccggg 3060

cctcgtcgtc tgattggctc tcggggccca gaaaactggc ccttgccatt ggctcgtgtt 3120

cgtgcaagtt gagtccatcc gccggccagc gggggcggcg aggaggcgct cccaggttcc 3180

ggccctcccc tcggccccgc gccgcagagt ctggccgcgc gcccctgcgc aacgtggcag 3240

gaagcgcgcg ctgggggcgg ggacgggcag tagggctgag cggctgcggg gcgggtgcaa 3300

gcacgtttcc gacttgagtt gcctcaagag gggcgtgctg agccagacct ccatcgcgca 3360

ctccggggag tggagggaag gagcgagggc tcagttgggc tgttttggag gcaggaagca 3420

cttgctctcc caaagtcgct ctgagttgtt atcagtaagg gagctgcagt ggagtaggcg 3480

gggagaaggc cgcacccttc tccggagggg ggaggggagt gttgcaatac ctttctggga 3540

gttctctgct gcctcctggc ttctgaggac cgccctgggc ctgggagaat cccttccccc 3600

tcttccctcg tgatctgcaa ctccagtctt tctagaagat gggcgggagt cttctgggca 3660

ggcttaaagg ctaacctggt gtgtgggcgt tgtcctgcag gggaattgaa caggtgtaaa 3720

attggaggga caagacttcc cacagatttt cggttttgtc gggaagtttt ttaatagggg 3780

caaataagga aaatgggagg ataggtagtc atctggggtt ttatgcagca aaactacagg 3840

ttattattgc ttgtgatccg cctcggagta ttttccatcg aggtagatta aagacatgct 3900

cacccgagtt ttatactctc ctgcttgaga tccttactac agtatgaaat tacagtgtcg 3960

cgagttagac tatgtaagca gaattttaat catttttaaa gagcccagta cttcatatcc 4020

atttctcccg ctccttctgc agccttatca aaaggtattt tagaacactc atttagcccc 4080

cattttcatt tattatactg gcttatccaa cccctagaca gagcattggc attttccctt 4140

tcctgatctt agaagtctga tgactcatga aaccagacag attagttaca tacaccacaa 4200

atcgaggctg tagctggggc ctcaacactg cagttctttt ataactcctt agtacacttt 4260

ttgttgatcc tttgccttga tccttaattt tcagtgtcta tcacctctcc cgtcagtggt 4320

gttccacatt tgggcctatt ctcagtccag ggagttttac aacaatagat gtattgagaa 4380

tccaacctaa agcttaactt tccactccca tgaatgcctc tctccttttt ctccatttat 4440

aaactgagct attaaccatt aatggttcca ggtggatgtc tcctccccat attacctgat 4500

gtatcttaca tattgccagg ctgatatttt aagacattaa aaggtatatt tcattattga 4560

gccacatggt attgattact gcttactaaa attttgtcat tgtacacatc tgtaaaaggt 4620

ggttcctttt ggaatgcaaa gttcaggtgt ttgttgtctt tcctgaccta aggtcttgtg 4680

agcttgtatt ttttctattt aagcagtgct ttctcttgga ctggcttgac tcatggcatt 4740

ctacacgtta ttgctggtct aaatgtgatt ttgccaagct tcttcaggac ctataatttt 4800

gcttgacttg tagccaaaca caagtaaaat gattaagcaa caaatgtatt tgtgaagctt 4860

ggtttttagg ttgttgtgtt gtgtgtgctt gtgctctata ataatactat ccaggggctg 4920

gagaggtggc tcggagttca agagcacaga ctgctcttcc agaagtcctg agttcaattc 4980

ccagcaacca catggtggct cacaaccatc tgtaatggga tctgatgccc tcttctggtg 5040

tgtctgaaga ccacaagtgt attcacatta aataaataaa tcctccttct tcttcttttt 5100

ttttttttta aagagaatac tgtctccagt agaatttact gaagtaatga aatactttgt 5160

gtttgttcca atatggtagc caataatcaa attactcttt aagcactgga aatgttacca 5220

aggaactaat ttttatttga agtgtaactg tggacagagg agccataact gcagacttgt 5280
gggatacaga agaccaatgc agactttaat gtcttttctc ttacactaag caataaagaa 5340
ataaaaattg aacttctagt atcctatttg tttaaactgc tagctttact taacttttgt 5400
gcttcatcta tacaaagctg aaagctaagt ctgcagccat tactaaacat gaaagcaagt 5460
aatgataatt ttggatttca aaaatgtagg gccagagttt agccagccag tggtggtgct 5520
tgcctttatg cctttaatcc cagcactctg gaggcagaga caggcagatc tctgagtttg 5580
agcccagcct ggtctacaca tcaagttcta tctaggatag ccaggaatac acacagaaac 5640
cctgttgggg aggggggctc tgagatttca taaaattata attgaagcat tccctaatga 5700
gccactatgg atgtggctaa atccgtctac ctttctgatg agatttgggt attatttttt 5760
ctgtctctgc tgttggttgg gtcttttgac actgtgggct ttctttaaag cctccttcct 5820
gccatgtggt ctcttgtttg ctactaactt cccatggctt aaatggcatg gctttttgcc 5880
ttctaagggc agctgctgag atttgcagcc tgatttccag ggtggggttg ggaaatcttt 5940
caaacactaa aattgtcctt taattttttt tttaaaaaat gggttatata ataaacctca 6000
taaaatagtt atgaggagtg aggtggacta atattaaatg agtccctccc ctataaaaga 6060
gctattaagg ctttttgtct tatacttaac ttttttttta aatgtggtat ctttagaacc 6120
aagggtctta gagttttagt atacagaaac tgttgcatcg cttaatcaga ttttctagtt 6180
tcaaatccag agaatccaaa ttcttcacag ccaaagtcaa attaagaatt tctgactttt 6240
aatgttaatt tgcttactgt gaatataaaa atgatagctt ttcctgaggc agggtctcac 6300
tatgtatctc tgcctgatct gcaacaagat atgtagacta aagttctgcc tgcttttgtc 6360
tcctgaatac taaggttaaa atgtagtaat acttttggaa cttgcaggtc agattctttt 6420
ataggggaca cactaaggga gcttgggtga tagttggtaa aatgtgtttc aagtgatgaa 6480
aacttgaatt attatcaccg caacctactt tttaaaaaaa aaagccaggc ctgttagagc 6540
atgcttaagg gatccctagg acttgctgag cacacaagag tagttacttg gcaggctcct 6600
ggtgagagca tatttcaaaa aacaaggcag acaaccaaga aactacagtt aaggttacct 6660
gtctttaaac catctgcata tacacaggga tattaaaata ttccaaataa tatttcattc 6720
aagttttccc ccatcaaatt gggacatgga tttctccggt gaataggcag agttggaaac 6780
taaacaaatg ttggttttgt gatttgtgaa attgttttca agtgatagtt aaagcccatg 6840
agatacagaa caaagctgct atttcgaggt ctcttggttt atactcagaa gcacttcttt 6900
gggtttccct gcactatcct gatcatgtgc taggcctacc ttaggctgat tgttgttcaa 6960
ataaacttaa gtttcctgtc aggtgatgtc atatgatttc atatatcaag gcaaaacatg 7020
ttatatatgt taaacatttg tacttaatgt gaaagttagg tctttgtggg tttgattttt 7080

aattttcaaa acctgagcta aataagtcat ttttacatgt cttacatttg gtggaattgt 7140

ataattgtgg tttgcaggca agactctctg acctagtaac cctacctata gagcactttg 7200

ctgggtcaca agtctaggag tcaagcattt caccttgaag ttgagacgtt ttgttagtgt 7260

atactagttt atatgttgga ggacatgttt atccagaaga tattcaggac tatttttgac 7320

tgggctaagg aattgattct gattagcact gttagtgagc attgagtggc ctttaggctt 7380

gaattggagt cacttgtata tctcaaataa tgctggcctt ttttaaaaag cccttgttct 7440

ttatcaccct gttttctaca taatttttgt tcaaagaaat acttgtttgg atctcctttt 7500

gacaacaata gcatgttttc aagccatatt tttttccttt tttttttttt tttttggttt 7560

ttcgagacag ggtttctctg tatagccctg gctgtcctgg aactcacttt gtagaccagg 7620

ctggcctcga actcagaaat ccgcctgcct ctgcctcctg agtgccggga ttaaaggcgt 7680

gcaccaccac gcctggctaa gttggatatt ttgttatata actataacca atactaactc 7740

cactgggtgg atttttaatt cagtcagtag tcttaagtgg tctttattgg cccttcatta 7800

aaatctactg ttcactctaa cagaggctgt tggtactagt ggcacttaag caacttccta 7860

cggatatact agcagattaa gggtcaggga tagaaactag tctagcgttt tgtataccta 7920

ccagctttat actaccttgt tctgatagaa atatttcagg acatctagag tgtactataa 7980

ggttgatggt aagcttataa ggaacttgaa agtggagtaa ctactccatt tctctgaggg 8040

gagaattaaa atttttgacc aagtgttgtt gagccactga gaatggtctc agaacataac 8100

ttcttaagga accttcccag attgccctca acactgcacc acatttggtc ctgcttgaac 8160

attgccatgg ctcttaaagt cttaattaag aatattaatt gtgtaattat tgtttttcct 8220

cctttagatc attccttgag gacaggacag tgcttgttta aggctatatt tctgctgtct 8280

gagcagcaac aggtcttcga gatcaacatg atgttcataa tcccaagatg ttgccattta 8340

tgttctcaga agcaagcaga ggcatgatgg tcagtgacag taatgtcact gtgttaaatg 8400

ttgctatgca gtttggattt ttctaatgta gtgtaggtag aacatatgtg ttctgtatga 8460

attaaactct taagttacac cttgtataat ccatgcaatg tgttatgcaa ttaccatttt 8520

aagtattgta gctttctttg tatgtgagga taaaggtgtt tgtcataaaa tgttttgaac 8580

atttccccaa agttccaaat tataaaacca caacgttaga acttatttat gaacaatggt 8640

tgtagtttca tgcttttaaa atgcttaatt attcaattaa caccgtttgt gttataatat 8700

atataaaact gacatgtaga agtgtttgtc cagaacattt cttaaatgta tactgtcttt 8760

agagagttta atatagcatg tcttttgcaa catactaact tttgtgttgg tgcgagcaat 8820

attgtgtagt cattttgaaa ggagtcattt caatgagtgt cagattgttt tgaatgttat 8880

tgaacatttt aaatgcagac ttgttcgtgt tttagaaagc aaaactgtca gaagctttga 8940

actagaaatt aaaaagctga agtatttcag aagggaaata agctacttgc tgtattagtt 9000

gaaggaaagt gtaatagctt agaaaattta aaaccatata gttgtcattg ctgaatatct 9060

ggcagatgaa aagaaatact cagtggttct tttgagcaat ataacagctt gttatattaa 9120

aaattttccc cacagatata aactctaatc tataactcat aaatgttaca aatggatgaa 9180

gcttacaaat gtggcttgac ttgtcactgt gcttgtttta gttatgtgaa agtttggcaa 9240

taaacctatg tcctaaatag tcaaactgtg gaatgacttt ttaatctatt ggtttgtcta 9300

gaacagttat gttgccattt gccctaatgg tgaaagaaaa agtggggagt gccttggcac 9360

tgttcatttg tggtgtgaac caaagagggg ggcatgcact tacacttcaa acatcctttt 9420

gaaagactga caagtttggg tcttcacagt tggaattggg catccctttt gtcagggagg 9480

gagggaggga gggaggctgg cttgttatgc tgacaagtgt gattaaattc aaactttgag 9540

gtaagttgga ggaacttgta cattgttagg agtgtgacaa tttggactct taatgatttg 9600

gtcatacaaa atgaacctag accaacttct ggaagatgta tataataact ccatgttaca 9660

ttgatttcac ctgactaata cttatccctt atcaattaaa tacagaagat gccagccatc 9720

tgggcctttt aacccagaaa tttagtttca aactcctagg ttagtgttct cactgagcta 9780

catcctgatc tagtcctgaa aataggacca ccatcacccc caaaaaaatc tcaaataaga 9840

tttatgctag tgtttcaaaa ttttaggaat aggtaagatt agaaagtttt aaattttgag 9900

aaatggcttc tctagaaaga tgtacatagt gaacactgaa tggctcctaa agagcctaga 9960

aaactggtac tgagcacaca ggactgagag gtctttcttg aaaagcatgt attgctttac 10020

gtgggtcaca gaaggcaggc aggaagaact tgggctgaaa ctggtgtctt aagtggctaa 10080

catcttcaca actgatgagc aagaacttta tcctgatgca aaaaccatcc aaacaaacta 10140

agtgaaaggt ggcaatggat cccaggctgc tctagaggag gacttgactt ctcatcccat 10200

cacccacacc agatagctca tagactgcca attaacacca gcttctagcc tccacaggca 10260

cctgcactgg tacacataat ttcacacaaa cacagtaaga agccttccac ctggcatggt 10320

attgcttatc tttagttccc aacacttggg aggcagaggc cagccagggc tatgtgacaa 10380

aaaccttgtc tagaggagaa acttcatagc ttatttccta ttcacgtaac caggttagca 10440

aaatttacca gccagagatg aagctaacag tgtccactat atttgtagtg ttttaagtca 10500

attttttaaa tatacttaat agaattaaag ctatggtgaa ccaagtacaa acctggtgta 10560

ttaacttgag aacttagcat aaaaagtagt tcatttgttc agtaaatatt aaatgcttac 10620

tggcaaagat tatgtcagga acttggtaaa tggtgatgaa acaatcatag ttgtacatct 10680

tggttctgtg atcaccttgg tttgaggtaa aagtggttcc tttgatcaag gatggaattt 10740

taagtttata ttcaatcaat aatgtattat tttgtgattg caaaattgcc tatctagggt 10800

ataaaacctt taaaaatttc ataataccag ttcattctcc agttactaat tccaaaaagc 10860

cactgactat ggtgccaatg tggattctgt tctcaaagga aggattgtct gtgcccttta 10920

ttctaataga aacatcacac tgaaaatcta agctgaaaga agccagactt tcctaaataa 10980

ataactttcc ataaagctca aacaaggatt acttttagga ggcactgtta aggaactgat 11040

aagtaatgag gttacttata taatgatagt cccacaagac tatctgagga aaaatcagta 11100

caactcgaaa acagaacaac cagctaggca ggaataacag ggctcccaag tcaggaggtc 11160

tatccaacac ccttttctgt tgagggcccc agacctacat attgtataca aacagggagg 11220

tgggtgattt taactctcct gaggtacctt ggtaaatctt tgtcctgagt aagcagtaca 11280

gtgtacagtt tacattttca tttaaagata cattagctcc ctctaccccc taagactgac 11340

aggcactttg ggggtgggga gggctttgga aaataacgct tccatacact aaaagagaaa 11400

tttctttaat taggcttgtt ggttccatac atctactggt gtttctacta cttagtaata 11460

ttataatagt cacacaagca tctttgctct gtttaggttg tatatttatt ttaaggcaga 11520

tgataaaact gtagatctta agggatgctt ctgcttctga gatgatacaa agaatttaga 11580

ccataaaaca gtaggttgca caagcaatag aatatggcct aaagtgttct gacacttaga 11640

agccaagcag tgtaggcttc ttaagaaata ccattacaat caccttgcta gaaatcaagc 11700

attctggagt ggtcaagcag tgtaacctgt actgtaagtt acttttctgc tatttttctc 11760

ccaaagcaag ttctttatgc tgatatttcc agtgttagga actacaaata ttaataagtt 11820

gtcttcactc ttttctttac caaggagggt ctcttccttc atcttgatct gaaggatgaa 11880

caaaggcttg agcagtgcgc tttagaagat aaactgcagc atgaaggccc ccgatgttca 11940

cccagactac atggaccttt cgccacacat gtcccattcc agataaggcc tggcacacac 12000

aaaaaacata agtcattagg ctaccagtct gattctaaaa caacctaaaa tcttcccact 12060

taaatgctat gggtggtggg ttggaaagtt gactcagaaa atcacttgct gtttttagag 12120

aggatctggg ttcagtttct gatacattgt ggcttacaac tataactcca gttctagggg 12180

gtccatccaa catcctcttc tgttgagggc accaaataaa tgtattgtgt acaaacaggg 12240

aggtgagtga tttaactctc gtgtatagta ccttggtaaa acatttcttg tcctgagtaa 12300

gcagtacagc tctgcctgtc cctggtctac agacacggct catttcccga aggcaagctg 12360

gatagagatt ccaatttctc ttcttggatc ccatcctata aaagaaggtc aagtttaatc 12420

tattgcaaaa ggtaaatagg tagtttctta catgagacaa gaacaaatct taggtgtgaa 12480

gcagtcatct tttacaggcc agagcctcta ttctatgcca atgaaggaaa ctgttagtcc 12540

agtgttatag agttagtcca gtgtatagtt ttctatcaga acactttttt tttaaacaac 12600

tgcaacttag cttattgaag acaaaccacg agtagaaatc tgtccaagaa gcaagtgctt 12660

ctcagcctac aatgtggaat aggaccatgt aatggtacag tgagtgaaat gaattatggc 12720

atgtttttct gactgagaag acagtacaat aaaaggtaaa ctcatggtat ttatttaaaa 12780

agaatccaat ttctaccttt ttccaaatgg catatctgtt acaataatat ccacagaagc 12840

agttctcagt gggaggttgc agatatccca ctgaacagca tcaatgggca aaccccaggt 12900

tgtttttctg tggagacaaa ggtaagatat ttcaatatat tttcccaagc taatgagatg 12960

gctcagcaaa taatggtact ggccattaag tctcatgacc tgagcttgat cctcagggac 13020

catgtggtac aaggagagac ctaaatcctt cagttggact tcaatcttct accctcatgt 13080

ccacacacaa ataaatacaa taaaaaacat tctgcagtcg aatttctaaa agggcgaat 13139

<210> 28
<211> 15200
<212> DNA
<213> Artificial

<220>
<223> Rosa 26 targeting vector

<400> 28
ttacgctagg gataacaggg taatatagcc gcggcaggcc ctccgagcgt ggtggagccg 60

ttctgtgaga cagccgggta cgagtcgtga cgctggaagg ggcaagcggg tggtgggcag 120

gaatgcggtc cgccctgcag caaccggagg gggagggaga agggagcgga aaagtctcca 180

ccggacgcgg ccatggctcg gggggggggg ggcagcggag gagcgcttcc ggccgacgtc 240

tcgtcgctga ttggcttctt ttcctcccgc cgtgtgtgaa aacacaaatg gcgtgttttg 300

gttggcgtaa ggcgcctgtc agttaacggc agccggagtg cgcagccgcc ggcagcctcg 360

ctctgcccac tgggtggggc gggaggtagg tggggtgagg cgagctggac gtgcgggcgc 420

ggtcggcctc tggcggggcg ggggagggga gggagggtca gcgaaagtag ctcgcgcgcg 480

agcggccgcc caccctcccc ttcctctggg ggagtcgttt tacccgccgc cggccgggcc 540

tcgtcgtctg attggctctc ggggcccaga aaactggccc ttgccattgg ctcgtgttcg 600

tgcaagttga gtccatccgc cggccagcgg gggcggcgag gaggcgctcc caggttccgg 660

ccctcccctc ggccccgcgc cgcagagtct ggccgcgcgc ccctgcgcaa cgtggcagga 720

agcgcgcgct gggggcgggg acgggcagta gggctgagcg gctgcggggc gggtgcaagc 780

acgtttccga cttgagttgc ctcaagaggg gcgtgctgag ccagacctcc atcgcgcact 840

ccggggagtg gagggaagga gcgagggctc agttgggctg ttttggaggc aggaagcact 900

tgctctccca aagtcgctct gagttgttat cagtaaggga gctgcagtgg agtaggcggg 960

gagaaggccg cacccttctc cggagggggg aggggagtgt tgcaatacct ttctgggagt 1020

tctctgctgc ctcctggctt ctgaggaccg ccctgggcct gggagaatcc cttccccctc 1080

ttccctcgtg atctgcaact ccagtctttc taggtaaccg atatccctgc aggggtgacc 1140

tgcacgtcta gggcgcagta gtccagggtt tccttgatga tgtcatactt atcctgtccc 1200

ttttttttcc acagctcgcg gttgaggaca aactcttcgc ggtctttcca gtactcctgc 1260

aggtgactga ctgagtcgac gccaccatgg agaagttcct attccgaagt tcctattctt 1320

caaatagtat aggaacttcg ctcgagctca agcttcgaat tctgcagtcg acggtaccgc 1380

gggcccggga tccaccggtc gccaccatgg cccagtccaa gcacggcctg accaaggaga 1440

tgaccatgaa gtaccgcatg gagggctgcg tggacggcca caagttcgtg atcaccggcg 1500

agggcatcgg ctaccccttc aagggcaagc aggccatcaa cctgtgcgtg gtggagggcg 1560

gccccttgcc cttcgccgag gacatcttgt ccgccgcctt catgtacggc aaccgcgtgt 1620

tcaccgagta cccccaggac atcgtcgact acttcaagaa ctcctgcccc gccggctaca 1680

cctgggaccg ctccttcctg ttcgaggacg gcgccgtgtg catctgcaac gccgacatca 1740

ccgtgagcgt ggaggagaac tgcatgtacc acgagtccaa gttctacggc gtgaacttcc 1800

ccgccgacgg ccccgtgatg aagaagatga ccgacaactg ggagccctcc tgcgagaaga 1860

tcatccccgt gcccaagcag ggcatcttga agggcgacgt gagcatgtac ctgctgctga 1920

aggacggtgg ccgcttgcgc tgccagttcg acaccgtgta caaggccaag tccgtgcccc 1980

gcaagatgcc cgactggcac ttcatccagc acaagctgac ccgcgaggac cgcagcgacg 2040

ccaagaacca gaagtggcac ctgaccgagc acgccatcgc ctccggctcc gccttgccct 2100

gagcggccga ccggttcgag atccaggcgc ggatcaataa aagatcatta ttttcaatag 2160

atctgtgtgt tggttttttg tgtgccttgg gggaggggga ggccagaatg aggcgcggcc 2220

aaggggggagg gggaggccag aatgaccttg gggagggggg aggccagaat gaccttgggg 2280

gagggggagg ccagaatgag gcgcgtatgc aggtcaattc taccgggtag gggaggcgct 2340

tttcccaagg cagtctggag catgcgcttt agcagccccg ctggcacttg gcgctacaca 2400

agtggcctct ggcctcgcac acattccaca tccaccggta gcgccaaccg gctccgttct 2460

ttggtggccc cttcgcgcca ccttctactc ctcccctagt caggaagttc ccccccgccc 2520

cgcagctcgc gtcgtgcagg acgtgacaaa tggaagtagc acgtctcact agtctcgtgc 2580

agatggacag caccgctgag caatggaagc gggtaggcct ttggggcagc ggccaatagc 2640

agctttgctc cttcgctttc tgggctcaga ggctgggaag gggtgggtcc gggggcgggc 2700

tcaggggcgg gctcaggggc ggggcgggcg cgaaggtcct cccgaggccc ggcattctcg 2760

cacgcttcaa aagcgcacgt ctgccgcgct gttctcctct tcctcatctc cgggcctttc 2820

gacgatccag ccgccaccat gaaaaagcct gaactcaccg cgacgtctgt cgagaagttt 2880

ctgatcgaaa agttcgacag cgtctccgac ctgatgcagc tctcggaggg cgaagaatct 2940

cgtgctttca gcttcgatgt aggagggcgt ggatatgtcc tgcgggtaaa tagctgcgcc 3000

gatggtttct acaaagatcg ttatgtttat cggcactttg catcggccgc gctcccgatt 3060

ccggaagtgc ttgacattgg ggaattcagc gagagcctga cctattgcat ctcccgccgt 3120

gcacagggtg tcacgttgca agacctgcct gaaaccgaac tgcccgctgt tctgcagccg 3180

gtcgcggagg ccatggatgc catcgctgcg gccgatctta gccagacgag cgggttcggc 3240

ccattcggac cgcaaggaat cggtcaatac actacatggc gtgatttcat atgcgcgatt 3300

gctgatcccc atgtgtatca ctggcaaact gtgatggacg acaccgtcag tgcgtccgtc 3360

gcgcaggctc tcgatgagct gatgctttgg gccgaggact gccccgaagt ccggcacctc 3420

gtgcacgcgg atttcggctc caacaatgtc ctgacggaca atggccgcat aacagcggtc 3480

attgactgga gcgaggcgat gttcggggat tcccaatacg aggtcgccaa catcttcttc 3540

tggaggccgt ggttggcttg tatggagcag cagacgcgct acttcgagcg gaggcatccg 3600

gagcttgcag gatcgccgcg gctccgggcg tatatgctcc gcattggtct tgaccaactc 3660

tatcagagct tggttgacgg caatttcgat gatgcagctt gggcgcaggg tcgatgcgac 3720

gcaatcgtcc gatccggagc cgggactgtc gggcgtacac aaatcgcccg cagaagcgcg 3780

gccgtctgga ccgatggctg tgtagaagta ctcgccgata gtggaaaccg acgccccagc 3840

actcgtccga gggcaaagga atagtcgatg cagaaattga tgatctatta aacaataaag 3900

atgtccacta aaatggaagt ttttcctgtc atactttgtt aagaagggtg agaacagagt 3960

acctacattt tgaatggaag gattggagct acgggggtgg gggtggggtg ggattagata 4020

aatgcctgct ctttactgaa ggctctttac tattgcttta tgataatgtt tcatagttgg 4080

atatcataat ttaaacaagc aaaaccaaat taagggccag ctcattcctc ccactcatga 4140

tctatagatc tatagatctc tcgtgggatc attgtttttc tcttgattcc cactttgtgg 4200

ttctaagtac tgtggtttcc aaatgtgtca gtttcatagc ctgaagaacg agatcagcag 4260

cctctgttcc acatacactt cattctcagt attgttttgc caagttctaa ttccatcaga 4320

agctgactct agatcctgca ttaattaagg ccatagcggc catttgggct agcttgcatg 4380

cctgcaggtt ttcgacattg attattgact agttattaat agtaatcaat tacggggtca 4440

ttagttcata gcccatatat ggagttccgc gttacataac ttacggtaaa tggcccgcct 4500

ggctgaccgc ccaacgaccc ccgcccattg acgtcaataa tgacgtatgt tcccatagta 4560

acgccaatag ggactttcca ttgacgtcaa tgggtggact atttacggta aactgcccac 4620

ttggcagtac atcaagtgta tcatatgcca agtacgcccc ctattgacgt caatgacggt 4680

aaatggcccg cctggcatta tgcccagtac atgaccttat gggactttcc tacttggcag 4740

tacatctacg tattagtcat cgctattacc atgggtcgag gtgagcccca cgttctgctt 4800

cactctcccc atctcccccc cctccccacc cccaattttg tatttattta ttttttaatt 4860

attttgtgca gcgatggggg cggggggggg gggggcgcgc gccaggcggg gcggggcggg 4920

gcgaggggcg gggcggggcg aggcggagag gtgcggcggc agccaatcag agcggcgcgc 4980

tccgaaagtt tccttttatg gcgaggcggc ggcggcggcg gccctataaa aagcgaagcg 5040

cgcggcgggc gggagtcgct gcgttgcctt cgccccgtgc cccgctccgc gccgcctcgc 5100

gccgcccgcc ccggctctga ctgaccgcgt tactcccaca ggtgagcggg cgggacggcc 5160

cttctcctcc gggctgtaat tagcgcttgg tttaatgacg gctcgtttct tttctgtggc 5220

tgcgtgaaag ccttaaaggg ctccgggagg gccctttgtg cgggggggag cggctcgggg 5280

ggtgcgtgcg tgtgtgtgtg cgtggggagc gccgcgtgcg gcccgcgctg cccggcggct 5340

gtgagcgctg cgggcgcggc gcggggcttt gtgcgctccg cgtgtgcgcg aggggagcgc 5400

ggccgggggc ggtgccccgc ggtgcggggg ggctgcgagg ggaacaaagg ctgcgtgcgg 5460

ggtgtgtgcg tgggggggtg agcagggggt gtgggcgcgg cggtcgggct gtaacccccc 5520

cctgcacccc cctccccgag ttgctgagca cggcccggct tcgggtgcgg ggctccgtgc 5580

ggggcgtggc gcggggctcg ccgtgccggg cggggggtgg cggcaggtgg gggtgccggg 5640

cggggcgggg ccgcctcggg ccggggaggg ctcgggggag gggcgcggcg gccccggagc 5700

gccggcggct gtcgaggcgc ggcgagccgc agccattgcc ttttatggta atcgtgcgag 5760

agggcgcagg gacttccttt gtcccaaatc tggcggagcc gaaatctggg aggcgccgcc 5820

gcaccccctc tagcgggcgc gggcgaagcg gtgcggcgcc ggcaggaagg aaatgggcgg 5880

ggagggcctt cgtgcgtcgc cgcgccgccg tccccttctc catctccagc ctcggggctg 5940

ccgcaggggg acggctgcct tcggggggga cggggcaggg cggggttcgg cttctggcgt 6000

gtgaccggcg gctctagtaa gcgttggggt gagtactccc tctcaaaagc gggcatgact 6060

tctgcgctaa gattgtcagt ttccaaaaac gaggaggatt tgatattcac ctggcccgcg 6120

gtgatgcctt tgagggtggc cgcgtccatc tggtcagaaa agacaatctt tttgttgtca 6180

agcttgaggt gtggcaggct tgagatctgg ccatacactt gagtgacatt gacatccact 6240

ttgcctttct ctccacaggt gtccactccc agggcggccc tagagcctct gctaaccatg 6300

ttcatgcctt cttctttttc ctacagctcc tgggcaacgt gctggttgtt gtgctgtctc 6360

atcattttgg caaagaatta cccgccgcca ccatggctcc caagaagaag aggaaggtga 6420

tgagtcaatt tgatatatta tgtaaaacac cacctaaggt cctggttcgt cagtttgtgg 6480

aaaggtttga aagaccttca ggggaaaaaa tagcatcatg tgctgctgaa ctaacctatt 6540

tatgttggat gattactcat aacggaacag caatcaagag agccacattc atgagctata 6600

atactatcat aagcaattcg ctgagtttcg atattgtcaa caaatcactc cagtttaaat 6660

acaagacgca aaaagcaaca attctggaag cctcattaaa gaaattaatt cctgcttggg 6720

aatttacaat tattccttac aatggacaaa aacatcaatc tgatatcact gatattgtaa 6780

gtagtttgca attacagttc gaatcatcgg aagaagcaga taagggaaat agccacagta 6840

aaaaaatgct taaagcactt ctaagtgagg gtgaaagcat ctgggagatc actgagaaaa 6900

tactaaattc gtttgagtat acctcgagat ttacaaaaac aaaaacttta taccaattcc 6960

tcttcctagc tactttcatc aattgtggaa gattcagcga tattaagaac gttgatccga 7020

aatcatttaa attagtccaa aataagtatc tgggagtaat aatccagtgt ttagtgacag 7080

agacaaagac aagcgttagt aggcacatat acttctttag cgcaaggggt aggatcgatc 7140

cacttgtata tttggatgaa tttttgagga attctgaacc agtcctaaaa cgagtaaata 7200

ggaccggcaa ttcttcaagc aacaaacagg aataccaatt attaaaagat aacttagtca 7260

gatcgtacaa caaggctttg aagaaaaatg cgccttatcc aatctttgct ataaagaatg 7320

gcccaaaatc tcacattgga agacatttga tgacctcatt tctgtcaatg aagggcctaa 7380

cggagttgac taatgttgtg ggaaattgga gcgataagcg tgcttctgcc gtggccagga 7440

caacgtatac tcatcagata acagcaatac ctgatcacta cttcgcacta gtttctcggt 7500

actatgcata tgatccaata tcaaaggaaa tgatagcatt gaaggatgag actaatccaa 7560

ttgaggagtg gcagcatata gaacagctaa agggtagtgc tgaaggaagc atacgatacc 7620

ccgcatggaa tgggataata tcacaggagg tactagacta cctttcatcc tacataaata 7680

gacgcatata atgatctaga gtcgagttag gccgcgtcga cctcgagatc caggcgcgga 7740

tcaataaaag atcattattt tcaatagatc tgtgtgttgg ttttttgtgt gccttggggg 7800

aggggaggc cagaatgagg cgcggccaag gggagggggg aggccagaat gaccttgggg 7860

gagggggagg ccagaatgac cttgggggag ggggaggcca gaatgaggcg cgccggtaac 7920

cgaagttcct atactttcta gagaatagga acttcggaat aggaacttca agcttaagcg 7980

ctagaagatg ggcgggagtc ttctgggcag gcttaaaggc taacctggtg tgtgggcgtt 8040

gtcctgcagg ggaattgaac aggtgtaaaa ttggagggac aagacttccc acagattttc 8100

ggttttgtcg ggaagttttt taataggggc aaataaggaa aatgggagga taggtagtca 8160

tctggggttt tatgcagcaa aactacaggt tattattgct tgtgatccgc ctcggagtat 8220

tttccatcga ggtagattaa agacatgctc acccgagttt tatactctcc tgcttgagat 8280

ccttactaca gtatgaaatt acagtgtcgc gagttagact atgtaagcag aattttaatc 8340

atttttaaag agcccagtac ttcatatcca tttctcccgc tccttctgca gccttatcaa 8400

aaggtatttt agaacactca ttttagcccc attttcattt attatactgg cttatccaac 8460

ccctagacag agcattggca ttttcccttt cctgatctta gaagtctgat gactcatgaa 8520

accagacaga ttagttacat acaccacaaa tcgaggctgt agctggggcc tcaacactgc 8580

agttctttta taactcctta gtacactttt tgttgatcct ttgccttgat ccttaatttt 8640

cagtgtctat cacctctccc gtcagtggtg ttccacattt gggcctattc tcagtccagg 8700

gagttttaca acaatagatg tattgagaat ccaacctaaa gcttaacttt ccactcccat 8760

gaatgcctct ctcctttttc tccatttata aactgagcta ttaaccatta atggttccag 8820

gtggatgtct cctccccata ttacctgatg tatcttacat attgccaggc tgatatttta 8880

agacattaaa aggtatattt cattattgag ccacatggta ttgattactg cttactaaaa 8940

ttttgtcatt gtacacatct gtaaaaggtg gttccttttg gaatgcaaag ttcaggtgtt 9000

tgttgtcttt cctgacctaa ggtcttgtga gcttgtattt tttctattta agcagtgctt 9060

tctcttggac tggcttgact catggcattc tacacgttat tgctggtcta aatgtgattt 9120

tgccaagctt cttcaggacc tataattttg cttgacttgt agccaaacac aagtaaaatg 9180

attaagcaac aaatgtattt gtgaagcttg gtttttaggt tgttgtgttg tgtgtgcttg 9240

tgctctataa taatactatc caggggctgg agaggtggct cggagttcaa gagcacagac 9300

tgctcttcca gaagtcctga gttcaattcc cagcaaccac atggtggctc acaaccatct 9360

gtaatgggat ctgatgccct cttctggtgt gtctgaagac cacaagtgta ttcacattaa 9420

ataaataaat cctccttctt cttctttttt ttttttttaa agagaatact gtctccagta 9480

gaatttactg aagtaatgaa atactttgtg tttgttccaa tatggtagcc aataatcaaa 9540

ttactcttta agcactggaa atgttaccaa ggaactaatt tttatttgaa gtgtaactgt 9600

ggacagagga gccataactg cagacttgtg ggatacagaa gaccaatgca gactttaatg 9660

tcttttctct tacactaagc aataaagaaa taaaaattga acttctagta tcctatttgt 9720

ttaaactgct agctttactt aacttttgtg cttcatctat acaaagctga aagctaagtc 9780

tgcagccatt actaaacatg aaagcaagta atgataattt tggatttcaa aaatgtaggg 9840

ccagagttta gccagccagt ggtggtgctt gcctttatgc ctttaatccc agcactctgg 9900

aggcagagac aggcagatct ctgagtttga gcccagcctg gtctacacat caagttctat 9960

ctaggatagc caggaataca cacagaaacc ctgttgggga ggggggctct gagatttcat 10020

aaaattataa ttgaagcatt ccctaatgag ccactatgga tgtggctaaa tccgtctacc 10080

tttctgatga gatttgggta ttattttttc tgtctctgct gttggttggg tcttttgaca 10140

ctgtgggctt tctttaaagc ctccttcctg ccatgtggtc tcttgtttgc tactaacttc 10200

ccatggctta aatggcatgg ctttttgcct tctaagggca gctgctgaga tttgcagcct 10260

gatttccagg gtggggttgg gaaatctttc aaacactaaa attgtccttt aatttttttt 10320

ttaaaaaatg ggttatataa taaacctcat aaaatagtta tgaggagtga ggtggactaa 10380

tattaaatga gtccctcccc tataaaagag ctattaaggc tttttgtctt atacttaact 10440

ttttttttaa atgtggtatc tttagaacca agggtcttag agttttagta tacagaaact 10500

gttgcatcgc ttaatcagat tttctagttt caaatccaga gaatccaaat tcttcacagc 10560

caaagtcaaa ttaagaattt ctgactttta atgttaattt gcttactgtg aatataaaaa 10620

tgatagcttt tcctgaggca gggtctcact atgtatctct gcctgatctg caacaagata 10680

tgtagactaa agttctgcct gcttttgtct cctgaatact aaggttaaaa tgtagtaata 10740

cttttggaac ttgcaggtca gattctttta taggggacac actaagggag cttgggtgat 10800

agttggtaaa atgtgtttca agtgatgaaa acttgaatta ttatcaccgc aacctacttt 10860

ttaaaaaaaa aagccaggcc tgttagagca tgcttaaggg atccctagga cttgctgagc 10920

acacaagagt agttacttgg caggctcctg gtgagagcat atttcaaaaa acaaggcaga 10980

caaccaagaa actacagtta aggttacctg tctttaaacc atctgcatat acacagggat 11040

attaaaatat tccaaataat atttcattca agttttcccc catcaaattg ggacatggat 11100

ttctccggtg aataggcaga gttggaaact aaacaaatgt tggttttgtg atttgtgaaa 11160

ttgttttcaa gtgatagtta aagcccatga gatacagaac aaagctgcta tttcgaggtc 11220

tcttggttta tactcagaag cacttctttg ggtttccctg cactatcctg atcatgtgct 11280

aggcctacct taggctgatt gttgttcaaa taaacttaag tttcctgtca ggtgatgtca 11340

tatgatttca tatatcaagg caaaacatgt tatatatgtt aaacatttgt acttaatgtg 11400

aaagttaggt ctttgtgggt ttgattttta attttcaaaa cctgagctaa ataagtcatt 11460

tttacatgtc ttacatttgg tggaattgta taattgtggt ttgcaggcaa gactctctga 11520

cctagtaacc ctacctatag agcactttgc tgggtcacaa gtctaggagt caagcatttc 11580

accttgaagt tgagacgttt tgttagtgta tactagttta tatgttggag gacatgttta 11640

tccagaagat attcaggact atttttgact gggctaagga attgattctg attagcactg 11700

ttagtgagca ttgagtggcc tttaggcttg aattggagtc acttgtatat ctcaaataat 11760

gctggccttt tttaaaaagc ccttgttctt tatcaccctg ttttctacat aatttttgtt 11820

caaagaaata cttgtttgga tctccttttg acaacaatag catgttttca agccatattt 11880

tttttccttt tttttttttt ttttggtttt tcgagacagg gtttctctgt atagccctgg 11940

ctgtcctgga actcactttg tagaccaggc tggcctcgaa ctcagaaatc cgcctgcctc 12000

tgcctcctga gtgccgggat taaaggcgtg caccaccacg cctggctaag ttggatattt 12060

tgttatataa ctataaccaa tactaactcc actgggtgga tttttaattc agtcagtagt 12120

cttaagtggt ctttattggc ccttcattaa aatctactgt tcactctaac agaggctgtt 12180

ggtactagtg gcacttaagc aacttcctac ggatatacta gcagattaag ggtcagggat 12240

agaaactagt ctagcgtttt gtatacctac cagctttata ctaccttgtt ctgatagaaa 12300

tatttcagga catctagcac ccaattcgcc ctatagtgag tcgtattaca attcactggc 12360

cgtcgtttta caacgtcgtg actgggaaaa ccctggcgtt acccaactta atcgccttgc 12420

agcacatccc cctttcgcca gctggcgtaa tagcgaagag gcccgcaccg atcgcccttc 12480

ccaacagttg cgcagcctga atggcgaatg ggacgcgccc tgtagcggcg cattaagcgc 12540

ggcgggtgtg gtggttacgc gcagcgtgac cgctacactt gccagcgccc tagcgcccgc 12600

tcctttcgct ttcttccctt cctttctcgc cacgttcgcc ggctttcccc gtcaagctct 12660

aaatcggggg ctccctttag ggttccgatt tagtgcttta cggcacctcg accccaaaaa 12720

acttgattag ggtgatggtt cacgtagtgg gccatcgccc tgatagacgg tttttcgccc 12780

tttgacgttg gagtccacgt tctttaatag tggactcttg ttccaaactg gaacaacact 12840

caaccctatc tcggtctatt cttttgattt ataagggatt ttgccgattt cggcctattg 12900

gttaaaaaat gagctgattt aacaaaaatt taacgcgaat tttaacaaaa tattaacgct 12960

tacaatttag gtggcacttt tcggggaaat gtgcgcggaa cccctatttg tttatttttc 13020

taaatacatt caaatatgta tccgctcatg agacaataac cctgataaat gcttcaataa 13080

tattgaaaaa ggaagagtat gagtattcaa catttccgtg tcgcccttat tccctttttt 13140

gcggcatttt gccttcctgt ttttgctcac ccagaaacgc tggtgaaagt aaaagatgct 13200

gaagatcagt tgggtgcacg agtgggttac atcgaactgg atctcaacag cggtaagatc 13260

cttgagagtt ttcgccccga agaacgtttt ccaatgatga gcacttttaa agttctgcta 13320

tgtggcgcgg tattatcccg tattgacgcc gggcaagagc aactcggtcg ccgcatacac 13380

tattctcaga atgacttggt tgagtactca ccagtcacag aaaagcatct tacggatggc 13440

atgacagtaa gagaattatg cagtgctgcc ataaccatga gtgataacac tgcggccaac 13500

ttacttctga caacgatcgg aggaccgaag gagctaaccg cttttttgca caacatgggg 13560

gatcatgtaa ctcgccttga tcgttgggaa ccggagctga atgaagccat accaaacgac 13620

gagcgtgaca ccacgatgcc tgtagcaatg gcaacaacgt tgcgcaaact attaactggc 13680

gaactactta ctctagcttc ccggcaacaa ttaatagact ggatggaggc ggataaagtt 13740

gcaggaccac ttctgcgctc ggcccttccg gctggctggt ttattgctga taaatctgga 13800

gccggtgagc gtgggtctcg cggtatcatt gcagcactgg ggccagatgg taagccctcc 13860

cgtatcgtag ttatctacac gacggggagt caggcaacta tggatgaacg aaatagacag 13920

atcgctgaga taggtgcctc actgattaag cattggtaac tgtcagacca agtttactca 13980

tatatacttt agattgattt aaaacttcat ttttaattta aaaggatcta ggtgaagatc 14040

ctttttgata atctcatgac caaaatccct taacgtgagt tttcgttcca ctgagcgtca 14100

gaccccgtag aaaagatcaa aggatcttct tgagatcctt tttttctgcg cgtaatctgc 14160

tgcttgcaaa caaaaaaacc accgctacca gcggtggttt gtttgccgga tcaagagcta 14220

ccaactcttt ttccgaaggt aactggcttc agcagagcgc agataccaaa tactgtcctt 14280

ctagtgtagc cgtagttagg ccaccacttc aagaactctg tagcaccgcc tacatacctc 14340

gctctgctaa tcctgttacc agtggctgct gccagtggcg ataagtcgtg tcttaccggg 14400

ttggactcaa gacgatagtt accggataag gcgcagcggt cgggctgaac ggggggttcg 14460

tgcacacagc ccagcttgga gcgaacgacc tacaccgaac tgagatacct acagcgtgag 14520

ctatgagaaa gcgccacgct tcccgaaggg agaaaggcgg acaggtatcc ggtaagcggc 14580

agggtcggaa caggagagcg cacgagggag cttccagggg gaaacgcctg gtatctttat 14640

agtcctgtcg ggtttcgcca cctctgactt gagcgtcgat ttttgtgatg ctcgtcaggg 14700

gggcggagcc tatggaaaaa cgccagcaac gcggcctttt tacggttcct ggccttttgc 14760

tggccttttg ctcacatgtt ctttcctgcg ttatcccctg attctgtgga taaccgtatt 14820

accgcctttg agtgagctga taccgctcgc cgcagccgaa cgaccgagcg cagcgagtca 14880

gtgagcgagg aagcggaaga gcgcccaata cgcaaaccgc ctctccccgc gcgttggccg 14940

attcattaat gcagctggca cgacaggttt cccgactgga aagcgggcag tgagcgcaac 15000

gcaattaatg tgagttagct cactcattag gcaccccagg ctttacactt tatgcttccg 15060

gctcgtatgt tgtgtggaat tgtgagcgga taacaatttc acacaggaaa cagctatgac 15120

catgattacg ccaagcgcgc aattaaccct cactaaaggg aacaaaagct gtcgagatct 15180

agatatcgat ggccatagag 15200

<210> 29
<211> 47
<212> DNA
<213> Artificial

<220>
<223> shRNA

<400> 29
tgagaagtct cccagtcagt tcaagagact gactgggaga cttctca 47

<210> 30
<211> 47
<212> DNA
<213> Artificial

<220>
<223> shRNA

<400> 30
gactccagtg gtaatctact tcaagagagt agattaccac tggagtc 47

<210> 31
<211> 47
<212> DNA
<213> Artificial

<220>
<223> shRNA

<400> 31
cggcaggact ccgggccgat tcaagagatc ggcccggagt cctgccg 47

<210>  32
<211>  47
<212>  DNA
<213>  Artificial

<220>
<223>  shRNA

<400>  32
cctcatgcag ttctctttgt tcaagagaca aagagaactg catgagg                47


<210>  33
<211>  50
<212>  DNA
<213>  Artificial

<220>
<223>  shRNA

<400>  33
aagatgaagc cactccctat ttcaagagaa aatagggagt ggcttcatct            50


<210>  34
<211>  41
<212>  DNA
<213>  Artificial

<220>
<223>  shRNA

<400>  34
gacagagcca agtggactca cagagtccac ttggctctgt c                     41


<210>  35
<211>  42
<212>  DNA
<213>  Artificial

<220>
<223>  shRNA

<400>  35
ctggacttcc agaagaacat tcgtgttctt ctggaagtcc ag                    42


<210>  36
<211>  47
<212>  DNA
<213>  Artificial

<220>
<223>  shRNA

<400>  36
gagattggtc cagaacagtt tcaagagaac tgttctggac caatctc               47


<210>  37
<211>  47

<212>  DNA
<213>  Artificial

<220>
<223>  shRNA

<400>  37
gcccttccga tcatggtagt tcaagagact accatgatcg gaagggc                47


<210>  38
<211>  47
<212>  DNA
<213>  Artificial

<220>
<223>  shRNA

<400>  38
tctttagaat tcttaagtat tcaagagata cttaagaatt ctaaaga                47


<210>  39
<211>  47
<212>  DNA
<213>  Artificial

<220>
<223>  shRNA

<400>  39
cattagctat atcaacatgt tcaagagaca tgttgatata gctaatg                47


<210>  40
<211>  47
<212>  DNA
<213>  Artificial

<220>
<223>  shRNA

<400>  40
accacaaacg gcggaacgat tcaagagatc gttccgccgt ttgtggt                47


<210>  41
<211>  47
<212>  DNA
<213>  Artificial

<220>
<223>  shRNA

<400>  41
gagggtcttg gaggtcttct tcaagagaga agacctccaa gaccctc                47


<210>  42
<211>  47
<212>  DNA
<213>  Artificial

<220>
<223>  shRNA

<400>  42
gtccatgccc agccgtacat tcaagagatg tacggctggg catggac                    47


<210>  43
<211>  47
<212>  DNA
<213>  Artificial

<220>
<223>  shRNA

<400>  43
gctggacacc ctcgtggagt tcaagagact ccacgagggt gtccagc                    47


<210>  44
<211>  47
<212>  DNA
<213>  Artificial

<220>
<223>  shRNA

<400>  44
gaatatcaga gaattgagtt tcaagagaac tcaattctct gatattc                    47


<210>  45
<211>  47
<212>  DNA
<213>  Artificial

<220>
<223>  shRNA

<400>  45
tggacttcat gaggaaatgt tcaagagaca tttcctcatg aagtcca                    47


<210>  46
<211>  47
<212>  DNA
<213>  Artificial

<220>
<223>  shRNA

<400>  46
tattgaatat cctgtggact tcaagagagt ccacaggata ttcaata                    47


<210>  47
<211>  47
<212>  DNA
<213>  Artificial

<220>
<223>  shRNA

<400>  47
ttgtactgag agaaactgct tcaagagagc agtttctctc agtacaa                47

<210>  48
<211>  47
<212>  DNA
<213>  Artificial

<220>
<223>  shRNA

<400>  48
gatcaatgat aggtttgaat tcaagagatt caaacctatc attgatc                47

<210>  49
<211>  47
<212>  DNA
<213>  Artificial

<220>
<223>  shRNA

<400>  49
ggagtttgag aagtttaaat tcaagagatt taaacttctc aaactcc                47

<210>  50
<211>  47
<212>  DNA
<213>  Artificial

<220>
<223>  shRNA

<400>  50
gaactcctcg cttgctgagt tcaagagact cagcaagcga ggagttc                47

<210>  51
<211>  47
<212>  DNA
<213>  Artificial

<220>
<223>  shRNA

<400>  51
ccgaatttaa cagagagaat tcaagagatt ctctctgtta aattcgg                47

<210>  52
<211>  47
<212>  DNA
<213>  Artificial

<220>
<223>  shRNA

<400>  52
gacagcagaa gaatgcagat tcaagagatc tgcattcttc tgctgtc                47

<210>  53
<211>  47
<212>  DNA
<213>  Artificial

<220>
<223>  shRNA

<400>  53
ataaagctca acgagaacct tcaagagagg ttctcgttga gctttat                47


<210>  54
<211>  47
<212>  DNA
<213>  Artificial

<220>
<223>  shRNA

<400>  54
ggtgaagtgg cagaagaatt tcaagagaat tcttctgcca cttcacc                47


<210>  55
<211>  47
<212>  DNA
<213>  Artificial

<220>
<223>  shRNA

<400>  55
gtattgcagt aatcatcact tcaagagagt gatgattact gcaatac                47


<210>  56
<211>  47
<212>  DNA
<213>  Artificial

<220>
<223>  shRNA

<400>  56
gatatggggt tccatgtcat tcaagagatg acatggaacc ccatatc                47


<210>  57
<211>  47
<212>  DNA
<213>  Artificial

<220>
<223>  shRNA

<400>  57
ggagacatgg ttcttagtgt tcaagagaca ctaagaacca tgtctcc                47


<210>  58
<211>  47

<212>  DNA
<213>  Artificial

<220>
<223>  shRNA

<400>  58
agcaccaagt tcgtctcagt tcaagagact gagacgaact tggtgct                    47


<210>  59
<211>  47
<212>  DNA
<213>  Artificial

<220>
<223>  shRNA

<400>  59
gatgcaacac tgaaagaact tcaagagagt tctttcagtg ttgcatc                    47


<210>  60
<211>  47
<212>  DNA
<213>  Artificial

<220>
<223>  shRNA

<400>  60
gtcaatggca gtgatgatat tcaagagata tcatcactgc cattgac                    47


<210>  61
<211>  47
<212>  DNA
<213>  Artificial

<220>
<223>  shRNA

<400>  61
cctgctagct gcctgtggct tcaagagagc cacaggcagc tagcagg                    47


<210>  62
<211>  47
<212>  DNA
<213>  Artificial

<220>
<223>  shRNA

<400>  62
ccacctttgc cagaaggagt tcaagagact ccttctggca aaggtgg                    47


<210>  63
<211>  47
<212>  DNA
<213>  Artificial

<220>
<223>  shRNA

<400>  63
ccctattgag gcaagtgtct tcaagagaga cacttgcctc aataggg                    47


<210>  64
<211>  47
<212>  DNA
<213>  Artificial

<220>
<223>  shRNA

<400>  64
gaaggaaaac ttgctgacgt tcaagagacg tcagcaagtt ttccttc                    47


<210>  65
<211>  47
<212>  DNA
<213>  Artificial

<220>
<223>  shRNA

<400>  65
ctcacctggg tccatgagat tcaagagatc tcatggaccc aggtgag                    47


<210>  66
<211>  47
<212>  DNA
<213>  Artificial

<220>
<223>  shRNA

<400>  66
gctgtcttac cgtgtggtct tcaagagaga ccacacggta agacagc                    47


<210>  67
<211>  47
<212>  DNA
<213>  Artificial

<220>
<223>  shRNA

<400>  67
cctggaccgc atgtatgact tcaagagagt catacatgcg gtccagg                    47


<210>  68
<211>  47
<212>  DNA
<213>  Artificial

<220>
<223>  shRNA

<400>  68
gtcaatggca gtgatgatat tcaagagata tcatcactgc cattgac                    47


<210>  69
<211>  47
<212>  DNA
<213>  Artificial

<220>
<223>  shRNA

<400>  69
cctgctagct gcctgtggct tcaagagagc cacaggcagc tagcagg                    47


<210>  70
<211>  47
<212>  DNA
<213>  Artificial

<220>
<223>  shRNA

<400>  70
ccacctttgc cagaaggagt tcaagagact ccttctggca aaggtgg                    47


<210>  71
<211>  47
<212>  DNA
<213>  Artificial

<220>
<223>  shRNA

<400>  71
ccctattgag gcaagtgtct tcaagagaga cacttgcctc aataggg                    47


<210>  72
<211>  47
<212>  DNA
<213>  Artificial

<220>
<223>  shRNA

<400>  72
ggaaatccga attgcttggt tcaagagacc aagcaattcg gatttcc                    47


<210>  73
<211>  47
<212>  DNA
<213>  Artificial

<220>
<223>  shRNA

<400>  73
cacatttctt caagtgtggt tcaagagacc acacttgaag aaatgtg                    47

<210>  74
<211>  47
<212>  DNA
<213>  Artificial

<220>
<223>  shRNA

<400>  74
cagcaggatg ctcaagaatt tcaagagaat tcttgagcat cctgctg                47


<210>  75
<211>  47
<212>  DNA
<213>  Artificial

<220>
<223>  shRNA

<400>  75
gctgaatacc tacattggct tcaagagagc caatgtaggt attcagc                47


<210>  76
<211>  47
<212>  DNA
<213>  Artificial

<220>
<223>  shRNA

<400>  76
gggcttgtgc ctggccttgt tcaagagaca aggccaggca caagccc                47


<210>  77
<211>  47
<212>  DNA
<213>  Artificial

<220>
<223>  shRNA

<400>  77
gccttgtcct gccaagaagt tcaagagact tcttggcagg acaaggc                47


<210>  78
<211>  47
<212>  DNA
<213>  Artificial

<220>
<223>  shRNA

<400>  78
gattgaagcc aagggaacgt tcaagagacg ttcccttggc ttcaatc                47


<210>  79
<211>  47

<212>  DNA
<213>  Artificial

<220>
<223>  shRNA

<400>  79
tggcgcctgc tccccatctt tcaagagaag atggggagca ggcgcca                47


<210>  80
<211>  47
<212>  DNA
<213>  Artificial

<220>
<223>  shRNA

<400>  80
gaaccagcag gctctgtggt tcaagagacc acagagcctg ctggttc                47


<210>  81
<211>  47
<212>  DNA
<213>  Artificial

<220>
<223>  shRNA

<400>  81
ggaagcataa ttatctgcct tcaagagagg cagataatta tgcttcc                47


<210>  82
<211>  47
<212>  DNA
<213>  Artificial

<220>
<223>  shRNA

<400>  82
agaagaagat gcttttcact tcaagagagt gaaaagcatc ttcttct                47


<210>  83
<211>  47
<212>  DNA
<213>  Artificial

<220>
<223>  shRNA

<400>  83
cttgcagagg aggaacccat tcaagagatg ggttcctcct ctgcaag                47


<210>  84
<211>  47
<212>  DNA
<213>  Artificial

<220>
<223>  shRNA

<400>  84
gcaaacaatc agcaatgcct tcaagagagg cattgctgat tgtttgc                    47


<210>  85
<211>  47
<212>  DNA
<213>  Artificial

<220>
<223>  shRNA

<400>  85
ttggactgat tcatgctatt tcaagagaat agcatgaatc agtccaa                    47


<210>  86
<211>  47
<212>  DNA
<213>  Artificial

<220>
<223>  shRNA

<400>  86
ctggcaattc gttgatgtat tcaagagata catcaacgaa ttgccag                    47


<210>  87
<211>  47
<212>  DNA
<213>  Artificial

<220>
<223>  shRNA

<400>  87
ttagatgggc ggaagccatt tcaagagaat ggcttccgcc catctaa                    47


<210>  88
<211>  47
<212>  DNA
<213>  Artificial

<220>
<223>  shRNA

<400>  88
gaggagtctc tgggctcggt tcaagagacc gagcccagag actcctc                    47


<210>  89
<211>  47
<212>  DNA
<213>  Artificial

<220>
<223>  shRNA

<400>  89
gagctgaagg gacaagaagt tcaagagact tcttgtccct tcagctc                    47


<210>  90
<211>  47
<212>  DNA
<213>  Artificial

<220>
<223>  shRNA

<400>  90
tgtcgggtag atgacaaggt tcaagagacc ttgtcatcta cccgaca                    47


<210>  91
<211>  47
<212>  DNA
<213>  Artificial

<220>
<223>  shRNA

<400>  91
cacagctgtt cttctgttct tcaagagaga acagaagaac agctgtg                    47


<210>  92
<211>  47
<212>  DNA
<213>  Artificial

<220>
<223>  shRNA

<400>  92
gtggaagcct ttacagatct tcaagagaga tctgtaaagg cttccac                    47


<210>  93
<211>  47
<212>  DNA
<213>  Artificial

<220>
<223>  shRNA

<400>  93
caacagctgc cttcatctgt tcaagagaca gatgaaggca gctgttg                    47


<210>  94
<211>  47
<212>  DNA
<213>  Artificial

<220>
<223>  shRNA

<400>  94
ccataggcag tcctcctaat tcaagagatt aggaggactg cctatgg                    47

<210>  95
<211>  47
<212>  DNA
<213>  Artificial

<220>
<223>  shRNA

<400>  95
tgtatcactg ccactggttt tcaagagaaa ccagtggcag tgataca                    47


<210>  96
<211>  47
<212>  DNA
<213>  Artificial

<220>
<223>  shRNA

<400>  96
catgttgggc agctgcagct tcaagagagc tgcagctgcc caacatg                    47


<210>  97
<211>  47
<212>  DNA
<213>  Artificial

<220>
<223>  shRNA

<400>  97
cacaactgga gacctgaagt tcaagagact tcaggtctcc agttgtg                    47


<210>  98
<211>  47
<212>  DNA
<213>  Artificial

<220>
<223>  shRNA

<400>  98
gtatgcctcc aagaaagagt tcaagagact ctttcttgga ggcatac                    47


<210>  99
<211>  47
<212>  DNA
<213>  Artificial

<220>
<223>  shRNA

<400>  99
cttcacagta catttctctt tcaagagaag agaaatgtac tgtgaag                    47


<210>  100
<211>  47

<212>  DNA
<213>  Artificial

<220>
<223>  shRNA

<400>  100
gtactttcaa ggccggggtt tcaagagaac cccggccttg aaagtac                    47


<210>  101
<211>  47
<212>  DNA
<213>  Artificial

<220>
<223>  shRNA

<400>  101
cttggacaag caagccaaat tcaagagatt tggcttgctt gtccaag                    47


<210>  102
<211>  47
<212>  DNA
<213>  Artificial

<220>
<223>  shRNA

<400>  102
gactattgtg actgatgttt tcaagagaaa catcagtcac aatagtc                    47


<210>  103
<211>  47
<212>  DNA
<213>  Artificial

<220>
<223>  shRNA

<400>  103
ggagaacttt ctgaagcgct tcaagagagc gcttcagaaa gttctcc                    47


<210>  104
<211>  47
<212>  DNA
<213>  Artificial

<220>
<223>  shRNA

<400>  104
gacgagagaa accttcacct tcaagagagg tgaaggtttc tctcgtc                    47


<210>  105
<211>  47
<212>  DNA
<213>  Artificial

<220>
<223>  shRNA

<400>  105
acattattct acattctttt tcaagagaaa agaatgtaga ataatgt                    47


<210>  106
<211>  47
<212>  DNA
<213>  Artificial

<220>
<223>  shRNA

<400>  106
agattcgcaa atggatgtat tcaagagata catccatttg cgaatct                    47


<210>  107
<211>  47
<212>  DNA
<213>  Artificial

<220>
<223>  shRNA

<400>  107
cattcccacc atgagtctgt tcaagagaca gactcatggt gggaatg                    47


<210>  108
<211>  47
<212>  DNA
<213>  Artificial

<220>
<223>  shRNA

<400>  108
gatcgcccga cacttccgct tcaagagagc ggaagtgtcg ggcgatc                    47


<210>  109
<211>  47
<212>  DNA
<213>  Artificial

<220>
<223>  shRNA

<400>  109
ccagcaggcc tacgtgctgt tcaagagaca gcacgtaggc ctgctgg                    47


<210>  110
<211>  47
<212>  DNA
<213>  Artificial

<220>
<223>  shRNA

<400>  110
gccagctcct ccacagcact tcaagagagt gctgtggagg agctggc                    47


<210>  111
<211>  47
<212>  DNA
<213>  Artificial

<220>
<223>  shRNA

<400>  111
cgccgccaag tggagcagat tcaagagatc tgctccactt ggcggcg                    47


<210>  112
<211>  47
<212>  DNA
<213>  Artificial

<220>
<223>  shRNA

<400>  112
gaagatgccc atgaattcct tcaagagagg aattcatggg catcttc                    47


<210>  113
<211>  47
<212>  DNA
<213>  Artificial

<220>
<223>  shRNA

<400>  113
caaacaggct gcgccaggct tcaagagagc ctggcgcagc ctgtttg                    47


<210>  114
<211>  47
<212>  DNA
<213>  Artificial

<220>
<223>  shRNA

<400>  114
acggcctagc gcctgatggt tcaagagacc atcaggcgct aggccgt                    47


<210>  115
<211>  47
<212>  DNA
<213>  Artificial

<220>
<223>  shRNA

<400>  115
ctgtaacctc tctgatcggt tcaagagacc gatcagagag gttacag                    47

<210>  116
<211>  47
<212>  DNA
<213>  Artificial

<220>
<223>  shRNA

<400>  116
tctgtcagtc catcctggct tcaagagagc caggatggac tgacaga                47


<210>  117
<211>  47
<212>  DNA
<213>  Artificial

<220>
<223>  shRNA

<400>  117
tgaagcgaga gtcttgtgat tcaagagatc acaagactct cgcttca                47


<210>  118
<211>  47
<212>  DNA
<213>  Artificial

<220>
<223>  shRNA

<400>  118
gatggagtgc taatggaaat tcaagagatt tccattagca ctccatc                47


<210>  119
<211>  47
<212>  DNA
<213>  Artificial

<220>
<223>  shRNA

<400>  119
ccttcagaga ttgacacgct tcaagagagc gtgtcaatct ctgaagg                47


<210>  120
<211>  47
<212>  DNA
<213>  Artificial

<220>
<223>  shRNA

<400>  120
cctgaccacg ttccgactgt tcaagagaca gtcggaacgt ggtcagg                47


<210>  121
<211>  47

<212>  DNA
<213>  Artificial

<220>
<223>  shRNA

<400>  121
gagttccttc gctgcctgat tcaagagatc aggcagcgaa ggaactc                47


<210>  122
<211>  47
<212>  DNA
<213>  Artificial

<220>
<223>  shRNA

<400>  122
gactgccttg ctgccttctt tcaagagaag aaggcagcaa ggcagtc                47


<210>  123
<211>  47
<212>  DNA
<213>  Artificial

<220>
<223>  shRNA

<400>  123
cgccgagggc tacgtactct tcaagagaga gtacgtagcc ctcggcg                47


<210>  124
<211>  47
<212>  DNA
<213>  Artificial

<220>
<223>  shRNA

<400>  124
ggcgagaaga aaggactgtt tcaagagaac agtcctttct tctcgcc                47


<210>  125
<211>  47
<212>  DNA
<213>  Artificial

<220>
<223>  shRNA

<400>  125
ggacgagaat tgataaagat tcaagagatc tttatcaatt ctcgtcc                47


<210>  126
<211>  47
<212>  DNA
<213>  Artificial

<220>
<223>  shRNA

<400>  126
gcacgagaat ttgggaatct tcaagagaga ttcccaaatt ctcgtgc                    47


<210>  127
<211>  47
<212>  DNA
<213>  Artificial

<220>
<223>  shRNA

<400>  127
ctacttcatg aaatattggt tcaagagacc aatatttcat gaagtag                    47


<210>  128
<211>  47
<212>  DNA
<213>  Artificial

<220>
<223>  shRNA

<400>  128
gataacagct tcttgtctat tcaagagata gacaagaagc tgttatc                    47


<210>  129
<211>  47
<212>  DNA
<213>  Artificial

<220>
<223>  shRNA

<400>  129
gagaatagga catcagggct tcaagagagc cctgatgtcc tattctc                    47


<210>  130
<211>  47
<212>  DNA
<213>  Artificial

<220>
<223>  shRNA

<400>  130
cttggaagac tgaacctgtt tcaagagaac aggttcagtc ttccaag                    47


<210>  131
<211>  47
<212>  DNA
<213>  Artificial

<220>
<223>  shRNA

<400>  131
caactccttt gtggatgcat tcaagagatg catccacaaa ggagttg                47

<210>  132
<211>  47
<212>  DNA
<213>  Artificial

<220>
<223>  shRNA

<400>  132
gatgttgtct ccaaatgcat tcaagagatg catttggaga caacatc                47

<210>  133
<211>  47
<212>  DNA
<213>  Artificial

<220>
<223>  shRNA

<400>  133
cgtggggact gtacctccct tcaagagagg gaggtacagt ccccacg                47

<210>  134
<211>  47
<212>  DNA
<213>  Artificial

<220>
<223>  shRNA

<400>  134
gtacagcttc agaaccaagt tcaagagact tggttctgaa gctgtac                47

<210>  135
<211>  47
<212>  DNA
<213>  Artificial

<220>
<223>  shRNA

<400>  135
gatgatcttc agagagcaat tcaagagatt gctctctgaa gatcatc                47

<210>  136
<211>  47
<212>  DNA
<213>  Artificial

<220>
<223>  shRNA

<400>  136
ggaacatcgg aatttgcctt tcaagagaag gcaaattccg atgttcc                47

<210>  137
<211>  47
<212>  DNA
<213>  Artificial

<220>
<223>  shRNA

<400>  137
gagctagtga gggactcttt tcaagagaaa gagtccctca ctagctc                47


<210>  138
<211>  47
<212>  DNA
<213>  Artificial

<220>
<223>  shRNA

<400>  138
gcagggttct ttaaggcaat tcaagagatt gccttaaaga accctgc                47


<210>  139
<211>  47
<212>  DNA
<213>  Artificial

<220>
<223>  shRNA

<400>  139
tcgatgattc ctctgaaact tcaagagagt ttcagaggaa tcatcga                47


<210>  140
<211>  47
<212>  DNA
<213>  Artificial

<220>
<223>  shRNA

<400>  140
gataatggaa atattgaact tcaagagagt tcaatatttc cattatc                47


<210>  141
<211>  47
<212>  DNA
<213>  Artificial

<220>
<223>  shRNA

<400>  141
gttcttcatt taaatgatat tcaagagata tcatttaaat gaagaac                47


<210>  142
<211>  47

<212>  DNA
<213>  Artificial

<220>
<223>  shRNA

<400>  142
gttaacaaac acataaagtt tcaagagaac tttatgtgtt tgttaac                47


<210>  143
<211>  47
<212>  DNA
<213>  Artificial

<220>
<223>  shRNA

<400>  143
gttagagaag attcttcgtt tcaagagaac gaagaatctt ctctaac                47


<210>  144
<211>  47
<212>  DNA
<213>  Artificial

<220>
<223>  shRNA

<400>  144
gttgattgga caattaaact tcaagagagt ttaattgtcc aatcaac                47


<210>  145
<211>  47
<212>  DNA
<213>  Artificial

<220>
<223>  shRNA

<400>  145
ggttgatacc gtaaagcgct tcaagagagc gctttacggt atcaacc                47


<210>  146
<211>  47
<212>  DNA
<213>  Artificial

<220>
<223>  shRNA

<400>  146
gcaatgaaac gtccaatggt tcaagagacc attggacgtt tcattgc                47


<210>  147
<211>  47
<212>  DNA
<213>  Artificial

<220>
<223>  shRNA

<400>  147
agctagagaa aattcttcgt tcaagagacg aagaattttc tctagct                    47


<210>  148
<211>  47
<212>  DNA
<213>  Artificial

<220>
<223>  shRNA

<400>  148
gatcctatat gatggatgat tcaagagatc atccatcata taggatc                    47


<210>  149
<211>  47
<212>  DNA
<213>  Artificial

<220>
<223>  shRNA

<400>  149
gttcttcttg tcagtgaaat tcaagagatt tcactgacaa gaagaac                    47


<210>  150
<211>  47
<212>  DNA
<213>  Artificial

<220>
<223>  shRNA

<400>  150
cttgagcttg agtgaccact tcaagagagt ggtcactcaa gctcaag                    47


<210>  151
<211>  47
<212>  DNA
<213>  Artificial

<220>
<223>  shRNA

<400>  151
gaccggccag cgagtctact tcaagagagt agactcgctg gccggtc                    47


<210>  152
<211>  47
<212>  DNA
<213>  Artificial

<220>
<223>  shRNA

<400>  152
ggacctgggc tacatctact tcaagagagt agatgtagcc caggtcc                    47


<210>  153
<211>  47
<212>  DNA
<213>  Artificial

<220>
<223>  shRNA

<400>  153
ctctgtggtc caggtgctct tcaagagaga gcacctggac cacagag                    47


<210>  154
<211>  47
<212>  DNA
<213>  Artificial

<220>
<223>  shRNA

<400>  154
gaccacacga tttgcctcat tcaagagatg aggcaaatcg tgtggtc                    47


<210>  155
<211>  47
<212>  DNA
<213>  Artificial

<220>
<223>  shRNA

<400>  155
tggcttgttt attgaaggat tcaagagatc cttcaataaa caagcca                    47


<210>  156
<211>  47
<212>  DNA
<213>  Artificial

<220>
<223>  shRNA

<400>  156
gtgaatttgg ggaagataat tcaagagatt atcttcccca aattcac                    47


<210>  157
<211>  47
<212>  DNA
<213>  Artificial

<220>
<223>  shRNA

<400>  157
cgctatagct tgaatgagtt tcaagagaac tcattcaagc tatagcg                    47

<210>  158
<211>  47
<212>  DNA
<213>  Artificial

<220>
<223>  shRNA

<400>  158
gatatcctgg ctccacacat tcaagagatg tgtggagcca ggatatc                47


<210>  159
<211>  47
<212>  DNA
<213>  Artificial

<220>
<223>  shRNA

<400>  159
gagccagtcg gatgtagatt tcaagagaat ctacatccga ctggctc                47


<210>  160
<211>  47
<212>  DNA
<213>  Artificial

<220>
<223>  shRNA

<400>  160
gtaaattctg aaggcgaatt tcaagagaat tcgccttcag aatttac                47


<210>  161
<211>  47
<212>  DNA
<213>  Artificial

<220>
<223>  shRNA

<400>  161
gccctcctaa atcaggcaat tcaagagatt gcctgattta ggagggc                47


<210>  162
<211>  47
<212>  DNA
<213>  Artificial

<220>
<223>  shRNA

<400>  162
gttgagaaat ggagtgaagt tcaagagact tcactccatt tctcaac                47


<210>  163
<211>  47

<212>  DNA
<213>  Artificial

<220>
<223>  shRNA

<400>  163
gcttggaaaa tgcaaggcgt tcaagagacg ccttgcattt tccaagc                47


<210>  164
<211>  47
<212>  DNA
<213>  Artificial

<220>
<223>  shRNA

<400>  164
ctgcatcata gaccagatct tcaagagaga tctggtctat gatgcag                47


<210>  165
<211>  47
<212>  DNA
<213>  Artificial

<220>
<223>  shRNA

<400>  165
gatcaccacg tatgtgtcct tcaagagagg acacatacgt ggtgatc                47


<210>  166
<211>  47
<212>  DNA
<213>  Artificial

<220>
<223>  shRNA

<400>  166
tgacaacaag tattccctgt tcaagagaca gggaatactt gttgtca                47


<210>  167
<211>  47
<212>  DNA
<213>  Artificial

<220>
<223>  shRNA

<400>  167
gaaatataag acagattcct tcaagagagg aatctgtctt atatttc                47


<210>  168
<211>  47
<212>  DNA
<213>  Artificial

<220>
<223>  shRNA

<400>  168
cccatcaagt ttagaggatt tcaagagaat cctctaaact tgatggg                47


<210>  169
<211>  47
<212>  DNA
<213>  Artificial

<220>
<223>  shRNA

<400>  169
ggtgtcccat gggaatatat tcaagagata tattcccatg ggacacc                47


<210>  170
<211>  47
<212>  DNA
<213>  Artificial

<220>
<223>  shRNA

<400>  170
gaatgccgac ctacaaagat tcaagagatc tttgtaggtc ggcattc                47


<210>  171
<211>  47
<212>  DNA
<213>  Artificial

<220>
<223>  shRNA

<400>  171
cagttatatt ctgtgatgtt tcaagagaac atcacagaat ataactg                47


<210>  172
<211>  47
<212>  DNA
<213>  Artificial

<220>
<223>  shRNA

<400>  172
gaggtgttgg ggacaaaggt tcaagagacc tttgtcccca acacctc                47


<210>  173
<211>  47
<212>  DNA
<213>  Artificial

<220>
<223>  shRNA

<400>  173
gtgggctcat tggctgaagt tcaagagact tcagccaatg agcccac                47

<210>  174
<211>  47
<212>  DNA
<213>  Artificial

<220>
<223>  shRNA

<400>  174
gagctactga ggacagaaat tcaagagatt tctgtcctca gtagctc                47

<210>  175
<211>  47
<212>  DNA
<213>  Artificial

<220>
<223>  shRNA

<400>  175
tcagcaggat gctcaggagt tcaagagact cctgagcatc ctgctga                47

<210>  176
<211>  47
<212>  DNA
<213>  Artificial

<220>
<223>  shRNA

<400>  176
gaagttctcc atccagaggt tcaagagacc tctggatgga gaacttc                47

<210>  177
<211>  47
<212>  DNA
<213>  Artificial

<220>
<223>  shRNA

<400>  177
gccggtcccc accagcagct tcaagagagc tgctggtggg gaccggc                47

<210>  178
<211>  47
<212>  DNA
<213>  Artificial

<220>
<223>  shRNA

<400>  178
cactcgggag ttgagagatt tcaagagaat ctctcaactc ccgagtg                47

<210>  179
<211>  47
<212>  DNA
<213>  Artificial

<220>
<223>  shRNA

<400>  179
gcccttgggt ctgtttgact tcaagagagt caaacagacc caagggc                47


<210>  180
<211>  47
<212>  DNA
<213>  Artificial

<220>
<223>  shRNA

<400>  180
ctcaacacta aacagcaagt tcaagagact tgctgtttag tgttgag                47


<210>  181
<211>  47
<212>  DNA
<213>  Artificial

<220>
<223>  shRNA

<400>  181
gatttcattg gacagcatat tcaagagata tgctgtccaa tgaaatc                47


<210>  182
<211>  47
<212>  DNA
<213>  Artificial

<220>
<223>  shRNA

<400>  182
catggggcac caactaattt tcaagagaaa ttagttggtg ccccatg                47


<210>  183
<211>  47
<212>  DNA
<213>  Artificial

<220>
<223>  shRNA

<400>  183
ggtgtctctg cgggattgtt tcaagagaac aatcccgcag agacacc                47


<210>  184
<211>  47

<212>  DNA
<213>  Artificial

<220>
<223>  shRNA

<400>  184
agttcagtag gtgtagactt tcaagagaag tctacaccta ctgaact                47


<210>  185
<211>  47
<212>  DNA
<213>  Artificial

<220>
<223>  shRNA

<400>  185
gagttcctga agctcctcat tcaagagatg aggagcttca ggaactc                47


<210>  186
<211>  47
<212>  DNA
<213>  Artificial

<220>
<223>  shRNA

<400>  186
ggatttgctg ggggcaaggt tcaagagacc ttgcccccag caaatcc                47


<210>  187
<211>  47
<212>  DNA
<213>  Artificial

<220>
<223>  shRNA

<400>  187
ctcagaaagc caacattcat tcaagagatg aatgttggct ttctgag                47


<210>  188
<211>  47
<212>  DNA
<213>  Artificial

<220>
<223>  shRNA

<400>  188
cgcattgtaa taagaaggtt tcaagagaac cttcttatta caatgcg                47


<210>  189
<211>  47
<212>  DNA
<213>  Artificial

<220>
<223>  shRNA

<400>  189
gggaggaaaa tgcagaaatt tcaagagaat ttctgcattt tcctccc                47


<210>  190
<211>  47
<212>  DNA
<213>  Artificial

<220>
<223>  shRNA

<400>  190
ttacaaattt aggaaatact tcaagagagt atttcctaaa tttgtaa                47


<210>  191
<211>  47
<212>  DNA
<213>  Artificial

<220>
<223>  shRNA

<400>  191
gttatgaatt gatatgcagt tcaagagact gcatatcaat tcataac                47


<210>  192
<211>  47
<212>  DNA
<213>  Artificial

<220>
<223>  shRNA

<400>  192
gtgataacac aactaatggt tcaagagacc attagttgtg ttatcac                47


<210>  193
<211>  47
<212>  DNA
<213>  Artificial

<220>
<223>  shRNA

<400>  193
gtagaggaga gttctgaaat tcaagagatt tcagaactct cctctac                47


<210>  194
<211>  47
<212>  DNA
<213>  Artificial

<220>
<223>  shRNA

<400>  194
gcctctaatc ctgataaggt tcaagagacc ttatcaggat tagaggc                    47


<210>  195
<211>  47
<212>  DNA
<213>  Artificial

<220>
<223>  shRNA

<400>  195
gatgatcttc aggctgccat tcaagagatg gcagcctgaa gatcatc                    47


<210>  196
<211>  47
<212>  DNA
<213>  Artificial

<220>
<223>  shRNA

<400>  196
gtatggacaa gagcgttggt tcaagagacc aacgctcttg tccatac                    47


<210>  197
<211>  47
<212>  DNA
<213>  Artificial

<220>
<223>  shRNA

<400>  197
cgaacccttc tggaacagtt tcaagagaac tgttccagaa gggttcg                    47


<210>  198
<211>  47
<212>  DNA
<213>  Artificial

<220>
<223>  shRNA

<400>  198
gtggcatgaa gattatagtt tcaagagaac tataatcttc atgccac                    47


<210>  199
<211>  47
<212>  DNA
<213>  Artificial

<220>
<223>  shRNA

<400>  199
ggtgaacaag gacagtatct tcaagagaga tactgtcctt gttcacc                    47

<210>  200
<211>  47
<212>  DNA
<213>  Artificial

<220>
<223>  shRNA

<400>  200
gcaatagagg atgattctgt tcaagagaca gaatcatcct ctattgc                47


<210>  201
<211>  47
<212>  DNA
<213>  Artificial

<220>
<223>  shRNA

<400>  201
tctgtgaatg ccaaagttct tcaagagaga actttggcat tcacaga                47


<210>  202
<211>  47
<212>  DNA
<213>  Artificial

<220>
<223>  shRNA

<400>  202
cacaccaggg aaggtctagt tcaagagact agaccttccc tggtgtg                47


<210>  203
<211>  47
<212>  DNA
<213>  Artificial

<220>
<223>  shRNA

<400>  203
gcaggaagat gcccatgaat tcaagagatt catgggcatc ttcctgc                47


<210>  204
<211>  47
<212>  DNA
<213>  Artificial

<220>
<223>  shRNA

<400>  204
gaatgtgcaa tatcctgagt tcaagagact caggatattg cacattc                47


<210>  205
<211>  47

<212>  DNA
<213>  Artificial

<220>
<223>  shRNA

<400>  205
tggatgatgc caaggtcact tcaagagagt gaccttggca tcatcca                    47


<210>  206
<211>  47
<212>  DNA
<213>  Artificial

<220>
<223>  shRNA

<400>  206
gctccgtgct aaacctctct tcaagagaga gaggtttagc acggagc                    47


<210>  207
<211>  47
<212>  DNA
<213>  Artificial

<220>
<223>  shRNA

<400>  207
gcctccacct caacagaggt tcaagagacc tctgttgagg tggaggc                    47


<210>  208
<211>  47
<212>  DNA
<213>  Artificial

<220>
<223>  shRNA

<400>  208
ctgcatcata gaccaaatct tcaagagaga tttggtctat gatgcag                    47


<210>  209
<211>  47
<212>  DNA
<213>  Artificial

<220>
<223>  shRNA

<400>  209
gatcactaca tacatttcct tcaagagagg aaatgtatgt agtgatc                    47


<210>  210
<211>  47
<212>  DNA
<213>  Artificial

<220>
<223>  shRNA

<400>  210
gtaaagagag cagaatgaat tcaagagatt cattctgctc tctttac                    47


<210>  211
<211>  47
<212>  DNA
<213>  Artificial

<220>
<223>  shRNA

<400>  211
cgcggggcgc agtggtatct tcaagagaga taccactgcg ccccgcg                    47


<210>  212
<211>  47
<212>  DNA
<213>  Artificial

<220>
<223>  shRNA

<400>  212
cagaaggcag tggggaagat tcaagagatc ttccccactg ccttctg                    47


<210>  213
<211>  47
<212>  DNA
<213>  Artificial

<220>
<223>  shRNA

<400>  213
gcctgggaga atcacaggtt tcaagagaac ctgtgattct cccaggc                    47


<210>  214
<211>  47
<212>  DNA
<213>  Artificial

<220>
<223>  shRNA

<400>  214
accagacaag gaaataccct tcaagagagg gtatttcctt gtctggt                    47


<210>  215
<211>  47
<212>  DNA
<213>  Artificial

<220>
<223>  shRNA

<400>  215
cacatccacc acatcgacct tcaagagagg tcgatgtggt ggatgtg                47

<210>  216
<211>  47
<212>  DNA
<213>  Artificial

<220>
<223>  shRNA

<400>  216
gtcacaaccc aagaccatgt tcaagagaca tggtcttggg ttgtgac                47

<210>  217
<211>  47
<212>  DNA
<213>  Artificial

<220>
<223>  shRNA

<400>  217
ctcaacagga caaatcccat tcaagagatg ggatttgtcc tgttgag                47

<210>  218
<211>  47
<212>  DNA
<213>  Artificial

<220>
<223>  shRNA

<400>  218
tagatcaatt attgtggatt tcaagagaat ccacaataat tgatcta                47

<210>  219
<211>  47
<212>  DNA
<213>  Artificial

<220>
<223>  shRNA

<400>  219
ggaacacctt attgatgaat tcaagagatt catcaataag gtgttcc                47

<210>  220
<211>  47
<212>  DNA
<213>  Artificial

<220>
<223>  shRNA

<400>  220
ctttaacaga aattgtctct tcaagagaga gacaatttct gttaaag                47

<210>  221
<211>  47
<212>  DNA
<213>  Artificial

<220>
<223>  shRNA

<400>  221
cctatgcagt acaaagtggt tcaagagacc actttgtact gcatagg                47


<210>  222
<211>  47
<212>  DNA
<213>  Artificial

<220>
<223>  shRNA

<400>  222
gatcttttct tgctttggat tcaagagatc caaagcaaga aaagatc                47


<210>  223
<211>  47
<212>  DNA
<213>  Artificial

<220>
<223>  shRNA

<400>  223
cagcatcctt caggccttat tcaagagata aggcctgaag gatgctg                47


<210>  224
<211>  47
<212>  DNA
<213>  Artificial

<220>
<223>  shRNA

<400>  224
gatagtgact cggatctgct tcaagagagc agatccgagt cactatc                47


<210>  225
<211>  47
<212>  DNA
<213>  Artificial

<220>
<223>  shRNA

<400>  225
gacatcacag cccgggagtt tcaagagaac tcccgggctg tgatgtc                47


<210>  226
<211>  47

<212>  DNA
<213>  Artificial

<220>
<223>  shRNA

<400>  226
ggacacagcc tatgtgctgt tcaagagaca gcacataggc tgtgtcc                    47


<210>  227
<211>  47
<212>  DNA
<213>  Artificial

<220>
<223>  shRNA

<400>  227
gtggaggaga tctacgacct tcaagagagg tcgtagatct cctccac                    47


<210>  228
<211>  47
<212>  DNA
<213>  Artificial

<220>
<223>  shRNA

<400>  228
ctcttgtgca actcatgcct tcaagagagg catgagttgc acaagag                    47


<210>  229
<211>  47
<212>  DNA
<213>  Artificial

<220>
<223>  shRNA

<400>  229
acagggcccc tgcagcctct tcaagagaga ggctgcaggg gccctgt                    47


<210>  230
<211>  47
<212>  DNA
<213>  Artificial

<220>
<223>  shRNA

<400>  230
gaagacctgg cggcaggtgt tcaagagaca cctgccgcca ggtcttc                    47


<210>  231
<211>  21
<212>  DNA
<213>  Artificial

<220>
<223>  siRNA

<400>  231
gcctgtgcct cttcagctac c                                                21


<210>  232
<211>  21
<212>  DNA
<213>  Artificial

<220>
<223>  siRNA

<400>  232
gcggagacag cgacgaagag c                                                21


<210>  233
<211>  19
<212>  DNA
<213>  Artificial

<220>
<223>  siRNA

<400>  233
cttattggag agagcacga                                                   19